# EUROPEAN PATENT APPLICATION

(11) **EP 4 831 182 A1**
(43) Date of publication of application: **28.10.2026**
(21) Application number: 24906351.2
(22) Date of filing: 17.12.2024
(51) Int. Cl.: C07D 498/04, C07D 519/00, A61K 31/444, A61K 31/519, A61P 35/02, A61P 35/00, A61P 29/00, A61P 37/02

(54) **COMPOUND HAVING DUAL DEGRADATION ACTIVITY AGAINST IRAK4 AND FLT3 PROTEINS, PHARMACEUTICAL COMPOSITION COMPRISING SAME, AND USE THEREOF**

(30) Priority: 20.12.2023 CN 202311765223
(71) Applicant: Accro Bioscience (HK) Limited, Central, Hong Kong (HK)
(72) Inventor: ZHANG, Xiaohu, Hong Kong (CN); HE, Sudan, Hong Kong (CN)
(74) Representative: Weickmann & Weickmann PartmbB
(86) International application number: PCT/CN2024/139964
(87) International publication number: WO 2025/130871

(57) **Abstract**

The present disclosure belongs to the field of pharmaceutical chemistry, and in particular relates to a compound having a dual degradation activity against IRAK4 and FLT3 proteins, a pharmaceutical composition comprising same, and the use thereof. Provided in the present disclosure is a compound having a structure of formula (I), which can effectively degrade IRAK4 and/or FLT3 proteins in cells, and can be used for preventing and/or treating diseases and/or conditions that are at least partially responsive to IRAK4 and/or FLT3.

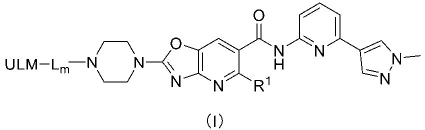

## Description

### Cross-Reference to Related Applications

The present disclosure claims priority to and the benefit of Chinese Patent Application No. 202311765223.2, filed with the China National Intellectual Property Administration on December 20, 2023, entitled "Compound having Dual Degradation Activity Against IRAK4 and FLT3 Proteins, Pharmaceutical Composition Comprising Same, and Use Thereof", the entire contents of which are incorporated herein by reference in their entirety.

### Technical Field

The present disclosure belongs to the field of medical technology, and relates to a compound with dual IRAK4 and FLT3 protein degrading activity, a pharmaceutical composition comprising the same, and a medical use thereof, in particular for the prevention and/or treatment of diseases or disorders at least partially responsive to IRAK4- and/or FLT3-mediated signaling, including tumors, inflammatory diseases, and autoimmune diseases.

### Background

Interleukin-1 receptor-associated kinase 4 (IRAK4) is a serine/threonine protein kinase that plays an important role in the innate immune response and is a key signaling factor in the downstream inflammatory signaling pathways mediated by the interleukin receptor family (IL-1, IL-18, IL-33) and the Toll-like receptor family (except TLR3). After extracellular signaling molecules bind to interleukin receptors or Toll-like receptors, intracellular recruitment of the MyD88-IRAK4-IRAK1/2 multiprotein complex Myddosome occurs, which in turn phosphorylates and activates IRAK1/2 and the downstream NF-κB and MAPK signaling pathways, promoting the release of related pro-inflammatory factors. Among them, IRAK4, as a key regulatory protein, can control TLR/IL-1R-mediated inflammatory signaling through its kinase and scaffold functions.

Excessive activation of the IRAK4-mediated signaling pathway leads to a surge in inflammatory factors in the body, which is closely related to a variety of autoimmune diseases, such as rheumatoid arthritis, systemic lupus erythematosus, inflammatory bowel disease, psoriasis and airway inflammation. In addition, high levels of IRAK4 phosphorylation have been found in a variety of tumors, such as hematological malignancies, colon cancer and melanoma. Studies have found that mutations in splicing factors U2AF1/SF3B1 can also cause aberrant protein splicing in cells, leading to overexpression of the oncogenic IRAK4-L (IRAK4 Long Form) isoform, which abnormally and continuously activates the downstream NF-κB signaling pathway, and is correlated with the occurrence and poor prognosis of myelodysplastic syndrome (MDS)/acute myeloid leukemia (AML).

FMS-like tyrosine kinase 3 (FLT3) belongs to the Type III receptor tyrosine kinase family, which is mainly expressed in a variety of lymphohematopoietic stem cells and tissues, and participates in the proliferation and differentiation of hematopoietic stem cells. FLT3 is a transmembrane protein composed of an extracellular receptor domain, a transmembrane domain, a juxtamembrane domain and an intracellular kinase domain. After binding to ligands extracellularly, FLT3 dimerizes and autophosphorylates, activating downstream signal transduction, including STAT5A, RAS/MAPK, PI3K/AKT and other pathways, promoting the survival, growth and differentiation of bone marrow hematopoietic stem cells.

Proteolysis-targeting chimera (PROTAC) is a technology different from traditional small-molecule inhibitors, which is a special heterobifunctional molecule. One end is a small-molecule inhibitor that can bind to the target protein, the other end is a ligand that can recognize E3 ubiquitin ligase, and the two are connected by a linking chain. PROTAC molecules can bring the target protein and E3 ubiquitin ligase close to form a ternary complex, and finally degrade the target protein through the ubiquitin-proteasome pathway.

At present, PROTAC technology has been used in the development of drugs for multiple targets. However, the research on degraders that can simultaneously degrade IRAK4 and FLT3 proteins has not been disclosed. Therefore, the development of novel degraders with dual IRAK4 and FLT3 protein degradation activities, which simultaneously inhibit the FLT3 and IRAK4-mediated signaling pathways, has the potential to improve the therapeutic effect of clinically FLT3-mutated AML patients and alleviate the drug resistance problem of clinical small-molecule therapy.

### SUMMARY

### Problems to be Solved

The purpose of the present disclosure is to provide a compound having dual degradation activity against IRAK4 and FLT3 proteins, pharmaceutical composition comprising same, and use thereof.

### Solution to the Problems

### First Aspect

The present disclosure provides a compound of formula (I), or a pharmaceutically acceptable salt or solvate thereof: 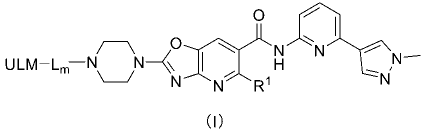 wherein,
R¹ is selected from the group consisting of the following moieties: 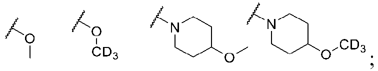
ULM is selected from the group consisting of the following moieties: 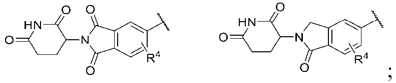
Lₘ is a linking moiety consisting of m fully identical, partially identical, or mutually different L groups, which covalently links ULM and the piperazine ring;
m is selected from the group consisting of 1, 2, 3, 4, and 5;
each L is independently selected from the group consisting of the following groups:
   1) C₃₋₁₂ cycloalkylene substituted with 1 to 3 R^{b} groups independent of each other;
   2) C₆₋₁₀ arylene substituted with 1 to 3 R^{b} groups independent of each other;
   3) 4- to 12-membered heterocycloalkylene substituted with 1 to 3 R^{b} groups independent of each other;
   4) 5- to 12-membered heteroarylene substituted with 1 to 3 R^{b} groups independent of each other;
   5) C₁₋₁₂ alkylene substituted with 1 to 3 R^{d} groups independent of each other;
   6) C₂₋₁₂ alkenylene substituted with 1 to 3 R^{d} groups independent of each other;
   7) C₂₋₁₂ alkynylene substituted with 1 to 3 R^{d} groups independent of each other;
   8) a moiety having 1 to 6 units of ethylene glycol or propylene glycol; and
   9) -C(O)-, -C(O)O-, -O-, -N(R^{c})-, -S-, -S(O)-, -C(S)-, -C(S)O-, -S(O)₂-, -S(O)N(R^{c})-, -S(O)₂N(R^{c})-, -C(O)-N(R^{c})-, -N(R^{c})C(O)-N(R^{c})-, and -OC(O)-N(R^{c})-;
if present, R⁴ is selected from the group consisting of hydrogen, deuterium, halogen, cyano, C₁₋₆ alkyl, C₃₋₆ cycloalkyl, C₆₋₁₀ aryl, C₅₋₁₂ heteroaryl, -OR^{c}, -C(O)R^{c}, -C(O)OR^{c}, -C(O) -N(R^{c})₂, -N(R^{c})₂, -N(R^{c})C(O) -R^{c}, -N(R^{c})C(O)O-R^{c}, -N(R^{c})C(O)N(R^{c})₂, -OC(O)R^{c}, -OC(O)-N(R^{c})₂, -SR^{c}, -S(O)R^{c}, -S(O)₂R^{c}, and -S(O)₂-N(R^{c})₂;
if present, each R^{b} is independently selected from the group consisting of hydrogen, deuterium, halogen, cyano, C₁₋₆ alkyl, C₃₋₆ cycloalkyl, C₆₋₁₀ aryl, C₅₋₁₂ heteroaryl, -OR^{e}, -C(O)R^{c}, -C(O)OR^{c}, -C(O)-N(R^{c})₂, -N(R^{c})₂, -N(R^{c})C(O)-R^{c}, -N(R^{c})C(O)O-R^{c}, -N(R^{c})C(O)N(R^{c})₂, -OC(O)R^{c}, -OC(O)-N(R^{c})₂, -SR^{c}, -S(O)R^{c}, -S(O)₂R^{c}, and -S(O)₂-N(R^{c})₂; if present, said C₁₋₆ alkyl, C₃₋₆ cycloalkyl, C₆₋₁₀ aryl, or C₅₋₁₂ heteroaryl is optionally substituted with 1 to 3 R^{d} groups; if present, any two non-adjacent R^{b} groups optionally form a bridged ring via carbon atoms; or any two R^{b} groups, together with the carbon atom to which they are attached, form a spiro ring;
if present, each R^{c} is independently selected from the group consisting of hydrogen, C₁₋₆ alkyl, and C₃₋₆ cycloalkyl;
if present, each R^{d} is independently selected from the group consisting of hydrogen, deuterium, halogen, hydroxy, amino, methylamino, dimethylamino, cyano, methyl, deuterated methyl, methoxy, and deuterated methoxy.

In one embodiment of the present disclosure, Lₘ is a linking moiety consisting of m fully identical, partially identical, or mutually different L groups, which covalently links ULM and the piperazine ring;
m is selected from the group consisting of 1, 2, 3, 4, and 5;
each L is independently selected from the group consisting of the following groups:
   1) C₃₋₁₂ cycloalkylene substituted with 1 to 3 R^{b} groups independent of each other;
   2) C₆₋₁₀ arylene substituted with 1 to 3 R^{b} groups independent of each other;
   3) 4- to 12-membered heterocycloalkylene substituted with 1 to 3 R^{b} groups independent of each other;
   4) 5- to 12-membered heteroarylene substituted with 1 to 3 R^{b} groups independent of each other;
   5) a moiety having 1 to 6 units of ethylene glycol or propylene glycol; and
   6)-C(O)-, -C(O)O-, -O-, -N(R^{c})-, -C(O)-N(R^{c})-, -N(R^{c})C(O)-N(R^{c})-, and -OC(O) -N(R^{c})-;
R^{b} and R^{c} are as defined in claim 1.

In one embodiment of the present disclosure, Lₘ is a linking moiety consisting of m fully identical, partially identical, or mutually different L groups, which covalently links ULM and the piperazine ring;
m is selected from the group consisting of 1, 2, 3, 4, and 5;
each L is independently selected from the group consisting of the following groups:
   (1) -C(O) -, -CH₂-, -C(D)(H)-, -C(D)₂-, -CR^{c}F-, -CF₂-, -O-, -N(R^{c})-, -C(O)-N(R^{c})-, -N(R^{c})C(O)-N(R^{c})-, and -OC(O)-N(R^{c})-; and
   (2) one of the following moieties: 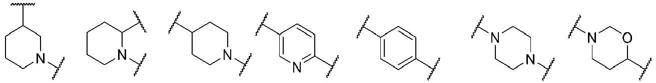 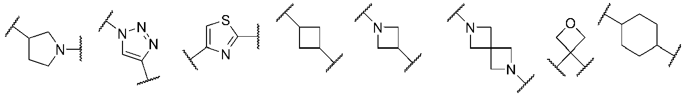 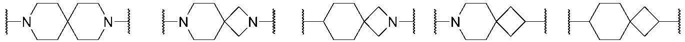 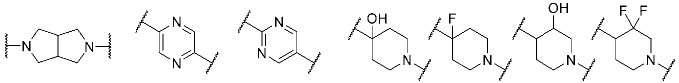 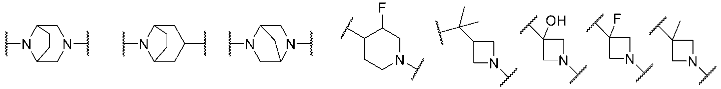 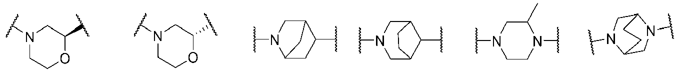 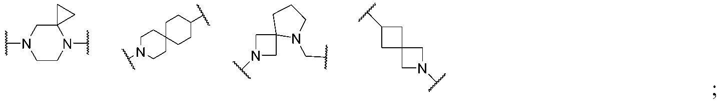
R^{c} is as defined in claim 1.

In a preferred embodiment of the present disclosure, at least one L is selected from the group consisting of the following moieties:

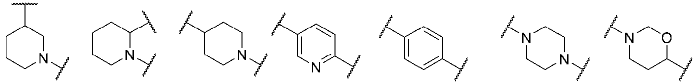

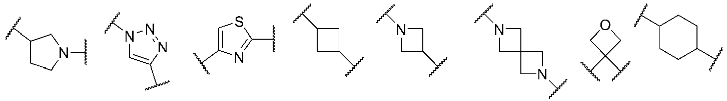

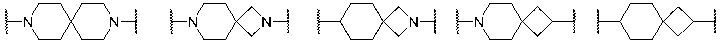

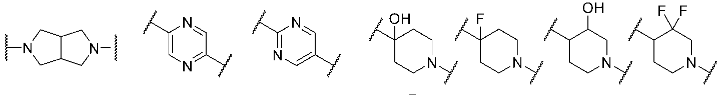

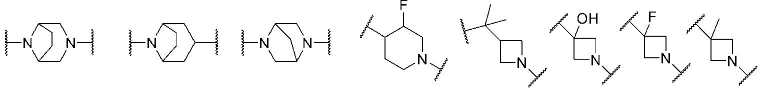

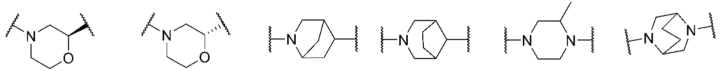

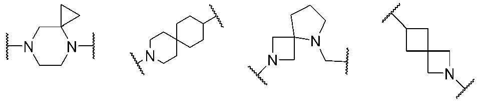

In one embodiment of the present disclosure, Lₘ is selected from the group consisting of the following moieties:

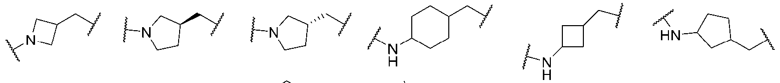

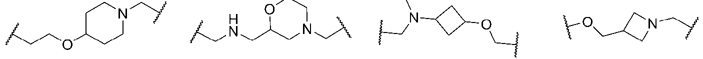

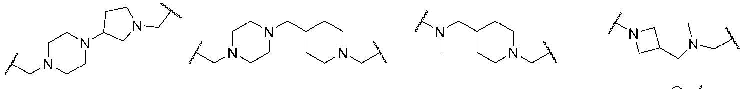

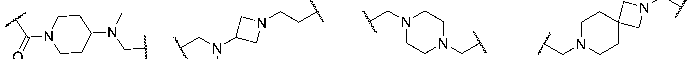

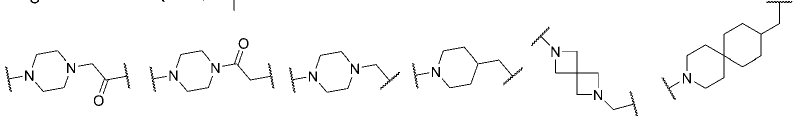

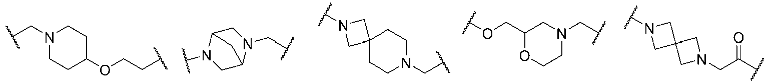

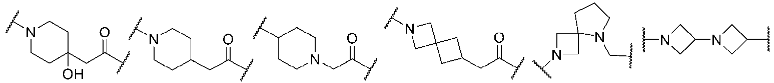

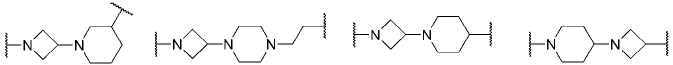

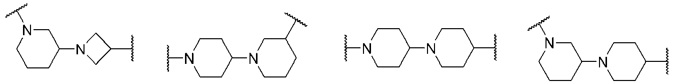

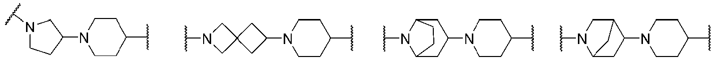

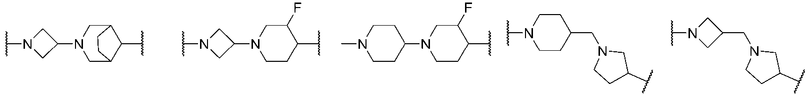

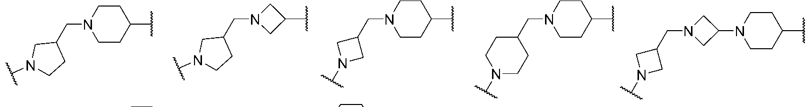

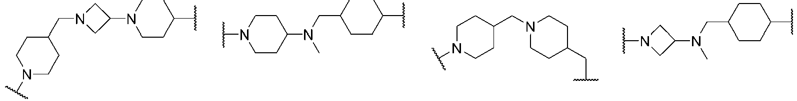

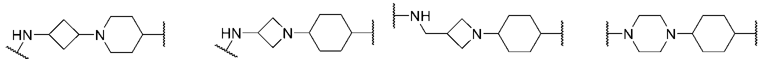

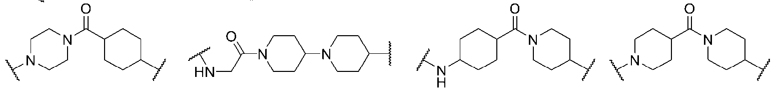

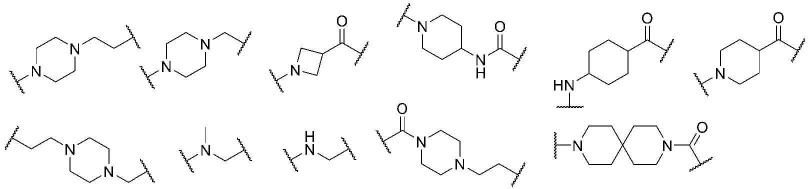

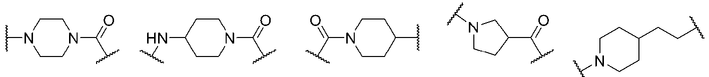

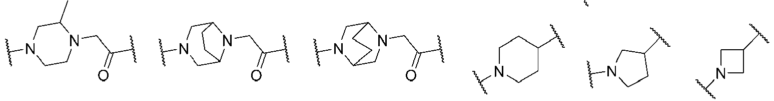

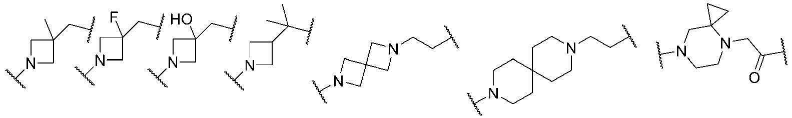

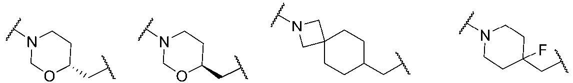

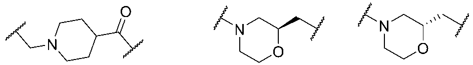

In one embodiment of the present disclosure, Lₘ is selected from the group consisting of the following moieties:

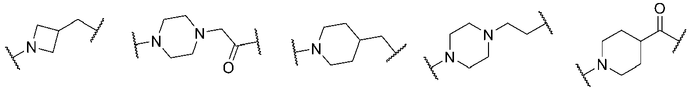

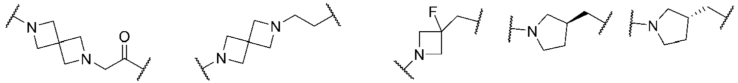

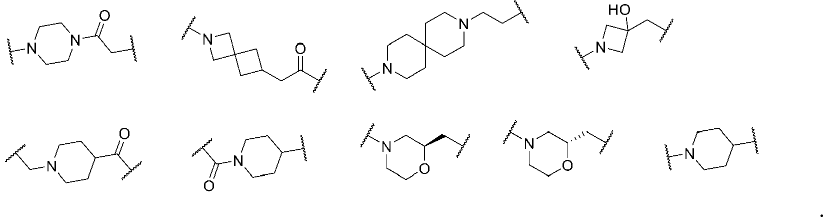

### Second Aspect

The present disclosure provides a compound selected from the group consisting of the following compounds, or a pharmaceutically acceptable salt or solvate thereof:

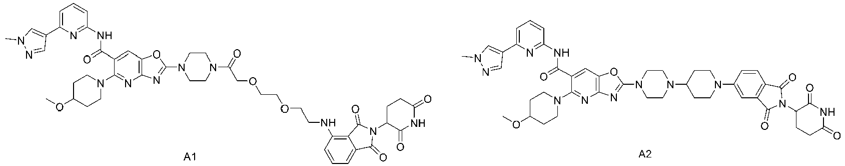

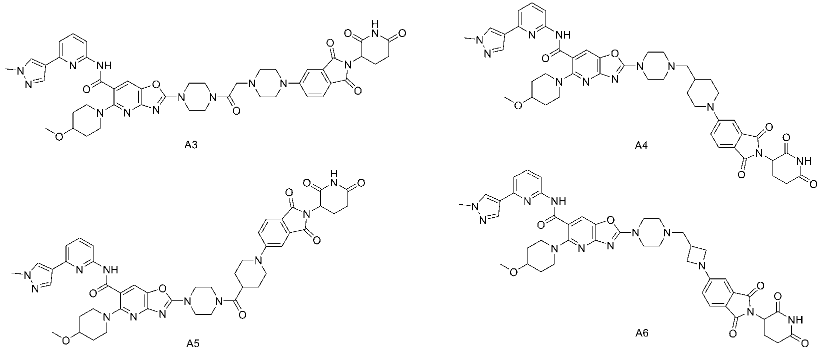

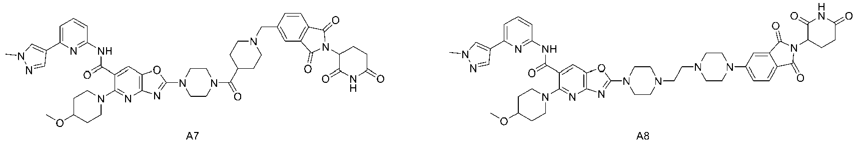

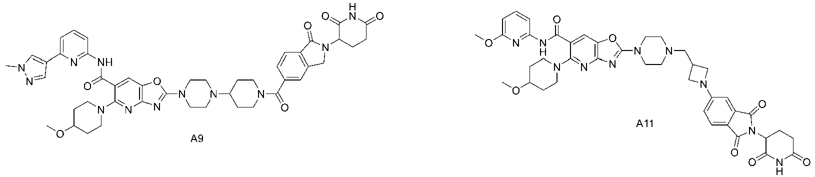

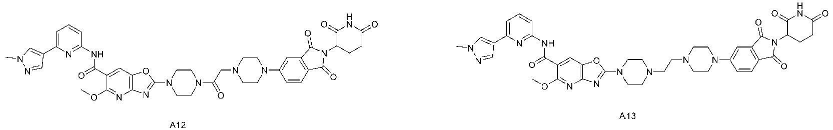

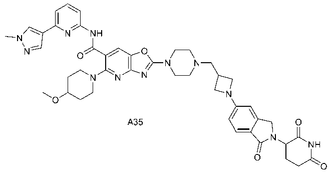

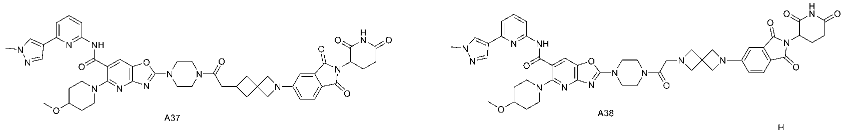

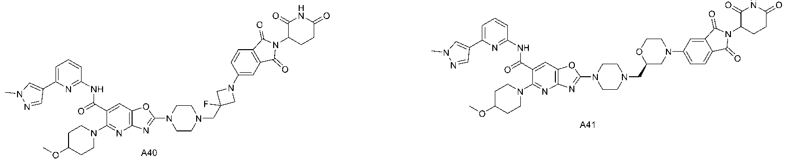

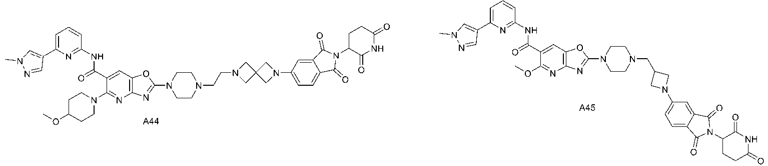

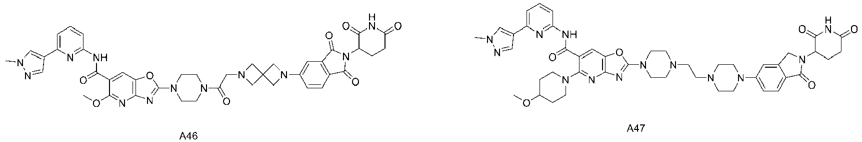

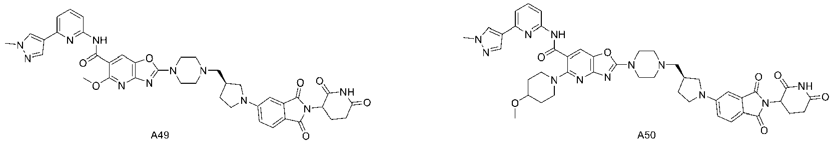

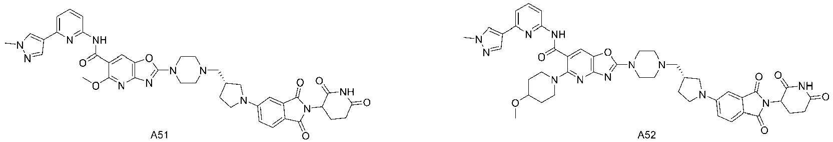

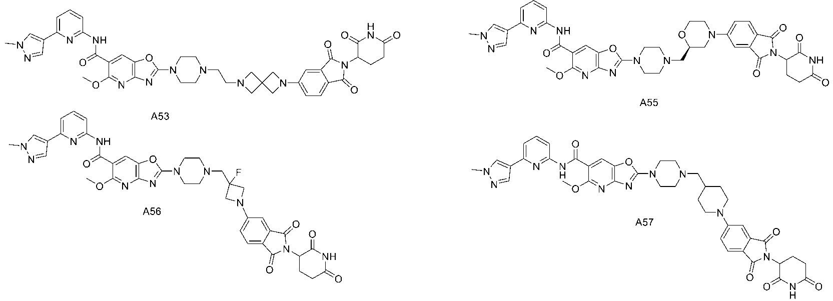

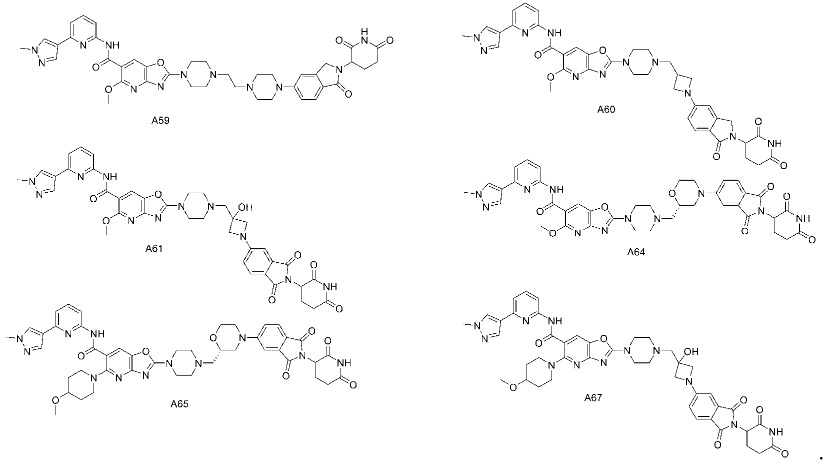

### Third Aspect

The present disclosure provides a pharmaceutical composition comprising the compound according to the First Aspect or the Second Aspect, or the pharmaceutically acceptable salt or solvate thereof.

Further, said pharmaceutical composition further comprises at least one pharmaceutically acceptable carrier.

### Fourth Aspect

The present disclosure provides a pharmaceutical combination comprising the compound according to the First Aspect or the Second Aspect, or the pharmaceutically acceptable salt or solvate thereof, or the pharmaceutical composition according to the Third Aspect.

Further, said pharmaceutical combination further comprises at least one additional agent for the prophylaxis and/or treatment of a disease and/or disorder responsive to IRAK4 and/or FLT3.

### Fifth Aspect

The present disclosure provides use of the compound or a pharmaceutically acceptable salt or solvate thereof according to the First Aspect or the Second Aspect, the pharmaceutical composition according to the Third Aspect, or the pharmaceutical combination according to the Fourth Aspect, in the manufacture of a medicament for the prophylaxis and/or treatment of the disease and/or disorder at least partially responsive to IRAK4 and/or FLT3.

Further, said disease and/or disorder include: lymphoma, leukemia, myelodysplastic syndrome, endometriosis, atopic dermatitis, hidradenitis suppurativa, psoriasis, systemic lupus erythematosus, multiple sclerosis, or rheumatoid arthritis.

### Sixth Aspect

The present disclosure provides the compound or a pharmaceutically acceptable salt or solvate thereof according to the First Aspect or the Second Aspect, the pharmaceutical composition according to the Third Aspect, or the pharmaceutical combination according to the Fourth Aspect, for use in the prophylaxis and/or treatment of the disease and/or disorder at least partially responsive to IRAK4 and/or FLT3.

Further, said disease and/or disorder include: lymphoma, leukemia, myelodysplastic syndrome, endometriosis, atopic dermatitis, hidradenitis suppurativa, psoriasis, systemic lupus erythematosus, multiple sclerosis, or rheumatoid arthritis.

### Seventh Aspect

The present disclosure provides a method for the prophylaxis and/or treatment of the disease and/or disorder at least partially responsive to IRAK4 and/or FLT3, comprising the step of: administering a prophylactically and/or therapeutically effective amount of the compound or the pharmaceutically acceptable salt or solvate thereof according to the First Aspect or the Second Aspect, the pharmaceutical composition according to the Third Aspect, or the pharmaceutical combination according to the Fourth Aspect, to a patient in need thereof.

Further, said disease and/or disorder include: lymphoma, leukemia, myelodysplastic syndrome, endometriosis, atopic dermatitis, hidradenitis suppurativa, psoriasis, systemic lupus erythematosus, multiple sclerosis, or rheumatoid arthritis.

### Effects of the disclosure

The compound having a dual degradation activity against IRAK4 and FLT3 proteins of the present disclosure can be used for the prophylaxis and/or treatment of the disease and/or disorder at least partially responsive to IRAK4 and/or FLT3.

### Brief Description of the Drawings

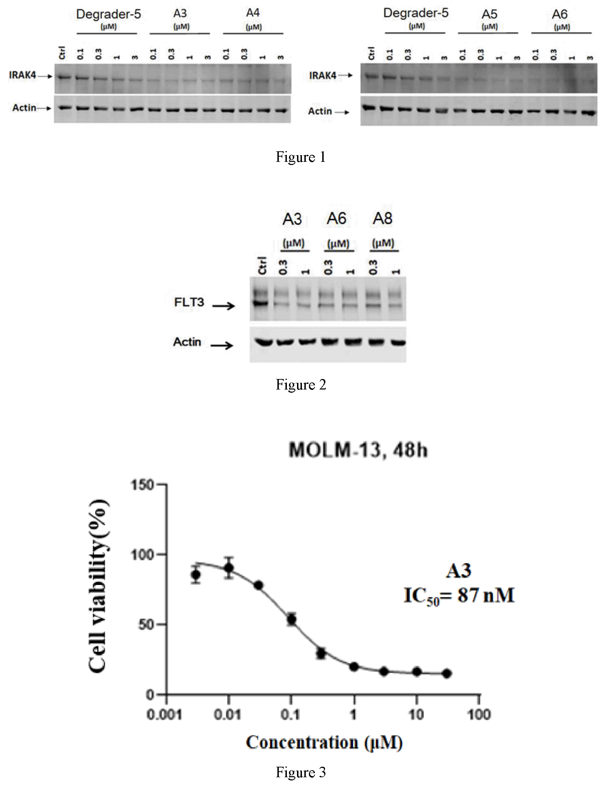

Figure 1 shows the Western blot results of IRAK4 protein degradation by Compounds A3, A4, A5 and A6 in THP-1 cells.
Figure 2 shows the Western blot results of FLT3 protein degradation by Compounds A3, A6 and A8 in MOLM-13 cells.
Figure 3 shows the inhibition curve of Compound A3 on the proliferation of MOLM-13 tumor cells.

### Detailed Description

For a clearer understanding of the technical features, purposes and beneficial effects of the present disclosure, the technical solutions of the present disclosure are described in detail below, which shall not be construed as limiting the scope of implementation of the present disclosure.

To describe the subject matter of the present disclosure more clearly, the terms used herein are defined as follows:
The term "halogen", alone or in combination, refers to fluorine, chlorine, bromine or iodine, in particular fluorine, chlorine or bromine.

The term "C₁₋₆ alkyl", alone or in combination, refers to a saturated linear or branched alkyl group containing 1 to 6 (especially 1 to 3) carbon atoms, including but not limited to methyl, ethyl, propyl, isopropyl, butyl, sec-butyl, isobutyl, tert-butyl, n-pentyl, 2-pentyl, 3-pentyl, 2-methyl-2-butyl, 3-methyl-2-butyl, 3-methyl-1-butyl, 2-methyl-1-butyl, n-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, and the like. Preferably, "C₁₋₆ alkyl" is any one of methyl, ethyl, n-propyl, isopropyl and tert-butyl. Similarly, the term "C₁₋₃ alkyl", alone or in combination, refers to a saturated linear or branched alkyl group containing 1 to 3 carbon atoms, including methyl, ethyl, propyl, isopropyl, and the like.

The term "OC₁₋₆ alkyl", alone or in combination, refers to a "C₁₋₆ alkyl-O-" group, wherein "C₁₋₆ alkyl" is as defined above, including but not limited to methoxy (-OCH₃), ethoxy (-OCH₂CH₃), n-propoxy (-OCH₂CH₂CH₃), isopropoxy (-OCH(CH₃)₂), n-butoxy (-OCH₂CH₂CH₂CH₃), sec-butoxy (-OCH(CH₃)CH₂CH₃), isobutoxy (-OCH₂CH(CH₃)₂), tert-butoxy (-OC(CH₃)₃), n-pentoxy (-OCH₂CH₂CH₂CH₂CH₃), neopentoxy (-OCH₂C(CH₃)₃), and the like.

The term "C₃₋₆ cycloalkyl", alone or in combination, refers to a saturated or partially unsaturated monocyclic or polycyclic cycloalkyl group containing 3 to 6 (especially 3 to 5) carbon atoms, including but not limited to cyclopropyl, cyclobutyl, cyclopentyl, cyclohexyl, and the like.

The term "3- to 8-membered heterocyclyl", alone or in combination, refers to a saturated or partially unsaturated monocyclic heterocyclyl containing 3 to 8 (especially 3 to 6, more particularly 5 or 6) ring-forming carbon atoms and heteroatoms or heteroatomic groups selected from O, S, NH, N, P(=O), S(=O) and S(=O)₂. "3- to 8-membered heterocyclyl" includes but is not limited to aziridinyl, azetidinyl, oxetanyl, pyrrolidinyl, tetrahydrofuranyl, tetrahydrothienyl, piperidinyl, morpholinyl, piperazinyl, thiomorpholinyl, tetrahydropyranyl, 1,1-dioxothiomorpholinyl, butyrolactamyl, valerolactamyl, caprolactamyl, butyrolactonyl, valerolactonyl, caprolactonyl, and the like.

The term "6- to 10-membered bridged heterobicyclyl", alone or in combination, refers to a saturated or partially unsaturated bridged heterobicyclic group containing 6 to 10 (especially 6 to 8) ring-forming carbon atoms and heteroatoms or heteroatomic groups selected from O, S, NH, N, P(=O), S(=O) and S(=O)₂. "6- to 10-membered bridged heterobicyclyl" includes but is not limited to 3,6-diazabicyclo[3.1.1]hept-1-yl, 3,6-diazabicyclo[3.1.1]hept-3-yl, 3,6-diazabicyclo[3.1.1]hept-6-yl, and the like.

The term "5- to 12-membered spiroheterobicyclyl", alone or in combination, refers to a saturated or partially unsaturated bicyclic group containing 5 to 12 (especially 6 to 9) ring-forming carbon atoms and heteroatoms or heteroatomic groups selected from O, S, NH, N, P(=O), S(=O) and S(=O)₂, wherein the two rings are connected via a single carbon atom. "5- to 12-membered spiroheterobicyclyl" includes but is not limited to 4,7-diazaspiro[2.5]oct-7-yl, 1-oxa-6-azaspiro[3.3]hept-6-yl, 2-oxa-6-azaspiro[3.3]hept-6-yl, 2,5-diazaspiro[3.4]oct-2-yl, 5-oxa-2-azaspiro[3.4]oct-2-yl, 6-oxa-2-azaspiro[3.4]oct-2-yl, 1-oxa-7-azaspiro[3.5]non-7-yl, and the like.

The term "6- to 12-membered fused heterobicyclyl", alone or in combination, refers to a saturated or partially unsaturated bicyclic group containing 6 to 12 (especially 7 to 10) ring-forming carbon atoms and heteroatoms or heteroatomic groups selected from O, S, NH, N, P(=O), S(=O) and S(=O)₂, wherein the two rings share a C-C bond, C-heteroatom bond or heteroatom-heteroatom bond. "6- to 12-membered fused heterobicyclyl" includes but is not limited to 3,7-diazabicyclo[3.3.0]oct-3-yl, 3,6-diazabicyclo[3.3.0]oct-3-yl, 2,7-diazabicyclo[3.3.0]oct-2-yl, and the like.

The term "C₆₋₁₀ aryl", alone or in combination, refers to any stable 6- to 10-membered monocyclic or bicyclic aromatic group, including but not limited to phenyl, naphthyl, tetrahydronaphthyl, indanyl, biphenyl, and the like.

The term "5- to 10-membered heteroaryl", alone or in combination, refers to any stable 5- to 10-membered monocyclic or bicyclic aromatic group in which one or more ring carbon atoms are replaced by heteroatoms or heteroatomic groups selected from O, S, NH, N, P(=O), S(=O) and S(=O)₂, including but not limited to thienyl, pyridyl, pyrimidinyl, pyrazinyl, pyridazinyl, pyridin-2(1H)-onyl, pyridin-4(1H)-onyl, pyrrolyl, pyrazolyl, thiazolyl, 1,2,3-triazolyl, 1,2,4-triazolyl, imidazolyl, tetrazolyl, isothiazolyl, oxazolyl, isoxazolyl, thiadiazolyl, oxadiazolyl, benzothienyl, indolyl, benzimidazolyl, benzothiazolyl, benzofuranyl, quinolinyl, isoquinolinyl, quinazolinyl, and the like.

The term "7- to 10-membered fused heterobicyclyl", alone or in combination, refers to a fused bicyclic group formed by fusion of one aryl and one heteroaryl, or two heteroaryl groups, including but not limited to benzothienyl, indolyl, benzimidazolyl, pyridoimidazolyl, pyridazinoimidazolyl, pyrazolopyrimidinyl, benzothiazolyl, benzofuranyl, quinolinyl, isoquinolinyl, quinazolinyl, and the like.

The term "alkenyl", alone or in combination, refers to an alkyl group as defined above composed of at least two carbon atoms and at least one carbon-carbon double bond. "C₂₋₁₂ alkenyl" refers to a linear or branched alkenyl group having 2 to 12 carbon atoms, including but not limited to vinyl, 1-propenyl, 1-butenyl, and the like, preferably "C₂₋₆ alkenyl".

The term "alkynyl", alone or in combination, refers to an alkyl group as defined above composed of at least two carbon atoms and at least one carbon-carbon triple bond. "C₂₋₁₂ alkynyl" refers to a linear or branched alkynyl group having 2 to 12 carbon atoms, including but not limited to ethynyl, 1-propynyl, 1-butynyl, and the like, preferably "C₂₋₆ alkynyl".

The term "diyl", alone or in combination, refers to a divalent group. For example, alkylene refers to divalent alkyl, alkenylene refers to divalent alkenyl, alkynylene refers to divalent alkynyl, cycloalkylene refers to divalent cycloalkyl, heterocycloalkylene refers to divalent heterocyclyl, arylene refers to divalent aryl, and heteroarylene refers to divalent heteroaryl, wherein alkyl, alkenyl, alkynyl, cycloalkyl, heterocyclyl, aryl and heteroaryl are as defined above.

The term "amino", alone or in combination, refers to primary amino (-NH₂), secondary amino (-NH-) or tertiary amino.

The terms "NH(C₁₋₆ alkyl)", "N(C₁₋₆ alkyl)₂", "NH(C₃₋₆ cycloalkyl)", "N(C₃₋₆ cycloalkyl)₂", alone or in combination, refer to an amino group as defined above wherein one or two hydrogen atoms are independently replaced by one or two C₁₋₆ alkyl or C₃₋₆ cycloalkyl groups, respectively, wherein "C₁₋₆ alkyl" and "C₃₋₆ cycloalkyl" are as defined above.

The term "pharmaceutically acceptable salt" refers to a compound of the present disclosure in the form of a pharmaceutically acceptable salt thereof, including acid addition salts and base addition salts. Pharmaceutically acceptable salts are described in Pharmaceutically Acceptable Salts by S. M. Berge et al., Journal of Pharmaceutical Sciences, 1977, 66, 1-19. In the present disclosure, a pharmaceutically acceptable non-toxic acid addition salt refers to a salt formed by a compound of the present disclosure with an organic or inorganic acid, including but not limited to hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, p-toluenesulfonic acid, malic acid, and the like. A pharmaceutically acceptable non-toxic base addition salt refers to a salt formed by a compound of the present disclosure with an organic or inorganic base, including but not limited to alkali metal salts such as lithium, sodium or potassium salts; alkaline earth metal salts such as calcium or magnesium salts; and salts with organic bases such as ammonium salts or N⁺(C₁₋₆ alkyl)₄ salts formed with nitrogen-containing organic bases, preferably lithium hydroxide, sodium hydroxide, potassium hydroxide, sodium carbonate, sodium bicarbonate, potassium carbonate, potassium bicarbonate, magnesium carbonate, calcium carbonate, aqueous ammonia, triethylamine, tetrabutylammonium hydroxide, and the like. "Pharmaceutically acceptable salts" can be synthesized by conventional chemical methods.

The term "solvate" refers to an association complex formed by one or more solvent molecules with a compound of the present disclosure. Solvents that form solvates include but are not limited to water, methanol, ethanol, isopropanol, ethyl acetate, tetrahydrofuran, N,N-dimethylformamide, dimethyl sulfoxide, and the like.

The term "each independently" means that at least two groups (or ring systems) having the same or similar variable scope present in a structure may have identical or different meanings under a given circumstance. For example, if substituent X and substituent Y are each independently hydrogen, halogen, hydroxy, cyano, alkyl or aryl, then when X is hydrogen, Y may be hydrogen, halogen, hydroxy, cyano, alkyl or aryl; similarly, when Y is hydrogen, X may be hydrogen, halogen, hydroxy, cyano, alkyl or aryl.

The term "optional" or "optionally" means that the subsequently described event or circumstance may but need not occur, and that the description includes instances where such event or circumstance occurs and instances where it does not.

The term "prevention" refers to complete or nearly complete inhibition of the onset of a disease or condition (e.g., infection, ischemia or reperfusion injury) when a patient or subject is susceptible to or at risk of the disease or condition; prevention may also include suppression, i.e., arresting the development of the condition.

The term "treatment" refers to:
1) inhibiting the disease, e.g., inhibiting the disease, condition or disorder in an individual experiencing or exhibiting pathology or symptomatology of the disease, condition or disorder (i.e., arresting further development of the pathology and/or symptomatology); or
2) alleviating the disease, e.g., ameliorating the disease, condition or disorder in an individual experiencing or exhibiting pathology or symptomatology of the disease, condition or disorder (i.e., reversing the pathology and/or symptomatology).

The term "additional" refers to a difference or distinction relative to a substance, element or object described in the present disclosure; for example, one or more additional drugs refer to one or more drugs having a different structure, composition or mechanism of action from the substances of the present disclosure.

The term "effective amount" refers to an amount of an active compound or agent that elicits a biological or medical response in a tissue, system, animal, individual or human, as sought by a researcher, veterinarian, physician or other clinician.

The experimental methods described in the following Preparation Examples are conventional methods unless otherwise specified; the reagents and materials used are commercially available unless otherwise specified.

In the following Preparation Examples, all solvents and chemicals used were of analytical grade or chemical grade; solvents were redistilled before use; anhydrous solvents were treated according to standard or literature methods.

Column chromatography silica gel (100-200 mesh) and thin-layer chromatography silica gel (GF254) were products of Qingdao Marine Chemical Factory and Yantai Chemical Factory. Unless otherwise specified, petroleum ether (60-90 °C)/ethyl acetate (v/v) was used as the eluent. Iodine or ethanolic phosphomolybdic acid solution was used as the developing agent.

All extraction solvents were dried over anhydrous Na₂SO₄ unless otherwise stated.
¹H-NMR spectra were recorded on a Varian-300 nuclear magnetic resonance spectrometer with TMS as the internal standard.

LC-MS spectra were recorded on an Agilent 1100 high-performance liquid chromatography-ion trap mass spectrometer (LC-MSD Trap) equipped with a diode array detector (DAD) at detection wavelengths of 214 nm and 254 nm, using an ESI ion source.

HPLC analysis was performed on an Agela Durashell C18 column (4.6 × 50 mm, 3.5 µm); mobile phase: 0.1% aqueous NH₄HCO₃:acetonitrile (from 5:95 to 95:5, v/v, within 5 min); flow rate: 1.8 mL/min.

The following abbreviations may be used in the present disclosure:
NBS: N-bromosuccinimide;
T₃P: n-propylphosphonic anhydride;
DMF: N,N-dimethylformamide;
LDA: lithium diisopropylamide;
DCE: 1,2-dichloroethane;
DCM: dichloromethane;
TEA: triethylamine;
EA: ethyl acetate;
THF: tetrahydrofuran;
TFA: trifluoroacetic acid;
TKB: potassium tert-butoxide;
DAST: diethylaminosulfur trifluoride;
DMEDA: N,N-dimethylethylenediamine;
EG: ethylene glycol;
Xantphos: 4,5-bis(diphenylphosphino)-9,9-dimethylxanthene;
HATU: N,N,N',N'-tetramethyl-O-(7-azabenzotriazol-1-yl)uronium hexafluorophosphate;
DIPEA: N,N-diisopropylethylamine;
Pd(dppf)Cl₂: [1,1'-bis(diphenylphosphino)ferrocene]palladium(II) dichloride;
Pd(OAc)₂: palladium(II) acetate;
DPPA: diphenylphosphoryl azide;
STAB: sodium triacetoxyborohydride;
DMSO: dimethyl sulfoxide;
DIBAL-H: diisobutylaluminum hydride;
DCC: N,N'-dicyclohexylcarbodiimide.

### Synthesis intermediate P-1

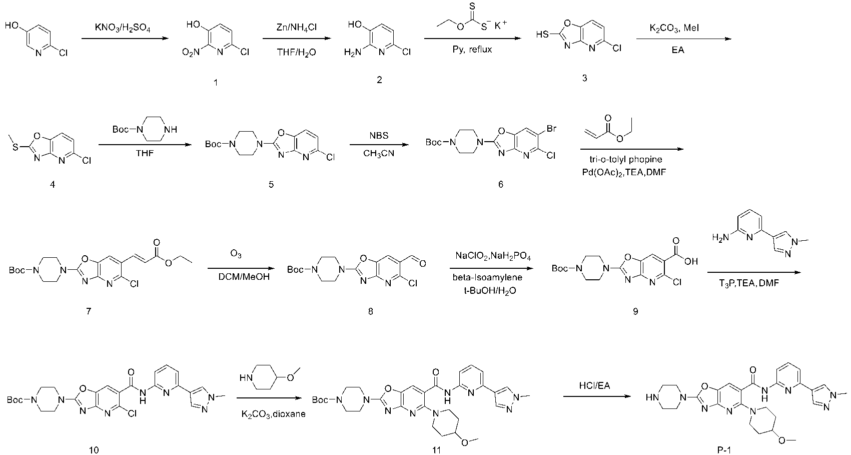

### Synthesis of Intermediate 1

2-Chloro-5-hydroxypyridine (12 g, 96 mmol) was dissolved in concentrated sulfuric acid (150 mL). Potassium nitrate (12 g, 116 mmol) was added portionwise at 0 °C. After the addition was completed over 2 hours, the mixture was slowly warmed to room temperature and stirred for 3 hours. Upon completion of the reaction, the mixture was slowly added dropwise into ice water, and a yellow solid precipitated. Filtration afforded yellow Intermediate 1 (15.4 g, 91%). ¹H-NMR (300 MHz, DMSO-d₆): δ 11.94 (s, 1H), 7.76 (d, J = 8.4 Hz, 1H), 7.71 (d, J = 8.4 Hz, 1H).

### Synthesis of Intermediate 2

Intermediate 1 (14 g, 83 mmol) was dissolved in a mixed solvent of tetrahydrofuran (300 mL)/water (100 mL). Zinc powder (44 g, 669 mmol) and ammonium chloride (89 g, 1673 mmol) were added portionwise at 50 °C. After stirring for 1 hour, the mixture was cooled to room temperature, diluted with ethyl acetate (200 mL), and water (150 mL) was added. After stirring, the layers were separated, filtered, and the organic phase was collected, dried over anhydrous sodium sulfate, and purified by silica gel column chromatography (petroleum ether/ethyl acetate = 2/1, v/v) to afford yellow Intermediate 2 (11 g, 94%).

¹H-NMR (300 MHz, DMSO-d₆): δ 9.72 (s, 1H), 6.82 (d, J = 7.8 Hz, 1H), 6.37 (d, J = 7.8 Hz, 1H), 5.88 (s, 2H).

LC-MS: m/z 145.1 [M+H]⁺.

### Synthesis of Intermediate 3

Intermediate 2 (23 g, 162 mmol) and potassium ethyl xanthate (39 g, 243 mmol) were dissolved in pyridine (100 mL) and refluxed at 120 °C for 4 hours. After cooling to room temperature, pyridine was removed by evaporation. The residue was treated with ice water (100 mL) and adjusted to pH 1 with 3 N dilute hydrochloric acid. A gray-white solid precipitated, which was stirred for 1 hour and filtered to afford gray-white Intermediate 3 (29 g, 96%).

¹H-NMR (300 MHz, DMSO-d₆): δ 7.91 (d, J = 8.4 Hz, 1H), 7.35 (d, J = 8.4 Hz, 1H).

LC-MS: m/z 187.1 [M+H]⁺.

### Synthesis of Intermediate 4

Intermediate 3 (29 g, 155 mmol) was dissolved in ethyl acetate (300 mL). Potassium carbonate (43 g, 313 mmol) was added, followed by iodomethane (20 mL, 311 mmol) at room temperature. After stirring for 2 hours, water (200 mL) was added. The layers were separated, and the organic phase was collected, dried over anhydrous sodium sulfate, and concentrated to afford gray solid Intermediate 4 (28 g, 91%).

¹H-NMR (300 MHz, DMSO-d₆): δ 8.14 (d, J = 8.4 Hz, 1H), 7.42 (d, J = 8.4 Hz, 1H), 2.79 (s, 3H).

### Synthesis of Intermediate 5

Intermediate 4 (2.0 g, 10 mmol) and tert-butyl piperazine-1-carboxylate (5.0 g, 50 mmol) were dissolved in tetrahydrofuran (70 mL) and stirred at 80 °C overnight. The reaction mixture was concentrated and purified by silica gel column chromatography (petroleum ether/ethyl acetate = 4/1, v/v) to afford white solid Intermediate 5 (3.0 g, 72%).

¹H-NMR (300 MHz, CDCl₃): δ 7.37 (d, J = 8.1 Hz, 1H), 6.93 (d, J = 8.1 Hz, 1H), 3.77-3.67 (m, 4H), 3.64-3.51 (m, 4H), 1.48 (s, 9H).

### Synthesis of Intermediate 6

Intermediate 5 (3.0 g, 8.8 mmol) was dissolved in dry acetonitrile (100 mL). NBS (2.2 g, 12.3 mmol) was slowly added portionwise in an ice-water bath. The mixture was stirred at room temperature for 5 hours, diluted with ethyl acetate (200 mL), washed with 1 N aqueous sodium hydroxide (50 mL × 4), dried over anhydrous sodium sulfate, and concentrated to afford brown solid Intermediate 6 (3.0 g, 83%).

¹H-NMR (300 MHz, CDCl₃): δ 7.68 (s, 1H), 3.77-3.71 (m, 4H), 3.62-3.55 (m, 4H), 1.49 (s, 9H).

### Synthesis of Intermediate 7

Intermediate 6 (1 g, 2.4 mmol), ethyl acrylate (4.2 g, 48 mmol), palladium acetate (54 mg, 0.24 mmol), tri(o-tolyl)phosphine (220 mg, 0.72 mmol) and triethylamine (726 mg, 7.2 mmol) were suspended in dry N,N-dimethylformamide (5 mL). The mixture was subjected to microwave reaction at 140 °C for 2 hours under nitrogen protection. The reaction was diluted with water (10 mL), extracted with ethyl acetate (20 mL × 3), washed with saturated brine, concentrated, and purified by silica gel column chromatography (petroleum ether/ethyl acetate = 2/1, v/v) to afford yellow solid Intermediate 7 (460 mg, 46%).

¹H-NMR (300 MHz, CDCl₃): δ 8.07 (d, J = 15.9 Hz, 1H), 7.64 (s, 1H), 6.30 (d, J = 15.9 Hz, 1H), 4.33-4.22 (m, 2H), 3.82-3.71 (m, 4H), 3.64-3.52 (m, 4H), 1.49 (s, 9H), 1.35 (t, J = 5.7 Hz, 3H). LC-MS: m/z 437.1 [M+H]⁺.

### Synthesis of Intermediate 8

Intermediate 7 (450 mg, 1.0 mmol) was dissolved in a mixed solvent of dichloromethane/methanol (30 mL/3 mL). Ozone was bubbled through the solution at -78 °C for 5 minutes, then dimethyl sulfide (0.5 mL) was added to quench the reaction. The mixture was warmed to room temperature, concentrated, and purified by silica gel column chromatography (petroleum ether/ethyl acetate = 3/1, v/v) to afford pale yellow solid Intermediate 8 (250 mg, 68%).

¹H-NMR (300 MHz, CDCl₃): δ 10.37 (s, 1H), 7.91 (s, 1H), 3.88-3.73 (m, 4H), 3.66-3.52 (m, 4H), 1.48 (s, 9H).

LC-MS: m/z 367.1 [M+H]⁺.

### Synthesis of Intermediate 9

Intermediate 8 (50 mg, 0.13 mmol) was dissolved in tert-butanol/water (5 mL/5 mL). 2-Methyl-2-butene (91 mg, 1.3 mmol) was added at room temperature, followed by a slow addition of an aqueous solution (5 mL) of sodium dihydrogen phosphate dihydrate (202 mg, 1.3 mmol) and sodium chlorite (59 mg, 0.65 mmol). After stirring at room temperature for 1 hour, the mixture was diluted with water (30 mL), adjusted to pH 3-4 with 1 N hydrochloric acid, extracted with dichloromethane (20 mL × 3). The organic phases were combined, washed with saturated brine (20 mL × 3), and concentrated to afford white solid Intermediate 9 (40 mg, 77%).

¹H-NMR (300 MHz, DMSO-d₆): δ 8.14 (s, 1H), 3.78-3.63 (m, 4H), 3.60-3.46 (m, 4H), 1.44 (s, 9H).

LC-MS: m/z 383.1 [M+H]⁺.

### Synthesis of Intermediate 10

Intermediate 9 (40 mg, 0.1 mmol) was dissolved in dry N,N-dimethylformamide (2 mL). Triethylamine (69 mg, 0.5 mmol) and 2-amino-6-(1-methyl-1H-pyrazol-4-yl)pyridine (34 mg, 0.2 mmol) were added, followed by 50% T₃P (159 mg, 0.5 mmol) in N,N-dimethylformamide under stirring. The mixture was stirred under microwave at 100 °C for 2 hours. The reaction was poured into ice water (10 mL), and the product precipitated. After stirring for 30 minutes, the mixture was filtered, washed with water, and dried to afford pale yellow solid Intermediate 10 (10 mg, 18%).

¹H-NMR (300 MHz, DMSO-d₆): δ 10.92 (s, 1H), 8.18 (s, 1H), 8.08 (s, 1H), 7.98-7.91 (m, 2H), 7.85-7.75 (m, 1H), 7.46-7.35 (m, 1H), 3.87 (s, 3H), 3.75-3.63 (m, 4H), 3.57-3.48 (m, 4H), 1.43 (s, 9H).

### Synthesis of Intermediate 11

Intermediate 10 (80 mg, 0.15 mmol), 4-methoxypiperidine (172 mg, 1.5 mmol) and potassium carbonate (103 mg, 0.75 mmol) were dissolved in dry 1,4-dioxane (5 mL) and stirred at 110 °C for 24 hours. The mixture was filtered, the filtrate was concentrated, and purified by preparative plate chromatography (dichloromethane/methanol = 20/1, v/v) to afford white solid Intermediate 11 (45 mg, 49%).

¹H-NMR (300 MHz, DMSO-d₆): δ 13.46-13.31 (m, 1H), 8.35-8.20 (m, 2H), 8.10-7.99 (m, 2H), 7.78 (s, 1H), 7.39 (s, 1H), 3.87 (s, 3H), 3.76-3.63 (m, 9H), 3.25-3.13 (m, 5H), 3.10-2.93 (m, 2H), 2.25-1.98 (m, 4H), 1.43 (s, 9H).

LC-MS: m/z 618.3 [M+H]⁺.

### Synthesis of Final Product P-1

Intermediate 11 (30 mg, 0.05 mmol) was dissolved in 2.8 N HCl in ethyl acetate (5 mL) and stirred at room temperature overnight. The reaction mixture was concentrated. Dichloromethane (20 mL × 4) and water (20 mL) were added, and back-extraction was performed 4 times. The aqueous phase was collected, adjusted to pH 7-8 with saturated aqueous sodium bicarbonate in an ice-water bath, and extracted with dichloromethane (30 mL × 3). The organic phases were combined and concentrated to afford P-1 (21 mg, 81%).

¹H NMR (300 MHz, DMSO-d₆): δ 13.42 (s, 1H), 8.26 (s, 1H), 8.22 (s, 1H), 8.08-8.00 (m, 2H), 7.78 (t, J = 7.8 Hz, 1H), 7.38 (d, J = 7.5 Hz, 1H), 3.87 (s, 3H), 3.66-3.56 (m, 4H), 3.48-3.43 (m, 1H), 3.38 (s, 3H), 3.23-3.17 (m, 3H), 3.04-2.94 (m, 2H), 2.88-2.75 (m, 4H), 2.22-2.12 (m, 2H), 2.10-1.94 (m, 2H).

LC-MS: m/z 517.8 [M+H]⁺.

### Example 1: Synthesis of Compound A1

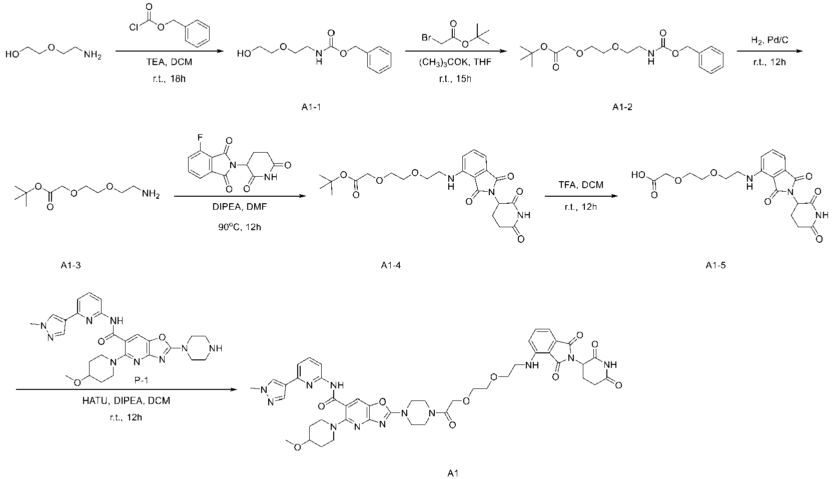

### Synthesis of Intermediate A1-1

Diethylene glycolamine (2.6 g, 25 mmol) and triethylamine (2.6 g, 25 mmol) were dissolved in dry dichloromethane (25 mL). A solution of benzyl chloroformate (4.3 g, 25 mmol) in dichloromethane (30 mL) was slowly added dropwise to the reaction system at 0 °C. The mixture was stirred at 0 °C for 2 hours, then at room temperature for 18 hours. The reaction was quenched with saturated aqueous NaHCO₃ (30 mL), extracted with dichloromethane three times (40 mL × 3). The organic phases were combined, dried over anhydrous sodium sulfate, and concentrated under reduced pressure to afford crude A1-1 (4.7 g, 79%), which was used directly in the next step.

### Synthesis of Intermediate A1-2

Intermediate A1-1 (500 mg, 2.1 mmol) and potassium tert-butoxide (255 mg, 2.1 mmol) were dissolved in dry tetrahydrofuran (10 mL). After stirring at 0 °C for 0.5 hour, tert-butyl bromoacetate (490 mg, 2.5 mmol) was slowly added dropwise. The mixture was stirred at 0 °C for 3 hours, then warmed to room temperature and stirred for 15 hours. The reaction was quenched with water (10 mL), extracted with ethyl acetate three times (15 mL × 3). The organic phases were combined, dried over anhydrous sodium sulfate, concentrated under reduced pressure, and the residue was purified by silica gel column chromatography (petroleum ether/ethyl acetate = 20/1, v/v) to afford pale yellow oil A1-2 (210 mg, 28%).

### Synthesis of Intermediate A1-3

Intermediate A1-2 (210 mg, 0.59 mmol) was dissolved in dry methanol (10 mL). Pd/C (200 mg) was added, and the mixture was stirred under hydrogen atmosphere at room temperature for 12 hours. The organic phase was filtered and concentrated under reduced pressure to afford colorless oil crude A1-3 (180 mg, 100%), which was used directly in the next step.

### Synthesis of Intermediate A1-4

### Intermediate A1-3 (665 mg, 2.4 mmol) and

2-(2,6-dioxopiperidin-3-yl)-4-fluoroisoindoline-1,3-dione (633 mg, 2.9 mmol, commercially available) were dissolved in dry DMF (10 mL). DIPEA (622 mg, 4.8 mmol) was added, and the mixture was stirred at 90 °C for 12 hours. The mixture was extracted with ethyl acetate three times (15 mL × 3), washed with saturated aqueous NaCl three times (20 mL × 3). The organic phases were combined, dried over anhydrous sodium sulfate, concentrated under reduced pressure, and the residue was purified by silica gel column chromatography (petroleum ether/ethyl acetate = 1/1, v/v) to afford yellow solid A1-4 (210 mg, 15%).

### Synthesis of Intermediate A1-5

Intermediate A1-4 (110 mg, 0.23 mmol) was dissolved in dry dichloromethane (5 mL). Trifluoroacetic acid (4 mL) was added, and the mixture was stirred at room temperature for 12 hours. The organic phase was concentrated under reduced pressure. Dry dichloromethane (5 mL) was added to the residue, and the mixture was concentrated again under reduced pressure to afford the crude product, which was used directly in the next step.

### Synthesis of Final Product A1

Intermediate A1-5 (97 mg, 0.23 mmol), Intermediate P-1 (63 mg, 0.23 mmol), HATU (43 mg, 0.35 mmol) and DIPEA (300 mg, 2.32 mmol) were dissolved in dry dichloromethane (3 mL). The mixture was stirred at room temperature for 12 hours. Water (3 mL) was added, and the mixture was extracted with ethyl acetate three times (10 mL × 3), washed with saturated aqueous NaCl three times (15 mL × 3). The organic phases were combined, dried over anhydrous sodium sulfate, concentrated under reduced pressure, and the residue was purified by silica gel column chromatography (dichloromethane/methanol = 20/1, v/v) to afford A1 (120 mg, 57%).

### Example 2: Synthesis of Compound A2

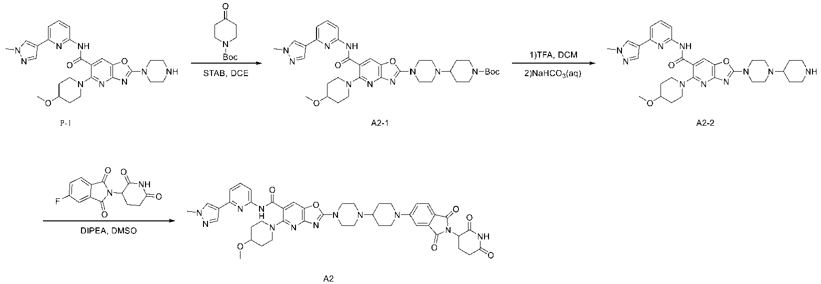

### Synthesis of Intermediate A2-1

P-1 (1.0 g, 1.9 mmol) and N-Boc-4-piperidone (950 mg, 4.8 mmol) were suspended in dry 1,2-dichloroethane (30 mL). After stirring at room temperature for 10 minutes, sodium triacetoxyborohydride (1.0 g, 4.8 mmol) was added, and the mixture was reacted at room temperature for 12 hours. Upon completion, the reaction was quenched with saturated aqueous sodium bicarbonate (30 mL), extracted with dichloromethane (50 mL × 2), washed with saturated brine (20 mL), dried over anhydrous sodium sulfate, and concentrated under reduced pressure. The residue was slurried in diethyl ether (40 mL) for 8 hours, filtered, washed with diethyl ether, and dried to afford white solid A2-1 (1.1 g, 81%).

¹H NMR (300 MHz, DMSO-d₆): δ 13.42 (s, 1H), 8.30-8.23 (m, 2H), 8.08-8.02 (m, 2H), 7.82-7.75 (m, 1H), 7.42-7.35 (m, 1H), 3.99-3.91 (m, 2H), 3.88 (s, 3H), 3.67 (s, 3H), 3.49-3.41 (m, 2H), 3.40-3.34 (m, 4H), 3.24-3.17 (m, 2H), 3.05-2.96 (m, 2H), 2.74-2.66 (m, 2H), 2.65-2.58 (m, 4H), 2.21-2.13 (m, 2H), 2.10-2.00 (m, 2H), 1.76-1.67 (m, 2H), 1.39 (s, 9H), 1.32-1.22 (m, 2H). LC-MS: m/z 700.8 [M+H]⁺.

### Synthesis of Intermediate A2-2

A2-1 (1.1 g, 1.6 mmol) was dissolved in dichloromethane (20 mL). Trifluoroacetic acid (2 mL) was added, and the mixture was stirred at room temperature for 8 hours. Upon completion, the solvent was evaporated, the residue was diluted with water (10 mL), adjusted to pH 7-8 with saturated aqueous sodium bicarbonate, extracted with dichloromethane (50 mL × 2), washed with saturated brine (20 mL), dried over anhydrous sodium sulfate, and concentrated to afford pale yellow solid A2-2 (860 mg, 91%).

¹H NMR (300 MHz, DMSO-d₆): δ 13.43 (s, 1H), 8.30-8.24 (m, 2H), 8.09-8.02 (m, 2H), 7.84-7.76 (m, 1H), 7.43-7.35 (m, 1H), 3.88 (s, 3H), 3.68 (s, 3H), 3.50-3.40 (m, 4H), 3.28-3.17 (m, 5H), 3.03-2.93 (m, 4H), 2.66-2.59 (m, 4H), 2.44-2.36 (m, 2H), 2.21-2.14 (m, 2H), 2.11-2.02 (m, 2H), 1.72-1.63 (m, 2H), 1.34-1.22 (m, 2H).

### Synthesis of Final Product A2

A2-2 (40 mg, 0.068 mmol) and 2-(2,6-dioxopiperidin-3-yl)-5-fluoroisoindoline-1,3-dione (23 mg, 0.081 mmol) were dissolved in DMSO (2 mL). DIPEA (45 mg, 0.35 mmol) was added, and the mixture was reacted at 95 °C for 6 hours. Water (5 mL) was added to precipitate a large amount of solid, which was filtered and dried. The filter cake was purified by silica gel column chromatography (dichloromethane/methanol = 30/1, v/v) to afford product A2 (21 mg, 36%).

### Example 3: Synthesis of Compound A3

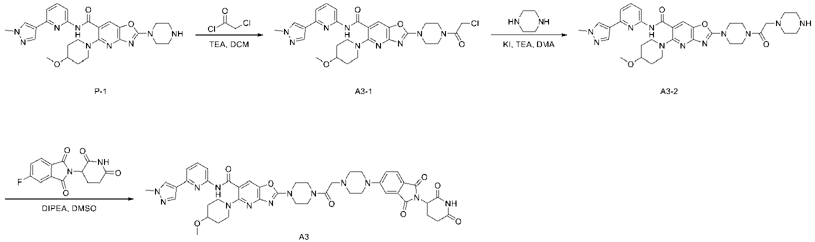

### Synthesis of Intermediate A3-1

P-1 (60 mg, 0.12 mmol) and triethylamine (23 mg, 0.057 mmol) were dissolved in dichloromethane (2.5 mL). Chloroacetyl chloride (17 mg, 0.038 mmol) was added dropwise in an ice-water bath, and the mixture was stirred for 2 hours. The reaction was quenched with water (10 mL) and extracted with dichloromethane (10 mL × 2). The organic phase was dried and concentrated to afford crude white solid A3-1 (60 mg).

¹H NMR (300 MHz, DMSO-d₆): δ 13.41 (s, 1H), 8.32-8.23 (m, 2H), 8.08-8.00 (m, 2H), 7.80 (t, *J =* 7.8 Hz, 1H), 7.40 (d, *J* = 7.5 Hz, 1H), 4.48 (s, 2H), 3.88 (s, 3H), 3.79-3.69 (m, 4H), 3.68-3.60 (m, 4H), 3.44-3.30 (m, 4H), 3.26-3.15 (m, 2H), 3.08-2.95 (m, 2H), 2.24-1.98 (m, 4H).

### Synthesis of Intermediate A3-2

A3-1 (40 mg, 0.070 mmol) and piperazine (29 mg, 0.35 mmol) were dissolved in DMA (5 mL). KI (5 mg, 0.030 mmol) was added at room temperature, and the mixture was stirred for 3 hours. Water (10 mL) was added to precipitate a white solid, which was filtered and dried to afford crude A3-2 (40 mg).

### Synthesis of Final Product A3

A3-2 (40 mg, 0.062 mmol) and 2-(2,6-dioxopiperidin-3-yl)-5-fluoroisoindoline-1,3-dione (23 mg, 0.074 mmol, commercially available) were dissolved in DMSO (2 mL). DIPEA (40 mg, 0.032 mmol) was added, and the mixture was heated at 95 °C for 6 hours. Water (5 mL) was added to afford a large amount of solid, which was filtered and dried. The filter cake was purified by silica gel column chromatography (dichloromethane/methanol = 30/1, v/v) to afford product A3 (17 mg, 29%).

### Example 4: Synthesis of Compound A4

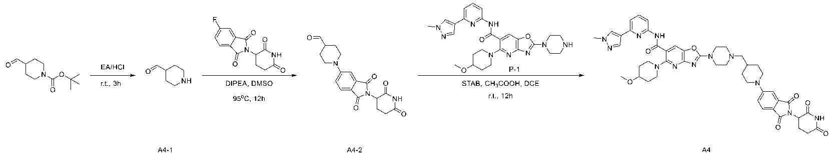

### Synthesis of Intermediate A4-1

1-(tert-Butoxycarbonyl)piperidine-4-carbaldehyde (350 mg, 1.6 mmol) was dissolved in dry ethyl acetate (5 mL). A 2.8 N HCl solution in ethyl acetate (10 mL) was added dropwise at 0 °C, and the mixture was stirred at room temperature for 3 hours. The reaction mixture was concentrated under reduced pressure. Dry dichloromethane (5 mL) was added to the residue, and the pH was adjusted to 7 with solid sodium bicarbonate powder. The mixture was filtered and concentrated under reduced pressure to afford crude A4-1 (185 mg, 99%), which was used directly in the next step.

### Synthesis of Intermediate A4-2

2-(2,6-Dioxopiperidin-3-yl)-5-fluoroisoindoline-1,3-dione (293 mg, 1.1 mmol, commercially available) and Intermediate A4-1 (180 mg, 1.6 mmol) were dissolved in dry dimethyl sulfoxide (5 mL). DIPEA (685 mg, 5.3 mmol) was added, and the mixture was stirred at 95 °C for 12 hours. The mixture was diluted with water, extracted with ethyl acetate three times (15 mL × 3), and washed with saturated aqueous NaCl solution three times (20 mL × 3). The combined organic phases were dried over anhydrous sodium sulfate and concentrated under reduced pressure. The residue was purified by silica gel column chromatography (dichloromethane/methanol = 25/1, v/v) to afford yellow solid A4-2 (55 mg, 14%).

¹H NMR (300 MHz, DMSO-d₆): δ 11.08 (s, 1H), 9.61 (s, 1H), 7.65 (d, *J* = 6.0 Hz, 1H), 7.48-7.12 (m, 2H), 5.19-4.98 (m, 1H), 4.16-3.82 (m, 2H), 3.29-3.07 (m, 2H), 2.97-2.82 (m, 1H), 2.77-2.52 (m, 4H), 2.08-1.86 (m, 2H), 1.72-1.42 (m, 2H).

LC-MS: *m*/*z* 402.1 [M+H+CH₃OH]⁺.

### Synthesis of Final Product A4

Intermediate A4-2 (55 mg, 0.15 mmol) and Intermediate P-1 (93 mg, 0.18 mmol) were dissolved in dry 1,2-dichloroethane (4 mL). One drop of acetic acid and activated molecular sieves (100 mg) were added, and the mixture was stirred at room temperature for 1 hour. Sodium triacetoxyborohydride (63 mg, 0.30 mmol) was added to the reaction mixture, which was then stirred at room temperature for 12 hours. The reaction was quenched with saturated aqueous NH₄Cl solution (1 mL), and the mixture was concentrated under reduced pressure. The residue was purified by silica gel column chromatography (dichloromethane/methanol = 20/1, v/v) to afford A4 (12 mg, 9%).

### Example 5: Synthesis of Compound A5

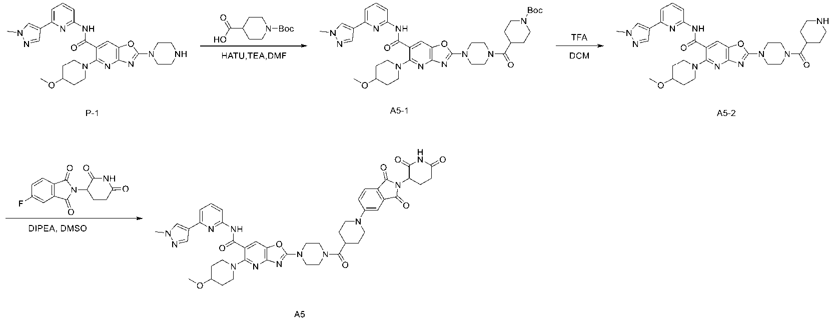

### Synthesis of Intermediate A5-1

P-1 (700 mg, 1.4 mmol), 1-(tert-butoxycarbonyl)piperidine-4-carboxylic acid (372 mg, 1.7 mmol), triethylamine (411 mg, 4.1 mmol) and HATU (792 mg, 2.1 mmol) were successively dissolved in DMF (15 mL), and the reaction was stirred at room temperature for 3 hours. Saturated brine (20 mL) was added, and the mixture was extracted with dichloromethane (30 mL × 2). The organic phase was dried, concentrated, and purified by silica gel column chromatography (dichloromethane/methanol = 40/1, v/v) to afford white solid A5-1 (800 mg, 78%).

LC-MS: *m*/*z* 315.2 [(M-Boc+2H)/2]²⁺.

### Synthesis of Intermediate A5-2

A5-1 (200 mg, 0.27 mmol) was dissolved in dichloromethane (5 mL). Trifluoroacetic acid (1.2 g, 11 mmol) was added at room temperature, and the reaction was stirred for 3 hours. The reaction mixture was concentrated, and a dichloromethane/methanol mixture (5 mL) was added. Saturated aqueous sodium bicarbonate was added dropwise in an ice-water bath to adjust the pH to 8. The mixture was extracted with dichloromethane (20 mL × 3) and concentrated to afford white solid A5-2 (60 mg, 36%).

¹H NMR (300 MHz, DMSO-d₆): δ 13.42 (s, 1H), 8.30 (s, 1H), 8.28 (s, 1H), 8.08-8.01 (m, 2H), 7.80 (t, *J* = 7.5 Hz, 1H), 7.40 (d, *J =* 7.5 Hz, 1H), 3.88 (s, 3H), 3.75-3.62 (m, 8H), 3.22 (s, 3H), 3.06-2.98 (m, 4H), 2.95-2.87 (m, 4H), 2.75-2.66 (m, 2H), 2.25-1.89 (m, 5H), 1.51-1.48 (m, 4H).

### Synthesis of Final Product A5

A5-2 (60 mg, 0.096 mmol) and 2-(2,6-dioxopiperidin-3-yl)-5-fluoroisoindoline-1,3-dione (31 mg, 0.14 mmol, commercially available) were dissolved in DMSO (2 mL). DIPEA (62 mg, 0.078 mmol) was added, and the mixture was stirred at 95 °C for 6 hours. Water (5 mL) was added to precipitate a large amount of solid, which was filtered and dried. The filter cake was purified by silica gel column chromatography (dichloromethane/methanol = 30/1, v/v) to afford product A5 (42 mg, 29%).

### Example 6: Synthesis of Compound A6

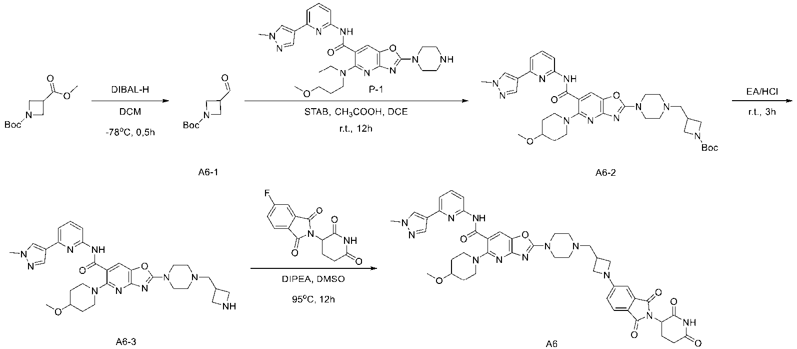

### Synthesis of Intermediate A6-1

Methyl 1-(tert-butoxycarbonyl)azetidine-3-carboxylate (800 mg, 3.7 mmol) was dissolved in dry DCM (5 mL). Under nitrogen protection, 1 N DIBAL-H in tetrahydrofuran (8.2 mL, 8.2 mmol) was added dropwise at -78 °C, and the mixture was stirred at -78 °C for 0.5 h. The reaction mixture was slowly warmed to 0 °C, and anhydrous methanol (2 mL) was added to quench the reaction. A 10% aqueous citric acid solution (30 mL) was added, and a white solid appeared. The mixture was stirred at 0 °C until the white solid dissolved. The mixture was extracted with dichloromethane three times (15 mL × 3). The combined organic phases were dried over anhydrous sodium sulfate, concentrated under reduced pressure, and purified by silica gel column chromatography (petroleum ether/ethyl acetate = 20/1, v/v) to afford colorless oil A6-1 (220 mg, 32%).

¹H NMR (300 MHz, CDCl₃): δ 9.84 (s, 1H), 4.14-3.94 (m, 2H), 3.81-3.64 (m, 2H), 3.52-3.44 (m, 1H), 1.43 (s, 9H).

### Synthesis of Intermediate A6-2

Intermediate P-1 (186 mg, 0.36 mmol) and Intermediate A6-1 (200 mg, 1.1 mmol) were dissolved in dry 1,2-dichloroethane (4 mL). One drop of acetic acid and activated molecular sieves (200 mg) were added, and the mixture was stirred at room temperature for 1 h. Sodium triacetoxyborohydride (229 mg, 1.1 mmol) was added, and the mixture was stirred at room temperature for 12 h. The reaction was quenched with saturated aqueous NH₄Cl solution (1 mL), concentrated under reduced pressure, and purified by silica gel column chromatography (dichloromethane/methanol = 30/1, v/v) to afford white solid A6-2 (140 mg, 53%).

¹H NMR (300 MHz, CDCl₃): δ 13.46 (s, 1H), 8.37 (s, 1H), 8.17 (s, 1H), 8.14 (d, *J* = 7.5 Hz, 1H), 7.93 (s, 1H), 7.68 (t, *J =* 7.8 Hz, 1H), 7.21 (d, *J =* 7.5 Hz, 1H), 4.10-3.99 (m, 2H), 3.96 (s, 3H), 3.89-3.70 (m, 4H), 3.67-3.54 (m, 2H), 3.47 (s, 3H), 3.45-3.39 (m, 1H), 3.39-3.26 (m, 2H), 3.25-3.07 (m, 2H), 2.81-2.70 (m, 1H), 2.70-2.61 (m, 2H), 2.60-2.47 (m, 4H), 2.39-2.13 (m, 4H), 1.44 (s, 9H).

LC-MS: m/z 687.4 [M+H]⁺.

### Synthesis of Intermediate A6-3

Intermediate A6-2 (140 mg, 0.21 mmol) was dissolved in dry ethyl acetate (2 mL). 2.8 N HCl in ethyl acetate (2 mL) was added, and the mixture was stirred at room temperature for 3 h. The reaction mixture was concentrated under reduced pressure. Dichloromethane (10 mL) and methanol (2 mL) were added to the residue, and the pH was adjusted to 7 with solid NaHCO₃ powder. The mixture was filtered and concentrated to afford white solid A6-3 (120 mg, 100%).

¹H NMR (300 MHz, CDCl₃): δ 13.45 (s, 1H), 8.37 (s, 1H), 8.26-8.06 (m, 2H), 7.93 (s, 1H), 7.68 (t, *J =* 7.5 Hz, 1H), 7.21 (d, *J =* 7.2 Hz, 1H), 3.96 (s, 3H), 3.90-3.70 (m, 4H), 3.69-3.54 (m, 2H), 3.53-3.45 (m, 4H), 3.40-3.26 (m, 2H), 3.25-3.13 (m, 2H), 3.09-2.89 (m, 6H), 2.75-2.66 (m, 1H), 2.64-2.48 (m, 3H), 2.29-2.18 (m, 2H), 2.09-1.98 (m, 2H).

LC-MS: m/z 587.3 [M+H]⁺.

### Synthesis of Final Product A6

Intermediate A6-3 (60 mg, 0.10 mmol) and 2-(2,6-dioxopiperidin-3-yl)-5-fluoroisoindoline-1,3-dione (37 mg, 0.13 mmol, commercially available) were dissolved in dry dimethyl sulfoxide (2 mL). DIPEA (66 mg, 0.51 mmol) was added, and the mixture was stirred at 95 °C for 12 h. Water (3 mL) was added to precipitate a yellow solid, which was filtered and purified by silica gel column chromatography (dichloromethane/methanol = 20/1, v/v) to afford A6 (50 mg, 58%).

### Example 7: Synthesis of Compound A7

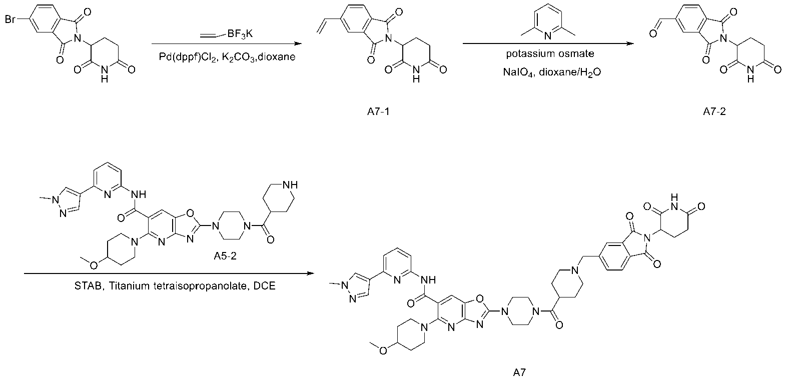

### Synthesis of Intermediate A7-1

5-Bromo-2-(2,6-dioxopiperidin-3-yl)isoindoline-1,3-dione (2.0 g, 5.9 mmol, commercially available), potassium vinyltrifluoroborate (2.4 g, 17.8 mmol), potassium carbonate (1.6 g, 11.8 mmol) and Pd(dppf)Cl₂ (428 mg, 0.59 mmol) were suspended in 1,4-dioxane (60 mL). After nitrogen purging, the mixture was stirred at 100 °C overnight. The reaction mixture was concentrated and purified by silica gel column chromatography to afford orange solid A7-1 (1.0 g, 62%).

¹H NMR (300 MHz, DMSO-d₆): δ 11.13 (s, 1H), 8.07 (s, 1H), 7.99-7.93 (m, 1H), 7.92-7.86 (m, 1H), 6.95 (dd, *J* = 17.4, 10.8 Hz, 1H), 6.20 (d, *J* = 17.4 Hz, 1H), 5.54 (d, *J* = 10.8 Hz, 1H), 5.21-5.10 (m, 1H), 2.98-2.81 (m, 1H), 2.65-2.60 (m, 1H), 2.59-2.54 (m, 1H), 2.13-2.00 (m, 1H).

### Synthesis of Intermediate A7-2

A7-1 (400 mg, 1.4 mmol) was dissolved in 1,4-dioxane/water (20 mL/10 mL). Potassium osmate dihydrate (52 mg, 0.14 mmol), sodium periodate (1.2 g, 5.6 mmol) and 2,6-lutidine (300 mg, 2.8 mmol) were added in one portion under ice-water bath, and the mixture was stirred for 5 hours. Sodium thiosulfate pentahydrate solution (10 mL) was added, and the mixture was extracted with dichloromethane (10 mL × 3). Purification by silica gel column chromatography afforded yellow solid A7-2 (80 mg, 20%).

LC-MS: *m*/*z* 285.1 [M-H]⁻.

### Synthesis of Final Product A7

A7-2 (33 mg, 0.12 mmol) and A5-2 (87 mg, 0.15 mmol) were dissolved in dry 1,2-dichloroethane (4 mL). Titanium(IV) isopropoxide (49 mg, 0.17 mmol) was added, and after 2 hours, sodium triacetoxyborohydride (96 mg, 0.46 mmol) was added. The mixture was stirred overnight. The reaction was quenched with saturated aqueous ammonium chloride solution (6 mL), extracted with dichloromethane (20 mL × 3), and purified by silica gel column chromatography (dichloromethane/methanol = 20/1, v/v) to afford A7 (26 mg, 24%).

### Example 8: Synthesis of Compound A8

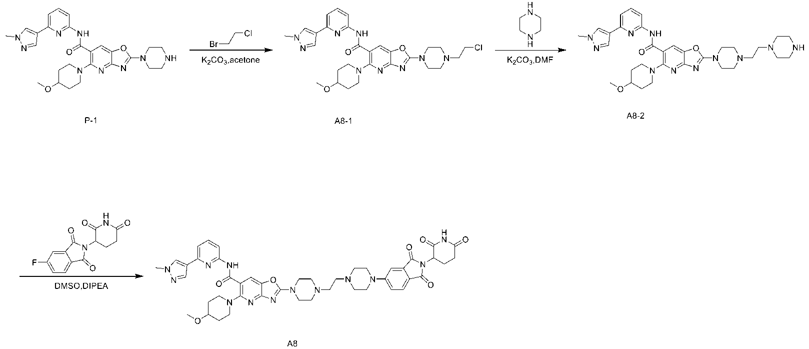

### Synthesis of Intermediate A8-1

P-1 (100 mg, 0.19 mmol) was dissolved in acetone (8 mL). 1-Bromo-2-chloroethane (42 mg, 0.29 mmol) and potassium carbonate (52 mg, 0.38 mmol) were added, and the mixture was stirred at 60 °C for 96 hours. The reaction mixture was concentrated under reduced pressure and purified by silica gel column chromatography to afford white solid Intermediate A8-1 (80 mg, 73%).

¹H NMR (300 MHz, DMSO-d₆): δ 13.43 (s, 1H), 8.35-8.20 (m, 2H), 8.13-7.96 (m, 2H), 7.88-7.76 (m, 1H), 7.45-7.35 (m, 1H), 3.87 (s, 3H), 3.83-3.59 (m, 5H), 3.30-3.12 (m, 6H), 3.01 (s, 3H), 2.84-2.55 (m, 6H), 2.23-1.98 (m, 4H).

LC-MS: *m*/*z* 580.2 [M+H]⁺.

### Synthesis of Intermediate A8-2

Intermediate A8-1 (60 mg, 0.10 mmol) was dissolved in dry N,N-dimethylformamide (8 mL). Piperazine (45 mg, 0.52 mmol) and potassium carbonate (71 mg, 0.52 mmol) were added, and the mixture was stirred at 80 °C for about 5 hours. The reaction mixture was cooled to room temperature, diluted with water (15 mL), extracted with ethyl acetate (20 mL × 3), and concentrated to afford pale yellow solid crude product A8-2 (65 mg, 100%), which was used directly in the next step.

LC-MS: *m*/*z* 630.4 [M+H]⁺.

### Synthesis of Final Product A8

Intermediate A8-2 (50 mg, 0.080 mmol) and 2-(2,6-dioxopiperidin-3-yl)-5-fluoroisoindoline-1,3-dione (29 mg, 0.11 mmol, commercially available) were dissolved in dimethyl sulfoxide (2 mL). N,N-Diisopropylethylamine (52 mg, 0.40 mmol) was added, and the mixture was stirred at 95 °C overnight. The reaction mixture was cooled to room temperature, and water (10 mL) was added to precipitate a solid, which was filtered. The filter cake was purified by silica gel column chromatography (dichloromethane/methanol = 20/1, v/v) to affordA8 (23 mg, 33%).

### Example 9: Synthesis of Compound A9

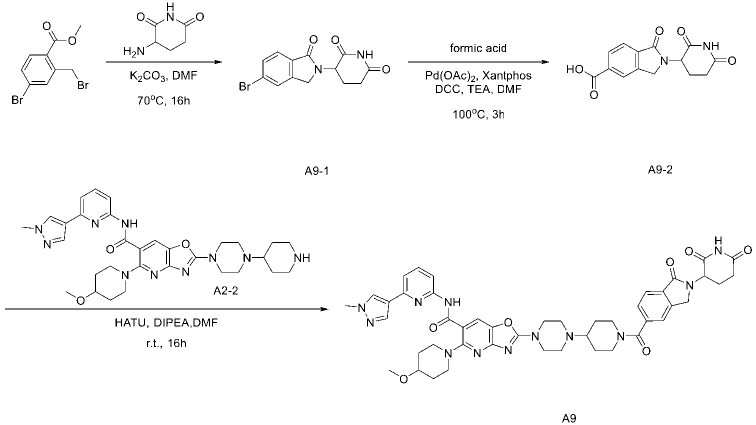

### Synthesis of Intermediate A9-1

Methyl 4-bromo-2-(bromomethyl)benzoate (450 mg, 1.5 mmol), 3-aminopiperidine-2,6-dione (206 mg, 1.6 mmol) and potassium carbonate (605 mg, 4.4 mmol) were dissolved in dry N,N-dimethylformamide (8 mL) and stirred at 70 °C for 16 hours. The reaction mixture was concentrated under reduced pressure. Water (10 mL) was added to the residue, and the mixture was slurried for 30 minutes, filtered, and washed with anhydrous diethyl ether to afford Intermediate A9-1 (200 mg, 42%).

¹H NMR (300 MHz, DMSO-d₆): δ 11.00 (s, 1H), 7.89 (s, 1H), 7.77-7.57 (m, 2H), 5.16-5.05 (m, 1H), 4.52-4.29 (m, 2H), 2.96-2.83 (m, 1H), 2.68-2.59 (m, 1H), 2.43-2.26 (m, 1H), 2.08-1.95 (m, 1H).

LC-MS: *m*/*z* 323.0 [M+H]⁺.

### Synthesis of Intermediate A9-2

Intermediate A9-1 (200 mg, 0.62 mmol), DCC (26 mg, 0.12 mmol), triethylamine (13 mg, 0.12 mmol), formic acid (100 mg, 2.2 mmol), palladium acetate (7.0 mg, 0.030 mmol) and Xantphos (18 mg, 0.030 mmol) were dissolved in dry N,N-dimethylformamide (8 mL). The mixture was stirred at 110 °C for 3 hours under nitrogen protection. The reaction mixture was filtered and concentrated under reduced pressure. Dry dichloromethane (5 mL) was added to the residue, and the mixture was slurried. The solution was filtered to afford Intermediate A9-2 (90 mg, 50%).

¹H NMR (300 MHz, DMSO-d₆): δ 11.03 (s, 1H), 8.17 (s, 1H), 8.07 (d, *J* = 7.8 Hz, 1H), 7.83 (d, *J* = 7.8 Hz, 1H), 5.21-5.08 (m, 1H), 4.59-4.34 (m, 2H), 2.96-2.83 (m, 1H), 2.67-2.55 (m, 1H), 2.45-2.31 (m, 1H), 2.09-1.96 (m, 1H).

LC-MS: m/z 289.1 [M+H]⁺.

### Synthesis of Final Product A9

Intermediate A9-2 (40 mg, 0.14 mmol), Intermediate A2-2 (83 mg, 0.14 mmol), HATU (53 mg, 0.14 mmol) and DIPEA (108 mg, 0.83 mmol) were dissolved in dry N,N-dimethylformamide (2 mL) and stirred at room temperature for 16 hours. Water (3 mL) was added, and the mixture was extracted with ethyl acetate three times (10 mL × 3), washed with saturated aqueous NaCl solution three times (15 mL × 3). The combined organic phases were dried over anhydrous sodium sulfate, concentrated under reduced pressure, and purified by silica gel column chromatography (dichloromethane/methanol = 20/1, v/v) to afford Final ProductA9 (5 mg, 4%).

### Example 10: Synthesis of Compound A11

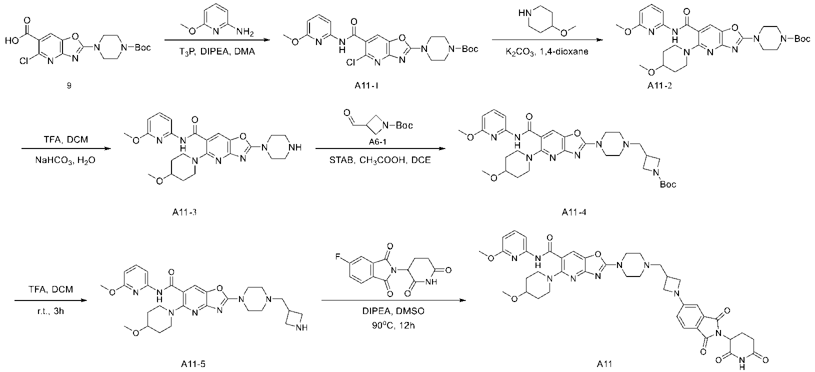

### Synthesis of Intermediate A11-1

Intermediate 9 (200 mg, 0.52 mmol) and 2-amino-6-methoxypyridine (98 mg, 0.79 mmol) were dissolved in DMA (0.4 mL). DIPEA (335 mg, 2.6 mmol) and 50% T₃P in ethyl acetate (1.6 g, 2.6 mmol) were added, and the mixture was subjected to microwave reaction at 110 °C for 2 hours. After completion, the ethyl acetate in the reaction mixture was evaporated, water (10 mL) was added for slurrying, filtered, and dried to afford brown solid A11-1 (210 mg, 82%).

¹H NMR (300 MHz, DMSO-d₆): δ 10.87 (s, 1H), 8.06 (s, 1H), 7.80-7.69 (m, 2H), 6.61-6.55 (m, 1H), 3.81 (s, 3H), 3.72-3.66 (m, 4H), 3.54-3.49 (m, 4H), 1.43 (s, 9H).

### Synthesis of Intermediate A11-2

A11-1 (210 mg, 0.43 mmol) and 4-methoxypiperidine (390 mg, 3.4 mmol) were dissolved in 1,4-dioxane (1.5 mL). Potassium carbonate (297 mg, 2.2 mmol) was added, and the mixture was stirred at 105 °C for 16 hours. After completion, the mixture was concentrated, diluted with water (5 mL), extracted with DCM (10 mL × 2), dried over anhydrous sodium sulfate, concentrated, slurried in diethyl ether (10 mL) for 8 hours, filtered, and dried to afford crude pale yellow solid A11-2 (170 mg).

### Synthesis of Intermediate A11-3

Crude A11-2 (170 mg, 0.29 mmol) was dissolved in dichloromethane (2 mL). Trifluoroacetic acid (1 mL) was added, and the mixture was stirred at room temperature for 6 hours. After completion, the mixture was concentrated, diluted with water (15 mL), and extracted with DCM (10 mL × 2) to afford an aqueous phase. The pH was adjusted to 7-8 with saturated sodium bicarbonate solution, extracted with DCM (20 mL × 2), dried over anhydrous sodium sulfate, and concentrated to afford white solid A11-3 (130 mg, 65%).

¹H NMR (300 MHz, DMSO-d₆): δ 13.23 (s, 1H), 8.22 (s, 1H), 7.84 (d, *J* = 7.8 Hz, 1H), 7.72 (t, *J* = 7.8 Hz, 1H), 6.55 (d, *J* = 7.8 Hz, 1H), 3.84 (s, 3H), 3.64-3.58 (m, 5H), 3.28 (s, 3H), 3.22-3.16 (m, 2H), 2.99-2.91 (m, 2H), 2.83-2.78 (m, 4H), 2.17-2.09 (m, 2H), 1.98-1.88 (m, 2H).

### Synthesis of Intermediate A11-4

A11-3 (130 mg, 0.28 mmol) was dissolved in DCE (5 mL). A6-1 (130 mg, 0.71 mmol) was added, and the mixture was stirred at room temperature for 0.5 hour. STAB (150 mg, 0.71 mmol) was added, and the mixture was stirred for 8 hours. After completion, saturated sodium bicarbonate solution (10 mL) was added to quench the reaction. The mixture was extracted with DCM (15 mL × 2), dried over anhydrous sodium sulfate, concentrated, and purified by silica gel column chromatography (DCM/MeOH = 25/1, v/v) to afford white solid A11-4 (108 mg, 61%).

¹H NMR (300 MHz, DMSO-d₆): δ 13.20 (s, 1H), 8.22 (s, 1H), 7.84 (d, *J =* 7.8 Hz, 1H), 7.72 (t, *J* = 7.8 Hz, 1H), 6.55 (d, *J* = 7.8 Hz, 1H), 3.95-3.88 (m, 2H), 3.84 (s, 3H), 3.70-3.64 (m, 4H), 3.52-3.47 (m, 2H), 3.37-3.34 (m, 5H), 3.29 (s, 3H), 3.23-3.16 (m, 2H), 3.00-2.90 (m, 2H), 2.80-2.71 (m, 1H), 2.60-2.55 (m, 2H), 2.16-2.08 (m, 2H), 1.98-1.87 (m, 2H), 1.37 (s, 9H).

### Synthesis of Intermediate A 11-5

Intermediate A11-4 (108 mg, 0.17 mmol) was dissolved in dry dichloromethane (2 mL). Trifluoroacetic acid (1 mL) was added, and the mixture was stirred at room temperature for 3 hours. The reaction mixture was concentrated under reduced pressure. Dichloromethane (10 mL) and methanol (2 mL) were added to the residue, and the pH was adjusted to 7 with solid NaHCO₃ powder. The mixture was filtered, and the filtrate was concentrated to afford crude Intermediate A11-5 (90 mg, 99%).

¹H NMR (300 MHz, DMSO-d₆): δ 13.21 (s, 1H), 8.23 (s, 1H), 7.84 (d, *J* = 7.2 Hz, 1H), 7.72 (t, *J* = 7.2 Hz, 1H), 6.54 (d, *J* = 7.5 Hz, 1H), 4.06-3.94 (m, 2H), 3.84 (s, 3H), 3.73-3.63 (m, 4H), 3.47-3.36 (m, 10H), 3.30-3.26 (m, 3H), 3.02-2.90 (m, 2H), 2.67-2.60 (m, 1H), 2.58-2.51 (m, 2H), 2.22-2.05 (m, 2H), 2.03-1.85 (m, 2H).

LC-MS: *m*/*z* 537.0 [M+H]⁺.

### Synthesis of Final Product A11

Intermediate A11-5 (90 mg, 0.17 mmol) and 2-(2,6-dioxopiperidin-3-yl)-5-fluoroisoindoline-1,3-dione (60 mg, 0.22 mmol, commercially available) were dissolved in dry dimethyl sulfoxide (2 mL). DIPEA (108 mg, 0.84 mmol) was added, and the mixture was stirred at 95 °C for 12 hours. Water (3 mL) was added to precipitate a yellow solid, which was filtered and purified by silica gel column chromatography (dichloromethane/methanol = 20/1, v/v) to afford A11 (100 mg, 75%).

### Example 11: Synthesis of Compound A12

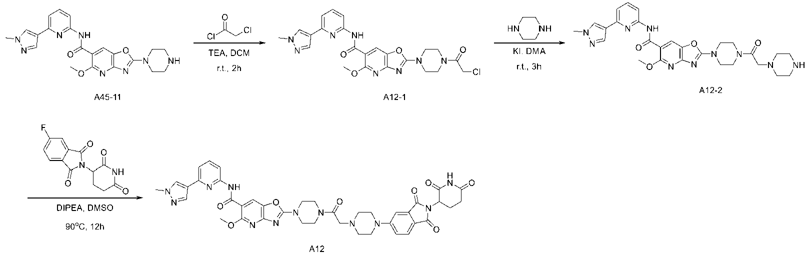

### Synthesis of Intermediate A 12-1

A45-11 (120 mg, 0.28 mmol) and triethylamine (56 mg, 0.55 mmol) were dissolved in dry dichloromethane (3 mL). Chloroacetyl chloride (41 mg, 0.36 mmol) was added under ice-water bath, and the mixture was stirred at room temperature for 2 hours. The reaction was quenched with water (2 mL), and extracted with dichloromethane three times (10 mL × 3). The combined organic phases were dried over anhydrous sodium sulfate, concentrated under reduced pressure, and purified by silica gel column chromatography (dichloromethane/methanol = 20/1, v/v) to afford brown solid A12-1 (120 mg, 85%).

¹H NMR (300 MHz, DMSO-d₆): δ 10.40 (s, 1H), 8.36-8.22 (m, 2H), 8.10-7.98 (m, 2H), 7.80 (t, *J* = 7.8 Hz, 1H), 7.41 (d, *J* = 7.5 Hz, 1H), 4.48 (s, 2H), 4.16 (s, 3H), 3.90 (s, 3H), 3.82-3.69 (m, 4H), 3.61-3.48 (m, 4H).

LC-MS: *m*/*z* 510.8 [M+H]⁺.

### Synthesis of Intermediate A12-2

A12-1 (80 mg, 0.16 mmol), piperazine (67 mg, 0.78 mmol) and potassium iodide (2 mg, 0.010 mmol) were dissolved in dry N,N-dimethylacetamide (2 mL) and stirred at room temperature for 3 hours. Water (2 mL) was added, and the mixture was extracted with ethyl acetate three times (10 mL × 3), washed with saturated NaCl solution three times (10 mL × 3). The combined organic phases were dried over anhydrous sodium sulfate, concentrated under reduced pressure, and purified by silica gel column chromatography (dichloromethane/methanol = 20/1, v/v) to afford brown solid A12-2 (30 mg, 34%).

LC-MS: *m*/*z* 560.9 [M+H]⁺.

### Synthesis of Final Product A12

Intermediate A12-2 (30 mg, 0.05 mmol) and 2-(2,6-dioxopiperidin-3-yl)-5-fluoroisoindoline-1,3-dione (19 mg, 0.070 mmol) were dissolved in dry dimethyl sulfoxide (2 mL). N-Ethyl diisopropylamine (35 mg, 0.27 mmol) was added, and the mixture was stirred at 90 °C for 12 hours. Water (3 mL) was added to precipitate a yellow solid, which was filtered and purified by silica gel column chromatography (dichloromethane/methanol = 20/1, v/v) to afford A12 (8 mg, 18%).

### Example 12: Synthesis of Compound A13

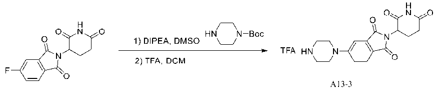

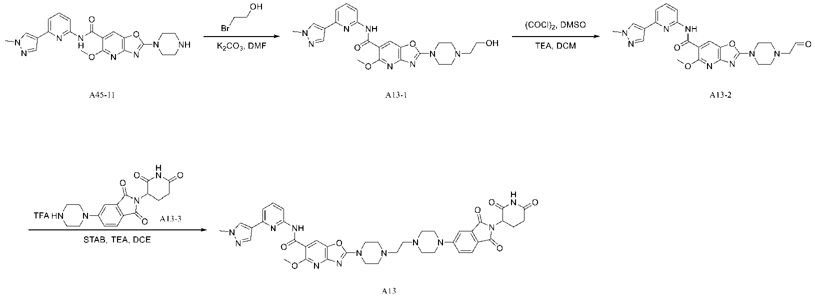

### Synthesis of Intermediate A13-1

A45-11 (150 mg, 0.35 mmol), 2-bromoethanol (430 mg, 3.5 mmol) and potassium carbonate (190 mg, 1.4 mmol) were dissolved in DMF (4 mL) and stirred at 80 °C for 6 hours. After completion, water (10 mL) was added, and the mixture was extracted with dichloromethane (20 mL × 2), washed with saturated brine (10 mL), dried over anhydrous sodium sulfate, filtered and concentrated. The residue was slurried in diethyl ether (10 mL), filtered and dried to afford white solid A13-1 (81 mg, 49%).

LC-MS: m/z 478.9 [M+H]⁺.

### Synthesis of Intermediate A13-2

Oxalyl chloride (35 mg, 0.28 mmol) was dissolved in DCM (0.5 mL) and cooled to -78 °C for 5 minutes. DMSO (43 mg, 0.55 mmol) was added, and after 30 minutes A13-1 (30 mg, 0.062 mmol) was added. After another 30 minutes, triethylamine (86 mg, 0.85 mmol) was added, and stirring continued for 30 minutes. The mixture was warmed to room temperature and stirred for a further 1.5 hours. After completion, saturated aqueous sodium bicarbonate (3 mL) was added to quench the reaction, and the mixture was extracted with dichloromethane (15 mL × 2), washed with saturated brine (5 mL), dried over anhydrous sodium sulfate, filtered and concentrated. The residue was slurried in diethyl ether (5 mL), filtered and dried to afford pale yellow solid A13-2 (20 mg, 67%).

LC-MS: *m*/*z* 508.9 [M+MeOH+H]⁺.

### Synthesis of Intermediate A13-3

2-(2,6-dioxopiperidin-3-yl)-5-fluoroisoindoline-1,3-dione (150 mg, 0.54 mmol) was dissolved in DMSO (5 mL). 1-(tert-butoxycarbonyl)piperazine (112 mg, 0.60 mmol) and DIPEA (350 mg, 2.7 mmol) were added, and the mixture was stirred at 90 °C overnight. After completion, water (10 mL) was added, and the mixture was extracted with dichloromethane (20 mL × 2), washed with saturated brine (5 mL × 3), dried over anhydrous sodium sulfate, filtered and concentrated. The residue was slurried in diethyl ether (10 mL), filtered and dried to afford a crude yellow solid (170 mg). The crude product was dissolved in dichloromethane (5 mL), trifluoroacetic acid (2 mL) was added, and the mixture was stirred at room temperature for 2 hours. After concentration, crude yellow solid A13-3 (190 mg) was obtained.

LC-MS: m/z 342.9 [M+H]⁺.

### Synthesis of Final Product A13

A13-3 (25 mg, 0.054 mmol) was dissolved in DCE (0.5 mL). Triethylamine (18 mg, 0.18 mmol) was added, and after 30 minutes A13-2 (20 mg, 0.042 mmol) was added. After another 30 minutes, sodium triacetoxyborohydride (23 mg, 0.11 mmol) was added, and the mixture was stirred overnight. After completion, saturated aqueous sodium bicarbonate (3 mL) was added to quench the reaction, and the mixture was extracted with dichloromethane (10 mL × 2), washed with saturated brine (5 mL), dried over anhydrous sodium sulfate, filtered and concentrated. Purification by preparative silica gel plate (dichloromethane/methanol = 15/1, v/v) afforded A13 (6 mg, 18%).

### Example 13: Synthesis of Compound A35

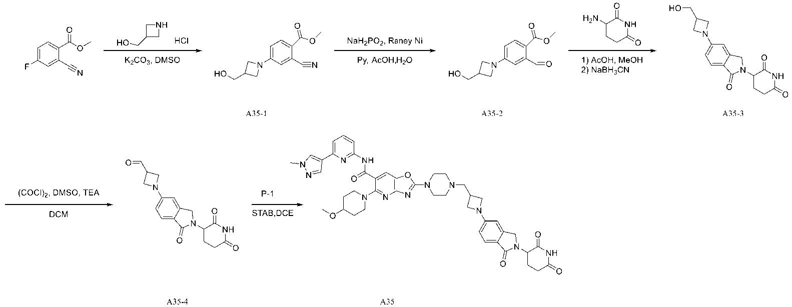

### Synthesis of Intermediate A35-1

Methyl 2-cyano-4-fluorobenzoate (2.6 g, 14.5 mmol), azetidin-3-ylmethanol hydrochloride (2.0 g, 16.2 mmol) and potassium carbonate (8.0 g, 58.0 mmol) were dissolved in DMSO (35 mL) and stirred at 110 °C overnight. After completion, the mixture was diluted with ethyl acetate (40 mL), water (100 mL) was added, and the mixture was extracted with ethyl acetate (100 mL × 2), washed with saturated brine (50 mL), dried over anhydrous sodium sulfate, filtered and concentrated. Purification by silica gel column chromatography (dichloromethane/ethyl acetate = 5/1, v/v) afforded white solid A35-1 (1.2 g, 34%).

LC-MS: m/z 246.9 [M+H]⁺.

### Synthesis of Intermediate A35-2

A35-1 (1 g, 4.1 mmol) and sodium hypophosphite monohydrate (4.3 g, 41 mmol) were dissolved in water (4 mL). Acetic acid (4 mL) and pyridine (8 mL) were added, and the mixture was warmed to 70 °C. Raney nickel (1 g) was added, and the reaction was stirred for 8 hours. Water (20 mL) was added, and the mixture was extracted with ethyl acetate (40 mL × 2). The organic phase was washed with 1 N hydrochloric acid twice (20 mL × 2), washed with saturated brine (20 mL), dried over anhydrous sodium sulfate, filtered and concentrated to afford crude yellow solid A35-2 (800 mg).

LC-MS: m/z 249.9 [M+H]⁺.

### Synthesis of Intermediate A35-3

Crude A35-2 (800 mg) was dissolved in methanol (20 mL). 3-Aminopiperidine-2,6-dione (240 mg, 1.9 mmol) and glacial acetic acid (1.0 g, 17.3 mmol) were added, and the mixture was stirred at room temperature. After 30 minutes, sodium cyanoborohydride (220 mg, 3.5 mmol) was added, and the mixture was stirred at 35 °C for 16 hours. After completion, the reaction mixture was poured into ice water (30 mL), and the pH was adjusted to 7-8 with saturated aqueous sodium bicarbonate to precipitate a solid. The solid was washed with diethyl ether (10 mL), slurried in ethyl acetate (10 mL), filtered and dried to afford dark blue solid (240 mg, 46%).

LC-MS: *m*/*z* 330.0 [M+H]⁺.

### Synthesis of Intermediate A35-4

Oxalyl chloride (191 mg, 1.5 mmol) was dissolved in DCM (8 mL) and cooled to -78 °C. After 5 minutes, DMSO (235 mg, 3.0 mmol) was added. After 30 minutes, A15-3 (100 mg, 0.30 mmol) was added. After another 30 minutes, triethylamine (455 mg, 4.5 mmol) was added, and stirring continued for 30 minutes. The mixture was warmed to room temperature and stirred for a further 1.5 hours. After completion, saturated aqueous sodium bicarbonate (8 mL) was added to quench the reaction, and the mixture was extracted with dichloromethane (25 mL × 2), washed with saturated brine (15 mL), dried over anhydrous sodium sulfate, filtered and concentrated to afford crude yellow oil A35-4 (100 mg).

LC-MS: m/z 359.9 [M+MeOH+H]⁺.

### Synthesis of Final Product A35

Crude A35-4 (80 mg) was dissolved in DCE (2 mL). P-1 (120 mg, 0.23 mmol) was added, and the mixture was stirred at room temperature for 30 minutes. STAB (146 mg, 0.69 mmol) was added, and the mixture was stirred overnight. After completion, saturated aqueous sodium bicarbonate (3 mL) was added to quench the reaction. The mixture was extracted with dichloromethane (20 mL × 2), washed with saturated brine (10 mL), dried over anhydrous sodium sulfate, filtered and concentrated. Purification by preparative silica gel plate (dichloromethane/methanol = 15/1, v/v) afforded A35 (10 mg, 5%).

### Example 14: Synthesis of Compound A37

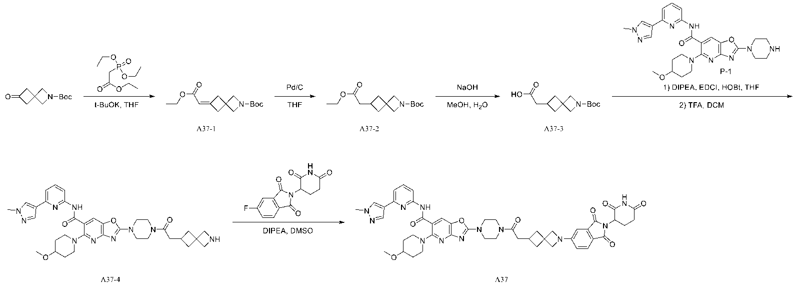

### Synthesis of Intermediate A37-1

Potassium tert-butoxide (1.3 g, 11.8 mmol) was dissolved in dry tetrahydrofuran (40 mL). Triethyl phosphonoacetate (2.6 g, 11.8 mmol) was added at 0 °C, and the mixture was slowly warmed to room temperature and stirred for 1 hour. Then tert-butyl 6-oxo-2-azaspiro[3.3]heptane-2-carboxylate (1.0 g, 4.7 mmol) was added at 0 °C, and the mixture was slowly warmed to room temperature and stirred for 16 hours. After completion, the reaction was quenched with saturated brine (20 mL), extracted with ethyl acetate (30 mL × 2), dried over anhydrous sodium sulfate, filtered and concentrated. Purification by silica gel column chromatography (petroleum ether/ethyl acetate = 1/1, v/v) afforded yellow oil A37-1 (800 mg, 60%).

LC-MS: *m*/*z* 304.1 [M+Na]⁺.

### Synthesis of Intermediate A37-2

A37-1 (800 mg, 2.8 mmol) was dissolved in methanol (30 mL). 5% Palladium on carbon (150 mg) was added, and the mixture was stirred under hydrogen balloon at room temperature overnight. After completion, the mixture was filtered through Celite, and the filtrate was concentrated to afford yellow oil A37-2 (720 mg, 89%).

¹H NMR (300 MHz, CDCl₃): δ 4.10 (q, *J* = 7.2 Hz, 2H), 3.95-3.89 (m, 2H), 3.83-3.75 (m, 2H), 2.56-2.46 (m, 1H), 2.39-2.28 (m, 4H), 1.91-1.81 (m, 2H), 1.42 (s, 9H), 1.23 (t, *J=* 7.2 Hz, 3H).

### Synthesis of Intermediate A37-3

A37-2 (720 mg, 2.5 mmol) was dissolved in methanol (10 mL). Sodium hydroxide (500 mg, 12.5 mmol) dissolved in water (10 mL) was added dropwise, and the mixture was stirred at room temperature overnight. After completion, the mixture was diluted with water (10 mL), concentrated, adjusted to pH 4-5 with 1 N hydrochloric acid, extracted with DCM (30 mL × 2), dried over anhydrous sodium sulfate, filtered and concentrated to afford white solid A37-3 (630 mg, 97%).

LC-MS: m/z 278.2 [M+Na]⁺.

### Synthesis of Intermediate A37-4

A37-3 (100 mg, 0.40 mmol) was dissolved in tetrahydrofuran (2 mL). P-1 (145 mg, 0.28 mmol), DIPEA (155 mg, 1.2 mmol), EDCI (77 mg, 0.40 mmol) and HOBT (54 mg, 0.40 mmol) were added successively, and the mixture was stirred at room temperature overnight. After completion, the reaction was quenched with water (10 mL), extracted with dichloromethane (15 mL × 2), dried over anhydrous sodium sulfate, filtered and concentrated. The residue was slurried in diethyl ether (10 mL), filtered and dried to afford a crude pale yellow solid. The crude product was dissolved in dichloromethane (4 mL), trifluoroacetic acid (2 mL) was added, and the mixture was stirred at room temperature for 1 hour. After concentration, the residue was adjusted to pH 7-8 with saturated aqueous sodium bicarbonate, extracted with dichloromethane (20 mL × 2), dried over anhydrous sodium sulfate, filtered and concentrated. The residue was slurried in diethyl ether (20 mL), filtered and dried to afford pale yellow solid A37-4 (120 mg, 66%).

LC-MS: m/z 654.9 [M+H]⁺.

### Synthesis of Final Product A37

A37-4 (50 mg, 0.080 mmol) was dissolved in dimethyl sulfoxide (1 mL). 2-(2,6-Dioxopiperidin-3-yl)-5-fluoroisoindoline-1,3-dione (28 mg, 0.10 mmol) and DIPEA (40 mg, 0.38 mmol) were added, and the mixture was stirred at 90 °C overnight. After completion, the mixture was extracted with dichloromethane (10 mL), washed with water (5 mL × 3) and saturated brine (5 mL), dried over anhydrous sodium sulfate, filtered and concentrated. Purification by preparative silica gel plate (dichloromethane/methanol = 15/1, v/v) afforded A37 (40 mg, 57%).

### Example 15: Synthesis of Compound A38

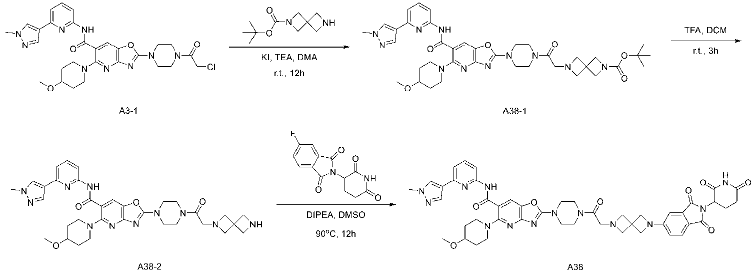

### Synthesis of Intermediate A38-1

A3-1 (80 mg, 0.13 mmol) and triethylamine (41 mg, 0.40 mmol) were dissolved in dry acetonitrile (2 mL). After stirring under ice-water bath for 0.5 hour, tert-butyl 2,6-diazaspiro[3.3]heptane-2-carboxylate (29 mg, 0.15 mmol) and potassium iodide (1 mg, 0.010 mmol) were added. The mixture was stirred at room temperature under nitrogen protection overnight. Water (2 mL) was added, and the mixture was extracted with ethyl acetate three times (10 mL × 3). The combined organic phases were dried over anhydrous sodium sulfate, concentrated under reduced pressure, and purified by silica gel column chromatography (dichloromethane/methanol = 20/1, v/v) to afford white solid A38-1 (45 mg, 44%).

¹H NMR (300 MHz, DMSO-d₆): δ 13.41 (s, 1H), 8.36-8.24 (m, 2H), 8.12-8.01 (m, 2H), 7.80 (t, *J* = 7.8 Hz, 1H), 7.40 (d, *J* = 7.2 Hz, 1H), 3.99-3.92 (m, 2H), 3.88 (s, 3H), 3.82-3.66 (m, 7H), 3.65-3.42 (m, 13H), 3.27-3.20 (m, 2H), 3.05-2.95 (m, 2H), 2.23-2.01 (m, 4H), 1.36 (s, 9H). LC-MS: m/z 755.9 [M+H]⁺.

### Synthesis of Intermediate A38-2

A38-1 (45 mg, 0.060 mmol) was dissolved in dry dichloromethane (1 mL). Trifluoroacetic acid (1 mL) was added under ice-water bath, and the mixture was stirred at room temperature for 3 hours. The reaction mixture was concentrated. Dichloromethane (2 mL) was added to the residue, and the pH was adjusted to 7-8 with saturated aqueous NaHCO₃ under ice-water bath. The mixture was extracted with dichloromethane three times (5 mL × 3). The combined organic phases were dried over anhydrous sodium sulfate and concentrated under reduced pressure to afford crude A38-2 (30 mg, 77%), which was used directly in the next step.

LC-MS: *m*/*z* 656.1 [M+H]⁺.

### Synthesis of Final Product A38

Intermediate A38-2 (30 mg, 0.050 mmol) and 2-(2,6-dioxopiperidin-3-yl)-5-fluoroisoindoline-1,3-dione (15 mg, 0.050 mmol) were dissolved in dry dimethyl sulfoxide (2 mL). N-Ethyl diisopropylamine (18 mg, 0.14 mmol) was added, and the mixture was stirred at 90 °C for 12 hours. Water (2 mL) was added, and the mixture was extracted with ethyl acetate three times (10 mL × 3), washed with saturated NaCl solution three times (10 mL × 3). The combined organic phases were dried over anhydrous sodium sulfate, concentrated under reduced pressure, and purified by silica gel column chromatography (dichloromethane/methanol = 10/1, v/v) to afford A38 (4 mg, 10%).

### Example 16: Synthesis of Compound A40

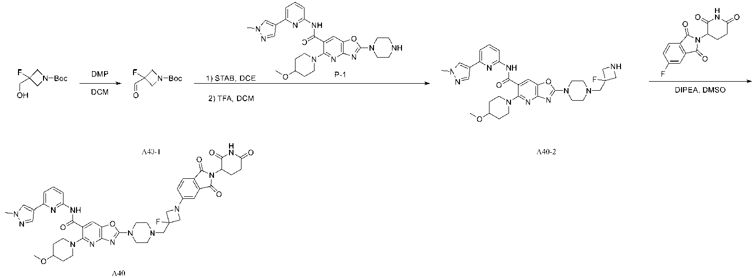

### Synthesis of Intermediate A40-1

tert-Butyl 3-fluoro-3-(hydroxymethyl)azetidine-1-carboxylate (500 mg, 2.4 mmol) was dissolved in dichloromethane (15 mL). DMP (1.2 g, 2.9 mmol) was added under ice bath, and the mixture was slowly warmed to room temperature. The reaction was completed in 2 hours. Saturated aqueous sodium thiosulfate pentahydrate (10 mL) was added to quench the reaction, followed by saturated aqueous sodium bicarbonate (10 mL). The mixture was filtered through Celite, and the filtrate was extracted with dichloromethane (15 mL × 2), dried over anhydrous sodium sulfate, filtered and concentrated to afford crude white solid A40-1 (300 mg).

LC-MS: m/z 257.9 [M+MeOH+Na]⁺.

### Synthesis of Intermediate A40-2

P-1 (50 mg, 0.19 mmol) and A40-1 (120 mg, 0.59 mmol) were dissolved in DCE (2 mL) and stirred at room temperature for 1 hour. Sodium triacetoxyborohydride (100 mg, 0.48 mmol) was added, and the mixture was stirred at room temperature overnight. After completion, saturated aqueous sodium bicarbonate (10 mL) was added to quench the reaction, and the mixture was extracted with dichloromethane (20 mL × 2), washed with saturated brine (10 mL), dried over anhydrous sodium sulfate, filtered and concentrated. Purification by silica gel column chromatography (dichloromethane/methanol = 30/1) afforded a crude white solid. The crude product was dissolved in dichloromethane (4 mL), trifluoroacetic acid (2 mL) was added, and the mixture was stirred at room temperature for 1 hour. After concentration, the residue was adjusted to pH 7-8 with saturated aqueous sodium bicarbonate, extracted with dichloromethane (20 mL × 2), dried over anhydrous sodium sulfate, filtered and concentrated. The residue was slurried in diethyl ether (5 mL), filtered and dried to afford white solid A40-2 (30 mg, 26%).

LC-MS: m/z 604.9 [M+H]⁺.

### Synthesis of Final Product A40

A40-2 (30 mg, 0.050 mmol) was dissolved in dimethyl sulfoxide (1.5 mL). 2-(2,6-Dioxopiperidin-3-yl)-5-fluoroisoindoline-1,3-dione (16 mg, 0.058 mmol) and DIPEA (32 mg, 0.25 mmol) were added, and the mixture was stirred at 90 °C overnight. After completion, the mixture was extracted with dichloromethane (10 mL), washed with water (5 mL × 3) and saturated brine (5 mL), dried over anhydrous sodium sulfate, filtered and concentrated. Purification by preparative silica gel plate (dichloromethane/methanol = 15/1, v/v) afforded A40 (22 mg, 51%).

### Example 17: Synthesis of Compound A41

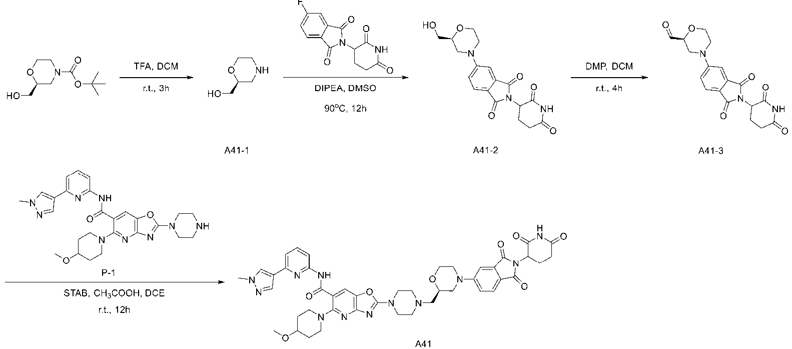

### Synthesis of Intermediate A41-1

(S)-tert-Butyl 2-(hydroxymethyl)morpholine-4-carboxylate (100 mg, 0.46 mmol) was dissolved in dry dichloromethane (2 mL). Trifluoroacetic acid (2 mL) was added under ice-water bath, and the mixture was stirred at room temperature for 3 hours. The reaction mixture was concentrated. Dichloromethane (2 mL) was added to the residue, and the pH was adjusted to 7-8 with solid NaHCO₃ powder under ice-water bath. The mixture was filtered and concentrated under reduced pressure to afford crude A41-1 (50 mg, 93%), which was used directly in the next step.

### Synthesis of Intermediate A41-2

Intermediate A41-1 (50 mg, 0.43 mmol) and 2-(2,6-dioxopiperidin-3-yl)-5-fluoroisoindoline-1,3-dione (107 mg, 0.39 mmol) were dissolved in dry dimethyl sulfoxide (2 mL). N-Ethyl diisopropylamine (200 mg, 1.6 mmol) was added, and the mixture was stirred at 90 °C for 12 hours. Water (2 mL) was added, and the mixture was extracted with ethyl acetate three times (10 mL × 3), washed with saturated NaCl solution three times (10 mL × 3). The combined organic phases were dried over anhydrous sodium sulfate, concentrated under reduced pressure, and purified by silica gel column chromatography (dichloromethane/methanol = 20/1, v/v) to afford yellow solid A41-2 (110 mg, 76%).

¹H NMR (300 MHz, DMSO-d₆): δ 11.09 (s, 1H), 7.71 (d, *J* = 8.7 Hz, 1H), 7.35 (s, 1H), 7.27 (d, *J* = 8.4 Hz, 1H), 5.13-5.02 (m, 1H), 4.88-4.76 (m, 1H), 4.00-3.81 (m, 2H), 3.67-3.45 (m, 3H), 2.99-2.82 (m, 2H), 2.75-2.65 (m, 1H), 2.63-2.51 (m, 4H), 2.08-1.96 (m, 1H).

LC-MS: m/z 373.9 [M+H]⁺.

### Synthesis of Intermediate A41-3

Intermediate A41-2 (30 mg, 0.080 mmol) was dissolved in dry dichloromethane (2 mL). Dess-Martin periodinane (41 mg, 0.10 mmol) was added under ice-water bath, and the mixture was stirred at room temperature for 2 hours. Saturated aqueous sodium sulfite (1 mL) and saturated aqueous sodium bicarbonate (2 mL) were added to quench the reaction. The mixture was extracted with dichloromethane three times (10 mL × 3). The combined organic phases were dried over anhydrous sodium sulfate, concentrated under reduced pressure, and purified by silica gel column chromatography (dichloromethane/methanol = 20/1, v/v) to afford yellow solid A41-3 (20 mg, 67%).

LC-MS: *m*/*z* 403.9 [M+MeOH+H]⁺.

### Synthesis of Final Product A41

A41-3 (20 mg, 0.050 mmol) and P-1 (25 mg, 0.050 mmol) were dissolved in dry 1,2-dichloroethane (3 mL). One drop of acetic acid and activated molecular sieves (50 mg) were added, and the mixture was stirred at room temperature for 0.5 hour. Sodium triacetoxyborohydride (31 mg, 0.15 mmol) was added, and the mixture was stirred at room temperature for 12 hours. The reaction was quenched with saturated aqueous NH₄Cl solution (1 mL), concentrated under reduced pressure, and purified by silica gel column chromatography (dichloromethane/methanol = 20/1, v/v) to afford A41 (4 mg, 9%).

### Example 18: Synthesis of Compound A44

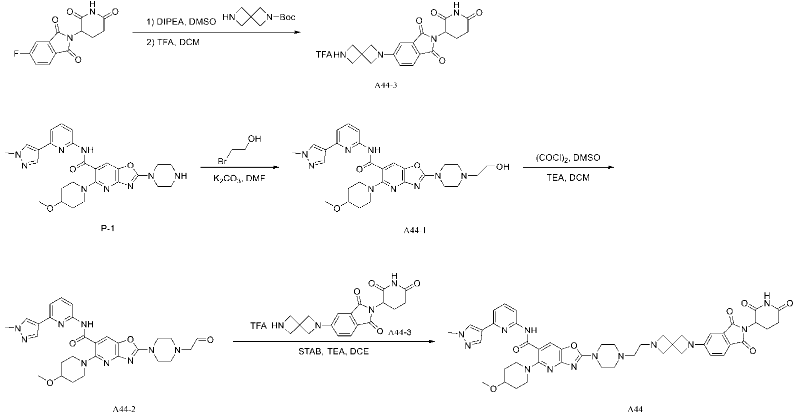

### Synthesis of Intermediate A44-1

P-1 (100 mg, 0.19 mmol), 2-bromoethanol (238 mg, 1.9 mmol) and potassium carbonate (106 mg, 0.77 mmol) were dissolved in DMF (3 mL) and stirred at 80 °C for 6 hours. After completion, water (10 mL) was added, and the mixture was extracted with dichloromethane (15 mL × 2), washed with saturated brine (10 mL), dried over anhydrous sodium sulfate, filtered and concentrated. The residue was slurried in diethyl ether (5 mL), filtered and dried to afford white solid A44-1 (80 mg, 73%).

LC-MS: *m*/*z* 562.0 [M+H]⁺.

### Synthesis of Intermediate A44-2

Oxalyl chloride (70 mg, 0.55 mmol) was dissolved in DCM (1 mL) and cooled to -78 °C. After 5 minutes, DMSO (86 mg, 1.1 mmol) was added. After 30 minutes, A44-1 (60 mg, 0.11 mmol) was added. After another 30 minutes, triethylamine (172 mg, 1.7 mmol) was added, and stirring continued for 30 minutes. The mixture was warmed to room temperature and stirred for a further 1.5 hours. After completion, saturated aqueous sodium bicarbonate (5 mL) was added to quench the reaction, and the mixture was extracted with dichloromethane (15 mL × 2), washed with saturated brine (10 mL), dried over anhydrous sodium sulfate, filtered and concentrated. The residue was slurried in diethyl ether (5 mL), filtered and dried to afford pale yellow solid A44-2 (40 mg, 67%).

LC-MS: *m*/*z* 591.9 [M+MeOH+H]⁺.

### Synthesis of Intermediate A44-3

2-(2,6-Dioxopiperidin-3-yl)-5-fluoroisoindoline-1,3-dione (200 mg, 0.72 mmol) was dissolved in DMSO (5 mL). tert-Butyl 2,6-diazaspiro[3.3]heptane-2-carboxylate (158 mg, 0.80 mmol) and DIPEA (460 mg, 3.6 mmol) were added, and the mixture was stirred at 90 °C overnight. After completion, water (10 mL) was added, and the mixture was extracted with dichloromethane (25 mL × 2), washed with saturated brine (5 mL × 3), dried over anhydrous sodium sulfate, filtered and concentrated. The residue was slurried in diethyl ether (10 mL), filtered and dried to afford a crude yellow solid (270 mg). The crude product was dissolved in dichloromethane (5 mL), trifluoroacetic acid (2 mL) was added, and the mixture was stirred at room temperature for 2 hours. After concentration, crude yellow solid A44-3 (290 mg) was obtained.

LC-MS: *m*/*z* 354.9 [M+H]⁺.

### Synthesis of Final Product A44

A44-3 (25 mg, 0.054 mmol) was dissolved in DCE (0.5 mL). Triethylamine (18 mg, 0.18 mmol) was added, and after 30 minutes A44-2 (20 mg, 0.036 mmol) was added. After another 30 minutes, sodium triacetoxyborohydride (23 mg, 0.11 mmol) was added, and the mixture was stirred overnight. After completion, saturated aqueous sodium bicarbonate (3 mL) was added to quench the reaction, and the mixture was extracted with dichloromethane (10 mL × 2), washed with saturated brine (5 mL), dried over anhydrous sodium sulfate, filtered and concentrated. Purification by preparative silica gel plate (dichloromethane/methanol = 15/1, v/v) afforded A44 (8 mg, 25%).

### Example 19: Synthesis of Compound A45

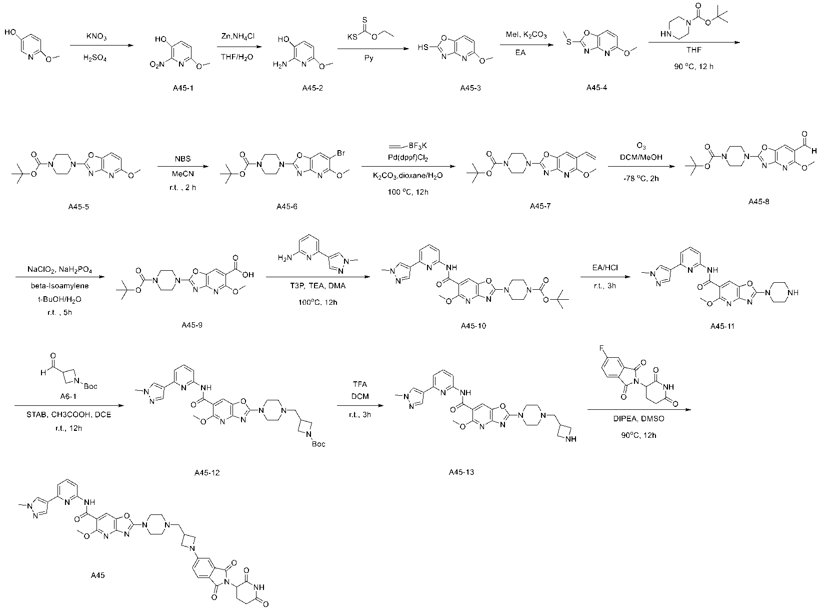

### Synthesis of Intermediate A45-1

5-Hydroxy-2-methoxypyridine (4.8 g, 38 mmol) was dissolved in concentrated sulfuric acid (20 mL). Potassium nitrate (4.6 g, 46 mmol) was added portionwise at -10 °C, and the mixture was stirred at low temperature for 2 hours. The reaction mixture was poured into ice water, filtered and dried to afford yellow solid A45-1 (5.0 g, 77%).

¹H NMR (300 MHz, CDCl₃): δ 10.18 (s, 1H), 7.54 (d, *J* = 9.0 Hz, 1H), 7.11 (d, *J* = 9.0 Hz, 1H), 3.98 (s, 3H).

### Synthesis of Intermediate A45-2

A45-1 (4.0 g, 24 mmol) and ammonium chloride (24 g, 470 mmol) were dissolved in THF/water (80 mL / 30 mL). Zinc powder (12 g, 188 mmol) was added slowly at 50 °C, and the mixture was stirred for 5 hours. The reaction mixture was filtered, and the filtrate was diluted with saturated aqueous sodium bicarbonate (30 mL), extracted with ethyl acetate (20 mL × 3), dried over anhydrous sodium sulfate, concentrated and purified by silica gel column chromatography (petroleum ether/ethyl acetate = 5/1, v/v) to afford black solid A45-2 (2.7 g, 82%).

¹H NMR (300 MHz, DMSO-d₆): δ 8.69 (s, 1H), 6.95-6.70 (m, 1H), 5.87-5.66 (m, 1H), 5.35 (s, 2H), 3.65 (s, 3H).

### Synthesis of Intermediate A45-3

A45-2 (1.0 g, 7.1 mmol) was dissolved in pyridine (20 mL). Potassium ethyl xanthate (1.7 g, 11 mmol) was added, and the mixture was refluxed at 120 °C overnight. The reaction mixture was poured into ice water, acidified with 3N aqueous HCl, filtered and washed with water to afford yellow solid A45-3 (1.0 g, 77%).

¹H NMR (300 MHz, CDCl₃): δ 7.49 (d, *J =* 8.8 Hz, 1H), 6.60 (d, *J* = 9.3 Hz, 1H), 3.92 (s, 3H).

LC-MS: *m*/*z* 183.1 [M+H]⁺.

### Synthesis of Intermediate A45-4

A45-3 (1.1 g, 6.0 mmol) was dissolved in ethyl acetate (20 mL). Potassium carbonate (1.6 g, 12 mmol) and iodomethane (1.7 g, 12 mmol) were added, and the mixture was stirred at room temperature for 3 hours. The reaction mixture was concentrated, diluted with water (20 mL), extracted with ethyl acetate (20 mL × 3), dried over anhydrous sodium sulfate and concentrated to afford yellow solid A45-4 (1.8 g, 81%).

¹H NMR (300 MHz, CDCl₃): δ 7.81 (d, *J* = 8.4 Hz, 1H), 6.82 (d, *J* = 7.5 Hz, 1H), 4.19 (s, 3H), 2.98 (s, 3H).

### Synthesis of Intermediate A45-5

A45-4 (17 g, 87 mmol) and N-Boc-piperazine (81 g, 434 mmol) were dissolved in tetrahydrofuran (120 mL) and stirred at 90 °C overnight. The reaction mixture was concentrated, and the residue was slurried with water, filtered and dried to afford gray solid A45-5 (20.4 g, 70%).

¹H NMR (300 MHz, DMSO-d₆): δ 7.71 (d, *J* = 8.7 Hz, 1H), 6.38 (d, *J* = 8.7 Hz, 1H), 3.81 (s, 3H), 3.65-3.55 (m, 4H), 3.52-3.44 (m, 4H), 1.42 (s, 9H).

LC-MS: m/z 334.9 [M+H]⁺.

### Synthesis of Intermediate A45-6

A45-5 (20 g, 60 mmol) was dissolved in acetonitrile (160 mL). N-Bromosuccinimide (13 g, 72 mmol) was added portionwise, and the mixture was stirred at room temperature for 2 hours. The reaction mixture was concentrated, water (120 mL) was added, and the mixture was extracted with ethyl acetate three times (300 mL × 3), washed with saturated NaCl solution three times (300 mL × 3). The combined organic phases were dried over anhydrous sodium sulfate, concentrated and purified by silica gel column chromatography (petroleum ether/ethyl acetate = 5/1, v/v) to afford brown solid A45-6 (17 g, 69%).

¹H NMR (300 MHz, DMSO-d₆): δ 8.13 (s, 1H), 3.88 (s, 3H), 3.67-3.58 (m, 4H), 3.51-3.43 (m, 4H), 1.42 (s, 9H).

LC-MS: m/z 412.8 [M+H]⁺.

### Synthesis of Intermediate A45-7

A45-6 (17 g, 41 mmol), potassium vinyltrifluoroborate (6.1 g, 45 mmol), potassium carbonate (11 g, 82 mmol) and Pd(dppf)Cl₂ (1.5 g, 2.1 mmol) were dissolved in 1,4-dioxane/water (400 mL / 40 mL) and stirred at 100 °C under nitrogen protection overnight. The reaction mixture was concentrated under reduced pressure and purified by silica gel column chromatography (dichloromethane/methanol = 20/1, v/v) to afford pale yellow solid A45-7 (8.6 g, 58%).

¹H NMR (300 MHz, DMSO-d₆): δ 7.99 (s, 1H), 6.85 (dd, *J* = 17.4, 11.4 Hz, 1H), 5.78 (d, *J*= 17.4 Hz, 1H), 5.19 (d, *J* = 11.4 Hz, 1H), 3.87 (s, 3H), 3.69-3.57 (m, 4H), 3.54-3.43 (m, 4H), 1.43 (s, 9H).

LC-MS: *m*/*z* 361.0 [M+H]⁺.

### Synthesis of Intermediate A45-8

A45-7 (7.0 g, 19 mmol) was dissolved in dichloromethane/methanol (80 mL / 8 mL). Ozone was bubbled through at -78 °C for 2 hours, then dimethyl sulfide (8 mL) was added to quench the reaction. The mixture was warmed to room temperature, concentrated under reduced pressure and purified by silica gel column chromatography (dichloromethane/methanol = 20/1, v/v) to afford pale yellow solid A45-8 (4.8 g, 68%).

¹H NMR (300 MHz, DMSO-d₆): δ 10.15 (s, 1H), 7.90 (s, 1H), 3.98 (s, 3H), 3.76-3.62 (m, 4H), 3.55-3.46 (m, 4H), 1.43 (s, 9H).

LC-MS: m/z 362.9 [M+M]⁺.

### Synthesis of Intermediate A45-9

A45-8 (4.8 g, 13 mmol) was dissolved in tert-butanol (150 mL). Isopentene (9.3 g, 133 mmol) was added at room temperature. Sodium dihydrogen phosphate dihydrate (24 g, 133 mmol) and sodium chlorite (6.0 g, 66 mmol) were dissolved separately in 75 mL water and added sequentially. The mixture was stirred at room temperature for 5 hours, adjusted to pH 3-4 with 1N aqueous HCl, extracted with dichloromethane three times (300 mL × 3). The combined organic phases were dried over anhydrous sodium sulfate and concentrated under reduced pressure to afford white solid A45-9 (3.0 g, 60%).

¹H NMR (300 MHz, DMSO-d₆): δ 8.04 (s, 1H), 3.89 (s, 3H), 3.71-3.59 (m, 4H), 3.54-3.42 (m, 4H), 1.43 (s, 9H).

LC-MS: m/z 378.9 [M+H]⁺.

### Synthesis of Intermediate A45-10

A45-9 (1.7 g, 4.5 mmol) was dissolved in dry N,N-dimethylacetamide (20 mL). N,N-Diisopropylethylamine (2.9 g, 22 mmol) and 2-amino-6-(1-methyl-1H-pyrazol-4-yl)pyridine (1.2 g, 6.7 mmol) were added, followed by 50% T₃P in ethyl acetate (15 mL, 22 mmol). The mixture was stirred at 100 °C overnight. The reaction mixture was poured into ice water (20 mL), extracted with ethyl acetate three times (40 mL × 3), washed with saturated NaCl solution three times (40 mL × 3). The combined organic phases were dried over anhydrous sodium sulfate, concentrated and purified by silica gel column chromatography (dichloromethane/methanol = 20/1, v/v) to afford brown solid A45-10 (1.3 g, 54%).

¹H NMR (300 MHz, DMSO-d₆): δ 10.39 (s, 1H), 8.32-8.17 (m, 2H), 8.08-7.94 (m, 2H), 7.83-7.75 (m, 1H), 7.40 (d, *J* = 4.2 Hz, 1H), 4.16 (s, 3H), 3.90 (s, 3H), 3.74-3.61 (m, 4H), 3.56-3.46 (m, 4H), 1.43 (s, 9H).

LC-MS: *m*/*z* 534.9 [M+H]⁺.

### Synthesis of Intermediate A45-11

A45-10 (1.3 g, 2.4 mmol) was dissolved in dry ethyl acetate (5 mL). 2.8N HCl in ethyl acetate (6 mL) was added under ice-water bath, and the mixture was stirred at room temperature overnight. The reaction mixture was concentrated, ethyl acetate (5 mL) was added, and the pH was adjusted to 7-8 with saturated aqueous NaHCO₃ under ice-water bath. The mixture was extracted with ethyl acetate three times (30 mL × 3). The combined organic phases were dried over anhydrous sodium sulfate and concentrated under reduced pressure to afford brown solid A45-11 (1.0 g, 95%).

¹H NMR (300 MHz, DMSO-d₆): δ 10.38 (s, 1H), 8.28 (s, 1H), 8.21 (s, 1H), 8.09-7.97 (m, 2H), 7.79 (t, *J* = 8.1 Hz, 1H), 7.40 (d, *J* = 7.5 Hz, 1H), 4.15 (s, 3H), 3.90 (s, 3H), 3.70-3.55 (m, 4H), 2.91-2.71 (m, 4H).

LC-MS: m/z 434.9 [M+H]⁺.

### Synthesis of Intermediate A45-12

A45-11 (59 mg, 0.14 mmol) and A6-1 (100 mg, 0.54 mmol) were dissolved in dry 1,2-dichloroethane (3 mL). One drop of acetic acid and activated molecular sieves (50 mg) were added, and the mixture was stirred at room temperature for 0.5 hour. Sodium triacetoxyborohydride (86 mg, 0.41 mmol) was added, and the mixture was stirred at room temperature for 12 hours. The reaction was quenched with saturated aqueous NH₄Cl (1 mL), concentrated under reduced pressure and purified by silica gel column chromatography (dichloromethane/methanol = 20/1, v/v) to afford white solid A45-12 (40 mg, 49%).

LC-MS: m/z 603.9 [M+H]⁺.

### Synthesis of Intermediate A45-13

A45-12 (40 mg, 0.070 mmol) was dissolved in dry ethyl acetate (1 mL). 2.8N HCl in ethyl acetate (1 mL) was added under ice-water bath, and the mixture was stirred at room temperature for 3 hours. The reaction mixture was concentrated, ethyl acetate (2 mL) was added, and the pH was adjusted to 7-8 with saturated aqueous NaHCO₃ under ice-water bath. The mixture was extracted with ethyl acetate three times (5 mL × 3). The combined organic phases were dried over anhydrous sodium sulfate and concentrated under reduced pressure to afford crude A45-13 (30 mg, 90%), which was used directly in the next step.

¹H NMR (300 MHz, DMSO-d₆): δ 10.37 (s, 1H), 8.33-8.13 (m, 2H), 8.11-7.92 (m, 2H), 7.79 (t, *J =* 7.5 Hz, 1H), 7.40 (d, *J* = 7.2 Hz, 1H), 4.15 (s, 3H), 3.89 (s, 3H), 3.79-3.72 (m, 4H), 3.70-3.66 (m, 4H), 3.42-3.35 (m, 4H), 2.89-2.81 (m, 1H), 2.71-2.56 (m, 2H).

LC-MS: *m*/*z* 503.9 [M+H]⁺.

### Synthesis of Final Product A45

Intermediate A45-13 (30 mg, 0.060 mmol) and 2-(2,6-dioxopiperidin-3-yl)-5-fluoroisoindoline-1,3-dione (20 mg, 0.070 mmol) were dissolved in dry dimethyl sulfoxide (2 mL). N-Ethyl diisopropylamine (38 mg, 0.30 mmol) was added, and the mixture was stirred at 90 °C for 12 hours. Water (3 mL) was added to precipitate a yellow solid, which was filtered and purified by silica gel column chromatography (dichloromethane/methanol = 20/1, v/v) to affordA45 (20 mg, 44%).

### Example 20: Synthesis of Compound A46

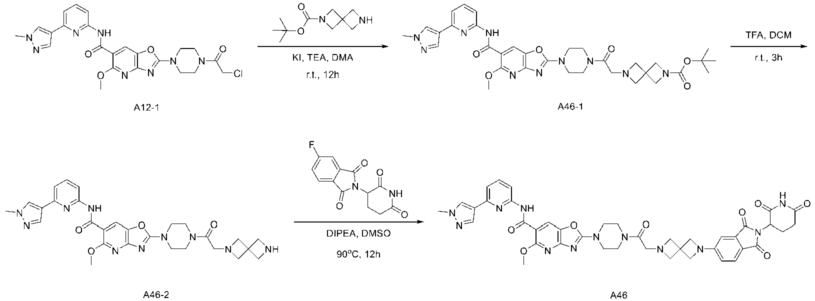

### Synthesis of Intermediate A46-1

A12-1 (85 mg, 0.17 mmol) and triethylamine (51 mg, 0.50 mmol) were dissolved in dry acetonitrile (3 mL). After stirring under ice bath for 0.5 hour, tert-butyl 2,6-diazaspiro[3.3]heptane-2-carboxylate (36 mg, 0.18 mmol) and potassium iodide (3 mg, 0.017 mmol) were added. The mixture was stirred at room temperature under nitrogen protection overnight. Water (2 mL) was added, and the mixture was extracted with ethyl acetate three times (10 mL × 3). The combined organic phases were dried over anhydrous sodium sulfate, concentrated under reduced pressure, and purified by silica gel column chromatography (dichloromethane/methanol = 20/1, v/v) to afford white solid A46-1 (40 mg, 36%).

¹H NMR (300 MHz, DMSO-d₆) δ 10.40 (s, 1H), 8.35-8.23 (m, 2H), 8.10-7.99 (m, 2H), 7.81 (t, J = 7.5 Hz, 1H), 7.41 (d, J = 7.2 Hz, 1H), 4.16 (s, 3H), 4.12-3.92 (m, 6H), 3.90 (s, 3H), 3.82-3.52 (m, 12H), 1.36 (s, 9H).

LC-MS: m/z 672.9 [M+H]⁺.

### Synthesis of Intermediate A46-2

A46-1 (40 mg, 0.060 mmol) was dissolved in dry dichloromethane (1 mL). Trifluoroacetic acid (1 mL) was added under ice bath, and the mixture was stirred at room temperature for 3 hours. The reaction mixture was concentrated. Dichloromethane (2 mL) was added to the residue, and the pH was adjusted to 7-8 with saturated aqueous NaHCO₃ under ice bath. The mixture was concentrated under reduced pressure to afford crude A46-2 (20 mg, 59%), which was used directly in the next step.

LC-MS: m/z 573.0 [M+H]⁺.

### Synthesis of Final Product A46

Intermediate A46-2 (20 mg, 0.035 mmol) and 2-(2,6-dioxopiperidin-3-yl)-5-fluoroisoindoline-1,3-dione (12 mg, 0.042 mmol) were dissolved in dry dimethyl sulfoxide (2 mL). N-Ethyl diisopropylamine (14 mg, 0.10 mmol) was added, and the mixture was stirred at 90 °C for 12 hours. Water (3 mL) was added to precipitate a yellow solid, which was filtered and purified by silica gel column chromatography (dichloromethane/methanol = 20/1, v/v) to affordA46 (4 mg, 14%).

### Example 21: Synthesis of Compound A47

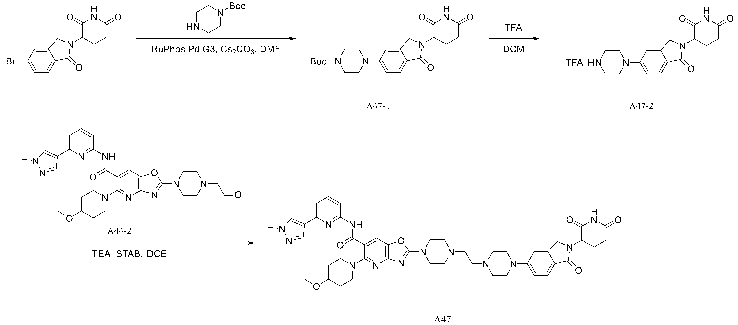

### Synthesis of Intermediate A47-1

3-(5-Bromo-1-oxoisoindol-2-yl)piperidine-2,6-dione (500 mg, 1.6 mmol), 1-(tert-butoxycarbonyl)piperazine (346 mg, 1.9 mmol) and cesium carbonate (1.5 g, 4.7 mmol) were dissolved in dry DMF (10 mL). RuPhos Pd G3 (CAS: 1445085-77-7) (134 mg, 0.16 mmol) was added, and the mixture was stirred at 95 °C under nitrogen protection overnight. After concentration, purification by silica gel column chromatography (dichloromethane/methanol = 20/1, v/v) afforded white solid A47-1 (180 mg, 27%).

LC-MS: m/z 428.9 [M+H]⁺.

### Synthesis of Intermediate A47-2

A47-1 (80 mg, 0.19 mmol) was dissolved in dichloromethane (2 mL). Trifluoroacetic acid (1 mL) was added dropwise, and the mixture was stirred at room temperature for 1 hour. After concentration, crude A47-2 (100 mg) was obtained.

LC-MS: m/z 329.1 [M+H]⁺.

### Synthesis of Final Product A47

Crude A47-2 (35 mg) was dissolved in DCE (1 mL). Triethylamine (27 mg, 0.27 mmol) was added, and the mixture was stirred at room temperature for 30 minutes. A44-2 (30 mg, 0.054 mmol) was added, followed by STAB (34 mg, 0.16 mmol) after another 30 minutes. The mixture was stirred at room temperature for 8 hours. The reaction was quenched with saturated aqueous sodium bicarbonate (2 mL), extracted with dichloromethane twice (10 mL × 2), washed with saturated brine (5 mL), dried over anhydrous sodium sulfate, filtered and concentrated. Purification by preparative silica gel plate (dichloromethane/methanol = 15/1, v/v) afforded A47 (6 mg, 13%).

### Example 22: Synthesis of Compound A49

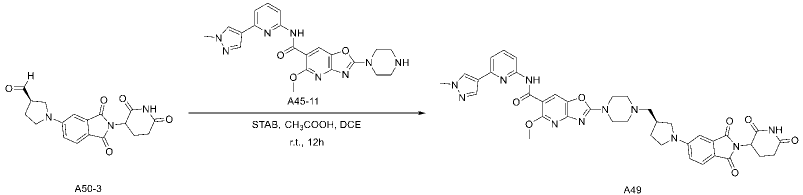

### Synthesis of Final Product A49

A45-11 (44 mg, 0.10 mmol) and A50-3 (40 mg, 0.11 mmol) were dissolved in dry 1,2-dichloroethane (2 mL). One drop of acetic acid and activated molecular sieves (50 mg) were added, and the mixture was stirred at room temperature for 0.5 hour. Sodium triacetoxyborohydride (65 mg, 0.31 mmol) was added, and the mixture was stirred at room temperature for 12 hours. The reaction was quenched with saturated aqueous NH₄Cl solution (1 mL), and the mixture was concentrated under reduced pressure. The residue was purified by silica gel column chromatography (dichloromethane/methanol = 20/1, v/v) to affordA49 (6 mg, 8%).

### Example 23: Synthesis of Compound A50

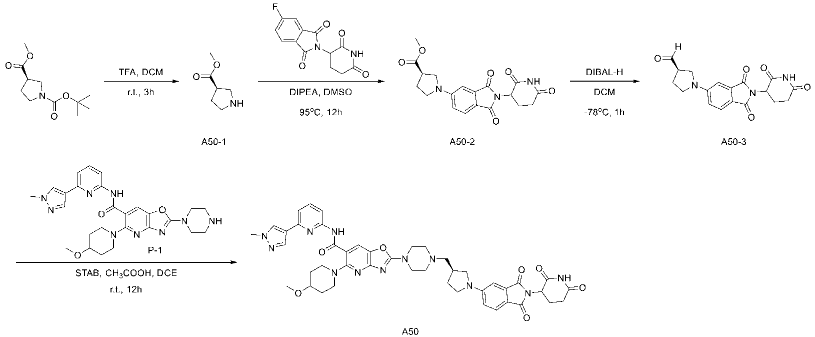

### Synthesis of Intermediate A50-1

Methyl (R)-1-Boc-3-carboxypyrrolidine (400 mg, 1.8 mmol) was dissolved in dry dichloromethane (2 mL). Trifluoroacetic acid (2 mL) was added under ice-water bath, and the mixture was stirred at room temperature for 3 hours. The reaction mixture was concentrated. Dichloromethane (3 mL) was added to the residue, and the pH was adjusted to 7-8 with solid NaHCO₃ powder under ice-water bath. The mixture was filtered and concentrated under reduced pressure to afford oil A50-1 (170 mg, 75%).

¹H NMR (300 MHz, CDCl₃): δ 4.81-4.62 (m, 2H), 3.75 (s, 3H), 3.64-3.22 (m, 4H), 2.52-2.16 (m, 2H).

### Synthesis of Intermediate A50-2

Intermediate A50-1 (170 mg, 1.3 mmol) and 2-(2,6-dioxopiperidin-3-yl)-5-fluoroisoindoline-1,3-dione (280 mg, 1.0 mmol) were dissolved in dry dimethyl sulfoxide (3 mL). N-Ethyl diisopropylamine (523 mg, 4.0 mmol) was added, and the mixture was stirred at 95 °C for 12 hours. Water (3 mL) was added, and the mixture was extracted with ethyl acetate three times (10 mL × 3), washed with saturated NaCl solution three times (10 mL × 3). The combined organic phases were dried over anhydrous sodium sulfate, concentrated under reduced pressure, and purified by silica gel column chromatography (dichloromethane/methanol = 20/1, v/v) to afford yellow solid A50-2 (320 mg, 82%).

¹H NMR (300 MHz, CDCl₃): δ 8.01 (s, 1H), 7.67 (d, J = 8.4 Hz, 1H), 6.96 (s, 1H), 6.70 (d, J = 8.4 Hz, 1H), 5.00-4.88 (m, 1H), 3.75 (s, 3H), 3.72-3.63 (m, 2H), 3.59-3.44 (m, 2H), 3.35-3.22 (m, 1H), 2.96-2.67 (m, 3H), 2.44-2.30 (m, 2H), 2.19-2.08 (m, 1H).

LC-MS: m/z 385.9 [M+H]⁺.

### Synthesis of Intermediate A50-3

Intermediate A50-2 (130 mg, 0.34 mmol) was dissolved in dry DCM (2 mL). 1 mol/L DIBAL-H in tetrahydrofuran (0.74 mL, 0.74 mmol) was added dropwise at -78 °C under nitrogen protection, and the mixture was stirred at -78 °C for 1 hour. The mixture was slowly warmed to 0 °C, and anhydrous methanol (1 mL) was added to quench the reaction. Saturated aqueous citric acid (10 mL) was added, and a white solid appeared. The mixture was stirred under ice-water bath until the solid dissolved. The mixture was extracted with dichloromethane three times (10 mL × 3). The combined organic phases were dried over anhydrous sodium sulfate, concentrated under reduced pressure, and purified by silica gel column chromatography (dichloromethane/methanol = 20/1, v/v) to afford yellow solid A50-3 (60 mg, 50%).

¹H NMR (300 MHz, CDCl₃): δ 9.78 (s, 1H), 8.00 (s, 1H), 7.68 (d, J = 8.4 Hz, 1H), 6.98 (s, 1H), 6.73 (d, J = 8.1 Hz, 1H), 4.99-4.87 (m, 1H), 3.89-3.77 (m, 1H), 3.64-3.39 (m, 3H), 3.35-3.23 (m, 1H), 2.93-2.65 (m, 3H), 2.48-2.27 (m, 2H), 2.18-2.07 (m, 1H).

LC-MS: m/z 387.9 [M+MeOH+H]⁺.

### Synthesis of Final Product A50

P-1 (26 mg, 0.050 mmol) and A50-3 (20 mg, 0.060 mmol) were dissolved in dry 1,2-dichloroethane (2 mL). One drop of acetic acid and activated molecular sieves (50 mg) were added, and the mixture was stirred at room temperature for 0.5 hour. Sodium triacetoxyborohydride (33 mg, 0.15 mmol) was added, and the mixture was stirred at room temperature for 12 hours. The reaction was quenched with saturated aqueous NH₄Cl solution (1 mL), and the mixture was concentrated under reduced pressure. The residue was purified by silica gel column chromatography (dichloromethane/methanol = 20/1, v/v) to afford A50 (4 mg, 9%).

### Example 24: Synthesis of Compound A51

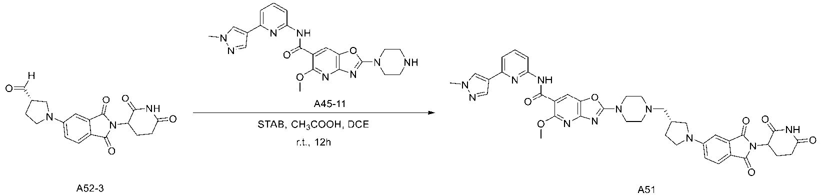

### Synthesis of Final Product A51

A45-11 (50 mg, 0.12 mmol) and A52-3 (45 mg, 0.13 mmol) were dissolved in dry 1,2-dichloroethane (2 mL). One drop of acetic acid and activated molecular sieves (50 mg) were added, and the mixture was stirred at room temperature for 0.5 hour. Sodium triacetoxyborohydride (73 mg, 0.35 mmol) was added, and the mixture was stirred at room temperature for 12 hours. The reaction was quenched with saturated aqueous NH₄Cl solution (1 mL), and the mixture was concentrated under reduced pressure. The residue was purified by silica gel column chromatography (dichloromethane/methanol = 20/1, v/v) to afford A51 (10 mg, 11%).

### Example 25: Synthesis of Compound A52

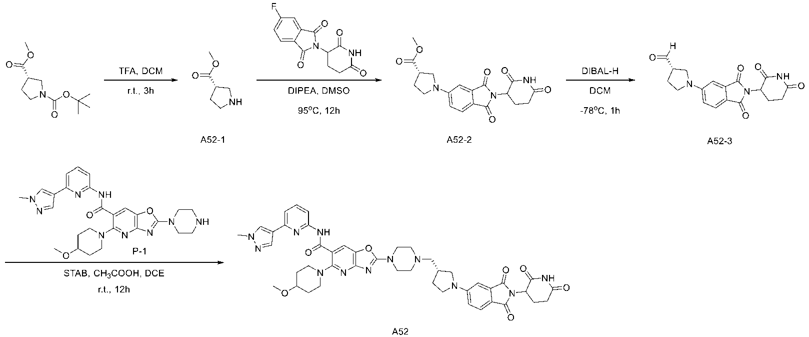

### Synthesis of Intermediate A52-1

Methyl (S)-1-Boc-3-carboxypyrrolidine (300 mg, 1.3 mmol) was dissolved in dry dichloromethane (2 mL). Trifluoroacetic acid (2 mL) was added under ice-water bath, and the mixture was stirred at room temperature for 3 hours. The reaction mixture was concentrated. Dichloromethane (3 mL) was added to the residue, and the pH was adjusted to 7-8 with solid NaHCO₃ powder under ice-water bath. The mixture was filtered and concentrated under reduced pressure to afford crude oily A52-1 (120 mg, 71%), which was used directly in the next step.

### Synthesis of Intermediate A52-2

Intermediate A52-1 (120 mg, 0.93 mmol) and 2-(2,6-dioxopiperidin-3-yl)-5-fluoroisoindoline-1,3-dione (214 mg, 0.78 mmol) were dissolved in dry dimethyl sulfoxide (3 mL). N-Ethyl diisopropylamine (400 mg, 3.10 mmol) was added, and the mixture was stirred at 90 °C for 12 hours. Water (3 mL) was added, and the mixture was extracted with ethyl acetate three times (10 mL × 3), washed with saturated NaCl solution three times (10 mL × 3). The combined organic phases were dried over anhydrous sodium sulfate, concentrated under reduced pressure, and purified by silica gel column chromatography (dichloromethane/methanol = 20/1, v/v) to afford yellow solid A52-2 (200 mg, 67%).

¹H NMR (300 MHz, DMSO-d₆): δ 11.08 (s, 1H), 7.65 (d, J = 8.4 Hz, 1H), 6.93 (s, 1H), 6.84 (d, J = 7.5 Hz, 1H), 5.10-4.99 (m, 1H), 3.66 (s, 3H), 3.63-3.46 (m, 4H), 2.94-2.80 (m, 1H), 2.64-2.52 (m, 2H), 2.36-2.16 (m, 2H), 2.13-1.86 (m, 2H).

LC-MS: m/z 385.9 [M+H]⁺.

### Synthesis of Intermediate A52-3

Intermediate A52-2 (160 mg, 0.42 mmol) was dissolved in dry DCM (2 mL). 1 mol/L DIBAL-H in tetrahydrofuran (0.91 mL, 0.91 mmol) was added dropwise at -78 °C under nitrogen protection, and the mixture was stirred at -78 °C for 1 hour. The mixture was slowly warmed to 0 °C, and anhydrous methanol (1 mL) was added to quench the reaction. Saturated aqueous citric acid (10 mL) was added, and a white solid appeared. The mixture was stirred under ice-water bath until the solid dissolved. The mixture was extracted with dichloromethane three times (10 mL × 3). The combined organic phases were dried over anhydrous sodium sulfate, concentrated under reduced pressure, and purified by silica gel column chromatography (dichloromethane/methanol = 20/1, v/v) to afford yellow solid A52-3 (95 mg, 64%).

¹H NMR (300 MHz, CDCl₃): δ 9.78 (s, 1H), 8.00 (s, 1H), 7.68 (d, J = 8.4 Hz, 1H), 6.98 (s, 1H), 6.73 (d, J = 8.1 Hz, 1H), 5.01-4.87 (m, 1H), 3.90-3.76 (m, 1H), 3.63-3.40 (m, 3H), 3.35-3.23 (m, 1H), 2.94-2.67 (m, 3H), 2.53-2.26 (m, 2H), 2.21-2.06 (m, 1H).

LC-MS: m/z 387.9 [M+MeOH+H]⁺.

### Synthesis of Final Product A52

P-1 (66 mg, 0.13 mmol) and A52-3 (50 mg, 0.14 mmol) were dissolved in dry 1,2-dichloroethane (2 mL). One drop of acetic acid and activated molecular sieves (50 mg) were added, and the mixture was stirred at room temperature for 0.5 hour. Sodium triacetoxyborohydride (81 mg, 0.38 mmol) was added, and the mixture was stirred at room temperature for 12 hours. The reaction was quenched with saturated aqueous NH₄Cl solution (1 mL), and the mixture was concentrated under reduced pressure. The residue was purified by silica gel column chromatography (dichloromethane/methanol = 20/1, v/v) to afford A52 (13 mg, 12%).

### Example 26: Synthesis of Compound A53

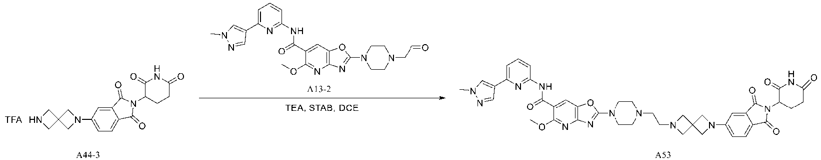

### Synthesis of Final Product A53

Crude A44-3 (40 mg) was dissolved in DCE (1 mL). Triethylamine (32 mg, 0.32 mmol) was added, and the mixture was stirred at room temperature for 30 minutes. A13-2 (30 mg, 0.063 mmol) was added, followed by STAB (40 mg, 0.19 mmol) after another 30 minutes. The mixture was stirred at room temperature for 8 hours. The reaction was quenched with saturated aqueous sodium bicarbonate (2 mL), extracted with dichloromethane twice (10 mL × 2), washed with saturated brine (5 mL), dried over anhydrous sodium sulfate, filtered and concentrated. Purification by preparative silica gel plate (dichloromethane/methanol = 15/1, v/v) afforded A53 (22 mg, 43%).

### Example 27: Synthesis of Compound A55

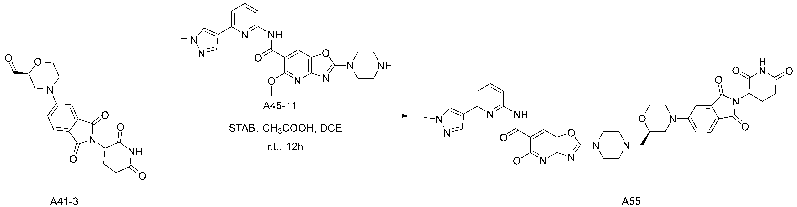

### Synthesis of Final Product A55

A41-3 (40 mg, 0.11 mmol) and A45-11 (43 mg, 0.10 mmol) were dissolved in dry 1,2-dichloroethane (3 mL). One drop of acetic acid and activated molecular sieves (50 mg) were added, and the mixture was stirred at room temperature for 0.5 hour. Sodium triacetoxyborohydride (62 mg, 0.29 mmol) was added, and the mixture was stirred at room temperature for 12 hours. The reaction was quenched with saturated aqueous NH₄Cl solution (1 mL), and the mixture was concentrated under reduced pressure. The residue was purified by silica gel column chromatography (dichloromethane/methanol = 20/1, v/v) to afford A55 (17 mg, 22%).

### Example 28: Synthesis of Compound A56

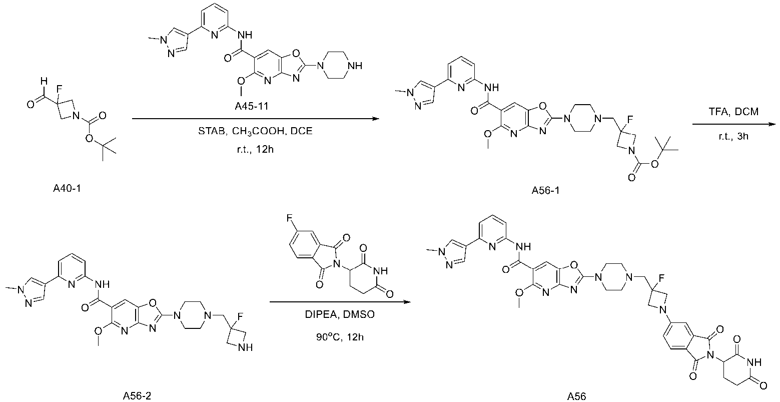

### Synthesis of Intermediate A56-1

A45-11 (100 mg, 0.23 mmol) and A40-1 (94 mg, 0.46 mmol) were dissolved in dry 1,2-dichloroethane (3 mL). One drop of acetic acid and activated molecular sieves (50 mg) were added, and the mixture was stirred at room temperature for 0.5 hour. Sodium triacetoxyborohydride (147 mg, 0.69 mmol) was added, and the mixture was stirred at room temperature for 12 hours. The reaction was quenched with saturated aqueous NH₄Cl solution (1 mL), and the mixture was concentrated under reduced pressure. The residue was purified by silica gel column chromatography (dichloromethane/methanol = 20/1, v/v) to afford gray solid A56-1 (65 mg, 45%).

¹H NMR (300 MHz, DMSO-d₆) δ 10.39 (s, 1H), 8.37-8.18 (m, 2H), 8.15-7.95 (m, 2H), 7.80 (t, J = 7.8 Hz, 1H), 7.40 (d, J = 7.5 Hz, 1H), 4.16 (s, 3H), 4.09-3.78 (m, 7H), 3.77-3.57 (m, 4H), 2.98-2.79 (m, 2H), 2.78-2.56 (m, 4H), 1.39 (s, 9H).

LC-MS: m/z 621.9 [M+H]⁺.

### Synthesis of Intermediate A56-2

A56-1 (65 mg, 0.10 mmol) was dissolved in dry dichloromethane (1 mL). Trifluoroacetic acid (1 mL) was added under ice-water bath, and the mixture was stirred at room temperature for 3 hours. The reaction mixture was concentrated. Dichloromethane (2 mL) was added to the residue, and the pH was adjusted to 7-8 with saturated aqueous NaHCO₃ under ice-water bath. The mixture was concentrated under reduced pressure to afford crude A56-2 (35 mg, 64%), which was used directly in the next step.

LC-MS: m/z 521.9 [M+H]⁺.

### Synthesis of Final Product A56

A56-2 (35 mg, 0.067 mmol) and 2-(2,6-dioxopiperidin-3-yl)-5-fluoroisoindoline-1,3-dione (20 mg, 0.074 mmol) were dissolved in dry dimethyl sulfoxide (2 mL). N-Ethyl diisopropylamine (15 mg, 0.12 mmol) was added, and the mixture was stirred at 90 °C for 12 hours. Water (2 mL) was added to precipitate a yellow solid. The mixture was extracted with ethyl acetate three times (10 mL × 3), washed with saturated NaCl solution three times (10 mL × 3). The combined organic phases were dried over anhydrous sodium sulfate, concentrated under reduced pressure, and purified by silica gel column chromatography (dichloromethane/methanol = 20/1, v/v) to afford A56 (8 mg, 15%).

### Example 29: Synthesis of Compound A57

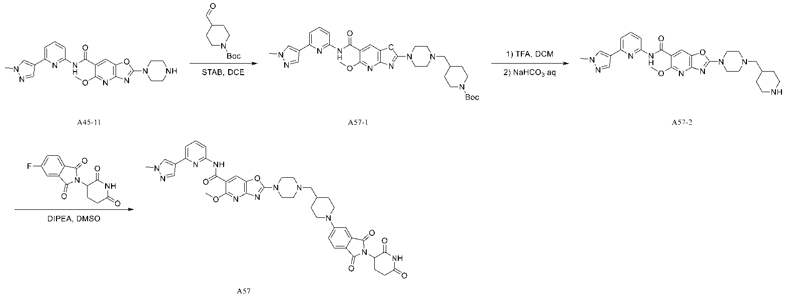

### Synthesis of Intermediate A57-1

A45-11 (50 mg, 0.12 mmol) and 1-Boc-piperidine-4-carboxaldehyde (30 mg, 0.14 mmol) were dissolved in DCE (1.5 mL) and stirred at room temperature for 30 minutes. Sodium triacetoxyborohydride (50 mg, 0.24 mmol) was added, and the mixture was stirred at room temperature overnight. The reaction was quenched with saturated aqueous sodium bicarbonate (5 mL), extracted with dichloromethane (20 mL × 2), washed with saturated brine (10 mL), dried over anhydrous sodium sulfate, filtered and concentrated. Purification by silica gel column chromatography (dichloromethane/methanol = 30/1, v/v), followed by trituration with diethyl ether (10 mL), filtration and drying afforded pale yellow solid A57-1 (50 mg, 68%).

LC-MS: m/z 631.9 [M+H]⁺.

### Synthesis of Intermediate A57-2

A57-1 (50 mg, 0.079 mmol) was dissolved in dichloromethane (2 mL). Trifluoroacetic acid (1 mL) was added, and the mixture was stirred at room temperature for 1 hour. After concentration, the residue was adjusted to pH 7-8 with saturated aqueous sodium bicarbonate, extracted with dichloromethane (20 mL × 2), dried over anhydrous sodium sulfate, filtered and concentrated. Trituration with diethyl ether (10 mL), filtration and drying afforded white solid (25 mg, 60%).

LC-MS: *m*/*z* 531.9 [M+H]⁺.

### Synthesis of Final Product A57

A57-2 (25 mg, 0.047 mmol) was dissolved in dimethyl sulfoxide (0.6 mL). 2-(2,6-Dioxopiperidin-3-yl)-5-fluoroisoindoline-1,3-dione (14 mg, 0.052 mmol) and DIPEA (31 mg, 0.24 mmol) were added, and the mixture was stirred at 90 °C overnight. The mixture was extracted with dichloromethane (15 mL), washed with water (5 mL × 3) and saturated brine (5 mL), dried over anhydrous sodium sulfate, filtered and concentrated. Purification by preparative silica gel plate (dichloromethane/methanol = 15/1, v/v) afforded A57 (13 mg, 35%).

### Example 30: Synthesis of Compound A59

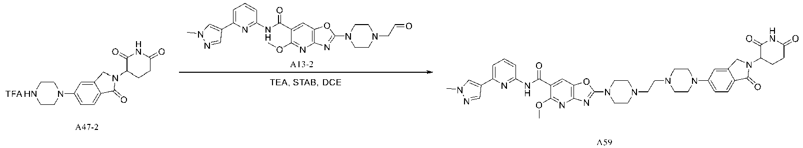

### Synthesis of Final Product A59

Crude A47-2 (42 mg) was dissolved in DCE (1 mL). Triethylamine (32 mg, 0.32 mmol) was added, and the mixture was stirred at room temperature for 30 minutes. A13-2 (30 mg, 0.063 mmol) was added, followed by STAB (40 mg, 0.19 mmol) after another 30 minutes. The mixture was stirred at room temperature for 8 hours. The reaction was quenched with saturated aqueous sodium bicarbonate (2 mL), extracted with dichloromethane twice (10 mL × 2), washed with saturated brine (5 mL), dried over anhydrous sodium sulfate, filtered and concentrated. Purification by preparative silica gel plate (dichloromethane/methanol = 15/1, v/v) afforded A59 (20 mg, 40%).

### Example 31: Synthesis of Compound A60

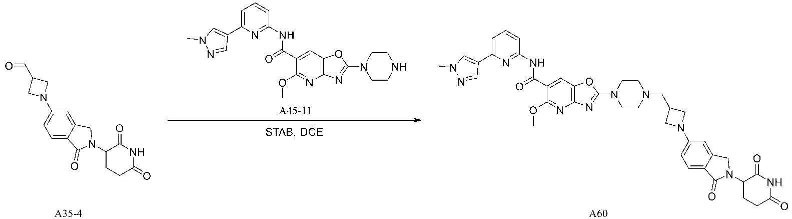

### Synthesis of Final Product A60

Crude A35-4 (50 mg) was dissolved in DCE (2 mL). A45-11 (50 mg, 0.12 mmol) was added, and the mixture was stirred at room temperature for 30 minutes. STAB (74 mg, 0.35 mmol) was added, and the mixture was stirred overnight. The reaction was quenched with saturated aqueous sodium bicarbonate (3 mL), extracted with dichloromethane twice (20 mL × 2), washed with saturated brine (10 mL), dried over anhydrous sodium sulfate, filtered and concentrated. Purification by silica gel column chromatography (dichloromethane/methanol = 10/1, v/v) afforded A60 (45 mg, 52%).

### Example 32: Synthesis of Compound A61

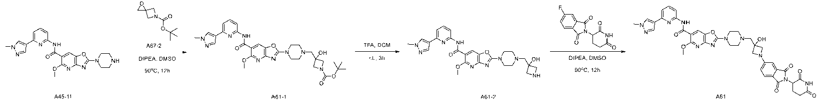

### Synthesis of Intermediate A61-1

Intermediate A45-11 (60 mg, 0.14 mmol) and A67-2 (38 mg, 0.21 mmol) were dissolved in dry dimethyl sulfoxide (2 mL). N-Ethyl diisopropylamine (36 mg, 0.28 mmol) was added, and the mixture was stirred at 90 °C for 12 hours. Water (2 mL) was added, and the mixture was extracted with ethyl acetate three times (10 mL × 3), washed with saturated NaCl solution three times (10 mL × 3). The combined organic phases were dried over anhydrous sodium sulfate, concentrated under reduced pressure, and purified by silica gel column chromatography (dichloromethane/methanol = 20/1, v/v) to afford gray solid A61-1 (60 mg, 70%).

¹H NMR (300 MHz, DMSO-d₆) δ 10.39 (s, 1H), 8.29 (s, 1H), 8.23 (s, 1H), 8.11-7.94 (m, 2H), 7.80 (t, J = 7.8 Hz, 1H), 7.41 (d, J = 7.5 Hz, 1H), 5.71 (s, 1H), 4.16 (s, 3H), 3.90 (s, 3H), 3.83-3.76 (m, 2H), 3.74-3.59 (m, 6H), 2.76-2.62 (m, 4H), 2.61-2.55 (m, 2H), 1.38 (s, 9H).

LC-MS: m/z 619.9 [M+H]⁺.

### Synthesis of Intermediate A61-2

A61-1 (60 mg, 0.097 mmol) was dissolved in dry dichloromethane (1 mL). Trifluoroacetic acid (1 mL) was added under ice-water bath, and the mixture was stirred at room temperature for 3 hours. The reaction mixture was concentrated. Dichloromethane (2 mL) was added to the residue, and the pH was adjusted to 7-8 with saturated aqueous NaHCO₃ under ice-water bath. The mixture was concentrated under reduced pressure to afford crude A61-2 (40 mg, 80%), which was used directly in the next step.

LC-MS: m/z 519.9 [M+H]⁺.

### Synthesis of Final Product A61

Intermediate A61-2 (40 mg, 0.077 mmol) and 2-(2,6-dioxopiperidin-3-yl)-5-fluoroisoindoline-1,3-dione (26 mg, 0.092 mmol) were dissolved in dry dimethyl sulfoxide (2 mL). N-Ethyl diisopropylamine (20 mg, 0.15 mmol) was added, and the mixture was stirred at 90 °C for 12 hours. Water (2 mL) was added to precipitate a yellow solid. The mixture was extracted with ethyl acetate three times (10 mL × 3), washed with saturated NaCl solution three times (10 mL × 3). The combined organic phases were dried over anhydrous sodium sulfate, concentrated under reduced pressure, and purified by silica gel column chromatography (dichloromethane/methanol = 20/1, v/v) to afford A61 (20 mg, 34%).

### Example 33: Synthesis of Compound A64

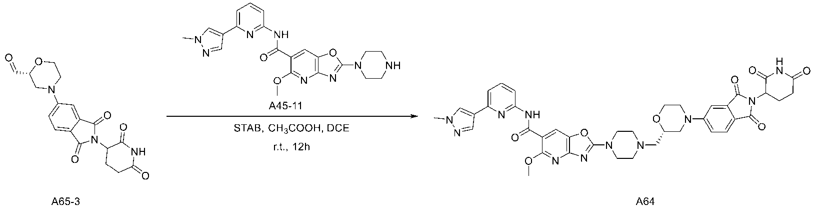

### Synthesis of Final Product A64

A65-3 (40 mg, 0.11 mmol) and A45-11 (39 mg, 0.090 mmol) were dissolved in dry 1,2-dichloroethane (3 mL). One drop of acetic acid and activated molecular sieves (50 mg) were added, and the mixture was stirred at room temperature for 0.5 hour. Sodium triacetoxyborohydride (57 mg, 0.27 mmol) was added, and the mixture was stirred at room temperature for 12 hours. The reaction was quenched with saturated aqueous NH₄Cl solution (1 mL), and the mixture was concentrated under reduced pressure. The residue was purified by silica gel column chromatography (dichloromethane/methanol = 20/1, v/v) to afford A64 (20 mg, 28%).

### Example 34: Synthesis of Compound A65

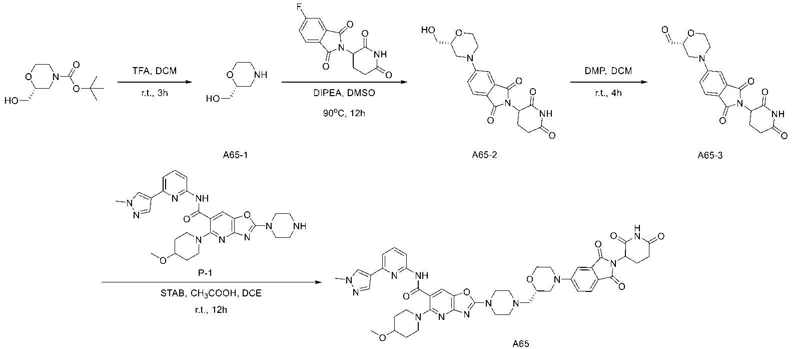

### Synthesis of Intermediate A65-1

(R)-N-Boc-2-(hydroxymethyl)morpholine (250 mg, 1.2 mmol) was dissolved in dry dichloromethane (2 mL). Trifluoroacetic acid (2 mL) was added under ice-water bath, and the mixture was stirred at room temperature for 3 hours. The reaction mixture was concentrated. Dichloromethane (2 mL) was added to the residue, and the pH was adjusted to 7-8 with solid NaHCO₃ powder under ice-water bath. The mixture was filtered and concentrated under reduced pressure to afford crude A65-1 (120 mg, 89%), which was used directly in the next step.

### Synthesis of Intermediate A65-2

Intermediate A65-1 (120 mg, 1.0 mmol) and 2-(2,6-dioxopiperidin-3-yl)-5-fluoroisoindoline-1,3-dione (236 mg, 0.85 mmol) were dissolved in dry dimethyl sulfoxide (3 mL). N-Ethyl diisopropylamine (441 mg, 3.4 mmol) was added, and the mixture was stirred at 90 °C for 12 hours. Water (2 mL) was added, and the mixture was extracted with ethyl acetate three times (10 mL × 3), washed with saturated NaCl solution three times (10 mL × 3). The combined organic phases were dried over anhydrous sodium sulfate, concentrated under reduced pressure, and purified by silica gel column chromatography (dichloromethane/methanol = 20/1, v/v) to affordA65-2 (200 mg, 63%).

¹H NMR (300 MHz, DMSO-d₆) δ 11.09 (s, 1H), 7.71 (d, J = 8.1 Hz, 1H), 7.35 (s, 1H), 7.27 (d, J = 7.8 Hz, 1H), 5.14-5.03 (m, 1H), 4.84 (t, J = 4.8 Hz, 1H), 4.01-3.79 (m, 3H), 3.67-3.43 (m, 4H), 2.99-2.82 (m, 2H), 2.75-2.52 (m, 3H), 2.08-1.96 (m, 1H).

LC-MS: m/z 373.9 [M+H]⁺.

### Synthesis of Intermediate A65-3

Intermediate A65-2 (140 mg, 0.38 mmol) was dissolved in dry dichloromethane (2 mL). Dess-Martin periodinane (191 mg, 0.45 mmol) was added under ice-water bath, and the mixture was stirred at room temperature for 3 hours. Saturated aqueous sodium sulfite (2 mL) and saturated aqueous sodium bicarbonate (4 mL) were added to quench the reaction. The mixture was extracted with dichloromethane three times (15 mL × 3). The combined organic phases were dried over anhydrous sodium sulfate, concentrated under reduced pressure, and purified by silica gel column chromatography (dichloromethane/methanol = 20/1, v/v) to afford yellow solid A65-3 (95 mg, 68%).

LC-MS: m/z 403.9 [M+MeOH+H]⁺.

### Synthesis of Final Product A65

A65-3 (55 mg, 0.15 mmol) and P-1 (70 mg, 0.13 mmol) were dissolved in dry 1,2-dichloroethane (3 mL). One drop of acetic acid and activated molecular sieves (50 mg) were added, and the mixture was stirred at room temperature for 0.5 hour. Sodium triacetoxyborohydride (86 mg, 0.40 mmol) was added, and the mixture was stirred at room temperature for 12 hours. The reaction was quenched with saturated aqueous NH₄Cl solution (1 mL), and the mixture was concentrated under reduced pressure. The residue was purified by silica gel column chromatography (dichloromethane/methanol = 20/1, v/v) to afford A65 (25 mg, 21%).

### Example 35: Synthesis of Compound A67

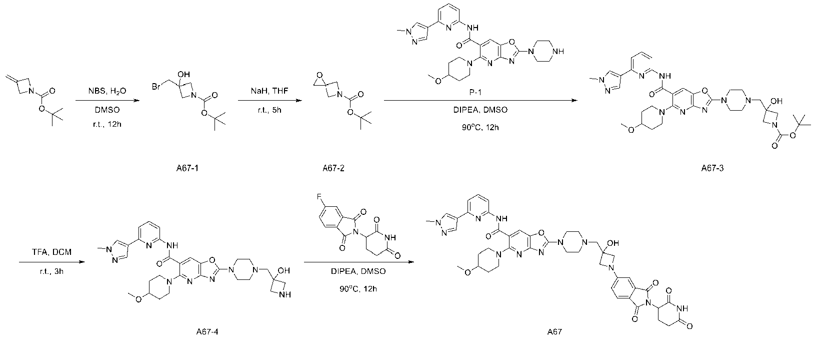

### Synthesis of Intermediate A67-1

tert-Butyl 3-methyleneazetidine-1-carboxylate (1.0 g, 5.9 mmol) was dissolved in dry dimethyl sulfoxide (5 mL). N-Bromosuccinimide (2.1 g, 12 mmol) and water (213 mg, 12 mmol) were added under ice-water bath, and the mixture was stirred at room temperature overnight. Water (5 mL) was added, and the mixture was extracted with ethyl acetate three times (10 mL × 3), washed with saturated NaCl solution three times (10 mL × 3). The combined organic phases were dried over anhydrous sodium sulfate, concentrated under reduced pressure, and purified by silica gel column chromatography (dichloromethane/methanol = 50/1, v/v) to afford a colorless oily mixture of isomers A67-1 (1.2 g, 76%), which was used directly in the next step.

### Synthesis of Intermediate A67-2

Intermediate A67-1 (300 mg, 1.1 mmol) was dissolved in dry tetrahydrofuran (5 mL). 60% Sodium hydride (68 mg, 1.7 mmol) was added under ice-water bath and nitrogen protection, and the mixture was stirred at room temperature for 5 hours. Water (1 mL) was added to quench the reaction. The mixture was extracted with ethyl acetate three times (10 mL × 3). The combined organic phases were dried over anhydrous sodium sulfate, concentrated under reduced pressure, and purified by silica gel column chromatography (dichloromethane/methanol = *50*/*1,* v/v) to afford pale yellow oil A67-2 (25 mg, 12%).

¹H NMR (300 MHz, CDCl₃) δ 4.29-4.15 (m, 1H), 2.85 (s, 2H), 1.45 (s, 9H).

### Synthesis of Intermediate A67-3

Intermediate P-1 (58 mg, 0.11 mmol) and A67-2 (25 mg, 0.14 mmol) were dissolved in dry dimethyl sulfoxide (2 mL). N-Ethyl diisopropylamine (29 mg, 0.23 mmol) was added, and the mixture was stirred at 90 °C for 12 hours. Water (2 mL) was added, and the mixture was extracted with ethyl acetate three times (10 mL × 3), washed with saturated NaCl solution three times (10 mL × 3). The combined organic phases were dried over anhydrous sodium sulfate, concentrated under reduced pressure, and purified by silica gel column chromatography (dichloromethane/methanol = 20/1, v/v) to afford gray solid A67-3 (45 mg, 57%).

¹H NMR (300 MHz, DMSO-d₆) δ 13.41 (s, 1H), 8.38-8.16 (m, 2H), 8.11-7.94 (m, 2H), 7.79 (t, J = 7.2 Hz, 1H), 7.39 (d, J = 6.3 Hz, 1H), 5.68 (s, 1H), 3.88 (s, 3H), 3.83-3.74 (m, 2H), 3.73-3.54 (m, 6H), 3.51-3.43 (m, 2H), 3.31-3.19 (m, 4H), 3.08-2.96 (m, 2H), 2.73-2.53 (m, 6H), 2.23-1.98 (m, 4H), 1.38 (s, 9H).

LC-MS: m/z 702.9 [M+H]⁺.

### Synthesis of Intermediate A67-4

Intermediate A67-3 (45 mg, 0.064 mmol) was dissolved in dry dichloromethane (1 mL). Trifluoroacetic acid (1 mL) was added under ice-water bath, and the mixture was stirred at room temperature for 3 hours. The reaction mixture was concentrated. Dichloromethane (2 mL) was added to the residue, and the pH was adjusted to 7-8 with saturated aqueous NaHCO₃ under ice-water bath. The mixture was concentrated under reduced pressure to afford crude A67-4 (35 mg, 91%), which was used directly in the next step.

LC-MS: m/z 603.1 [M+H]⁺.

### Synthesis of Final Product A67

### Intermediate A67-4 (35 mg, 0.058 mmol) and

2-(2,6-dioxopiperidin-3-yl)-5-fluoroisoindoline-1,3-dione (19 mg, 0.070 mmol) were dissolved in dry dimethyl sulfoxide (2 mL). N-Ethyl diisopropylamine (15 mg, 0.12 mmol) was added, and the mixture was stirred at 90 °C for 12 hours. Water (2 mL) was added to precipitate a yellow solid. The mixture was extracted with ethyl acetate three times (10 mL × 3), washed with saturated NaCl solution three times (10 mL × 3). The combined organic phases were dried over anhydrous sodium sulfate, concentrated under reduced pressure, and purified by silica gel column chromatography (dichloromethane/methanol = 20/1, v/v) to afford A67 (16 mg, 32%).

**Table 1 Structural formulas and Spectral Data of Compounds**

| NO. | Structure | ¹H-NMR & LC-MS |
|---|---|---|
| A1 | 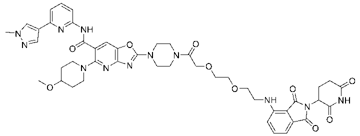 | ¹H NMR (300 MHz, DMSO-*d₆*): δ 13.41 (s, 1H), 11.09 (s, 1H), 8.28 (s, 1H), 8.19 (s, 1H), 8.11-7.97 (m, 2H), 7.79 (t, *J* = 7.5 Hz, 1H), 7.52 (t, *J =* 7.8 Hz, 1H), 7.40 (d, *J =* 7.5 Hz, 1H), 7.11 (d, *J =* 7.5 Hz, 1H), 6.91 (d, *J= 6.9* Hz, 1H), 6.57 (s, 1H), 5.09-4.99 (m, 1H), 4.29-4.14 (m, 2H), 3.88 (s, 3H), 3.78-3.70 (m, 2H), 3.69-3.63 (m, 4H), 3.60-3.53 (m, 4H), 3.51-3.42 (m, 6H), 3.27-3.19 (m, 2H), 3.17-3.08 (m, 4H), 3.06-2.96 (m, 2H), 2.92-2.81 (m, 1H), 2.73-2.65 (m, 1H), 2.63-2.53 (m, 2H), 2.25-2.14 (m, 2H), 2.13-2.02 (m, 2H). LC-MS: m/z 918.9 [M+H]⁺. |
| A2 | 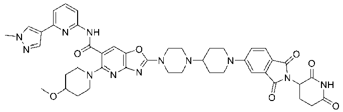 | ¹H NMR (300 MHz, DMSO-*d₆*): δ 13.43 (s, 1H), 11.09 (s, 1H), 8.32-8.23 (m, 2H), 8.10-7.99 (m, 2H), 7.80 (t, *J =* 7.5 Hz, 1H), 7.66 (d, *J* = 7.8 Hz, 1H), 7.40 (d, *J* = 7.8 Hz, 1H), 7.34 (s, 1H), 7.29-7.22 (m, 1H), 5.10-5.01 (m, 1H), 4.18-4.02 (m, 2H), 3.88 (s, 3H), 3.73-3.66 (m, 3H), 3.46 (s, 3H), 3.29-3.20 (m, 2H), 3.06-2.95 (m, 4H), 2.91-2.76 (m, 1H), 2.66-2.54 (m, 6H), 2.53-2.50 (m, 2H),2.24-2.15 (m, 2H), 2.13-1.94 (m, 4H), 1.91-1.77 (m, 2H), 1.56-1.40 (m, 2H). LC-MS: m/z 429.3 [(M+2H)/2]²⁺. |
| A3 | 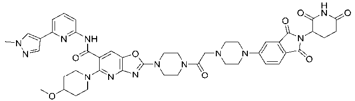 | ¹H NMR (300 MHz, DMSO-*d₆*): δ 13.43 (s, 1H), 11.09 (s, 1H), 8.29 (s, 1H), 8.28 (s, 1H), 8.10-8.01 (m, 2H), 7.83-7.76 (m, 1H), 7.68 (d, *J* = 8.3 Hz, 1H), 7.43-7.38 (m, 1H), 7.35 (s, 1H), 7.29-7.23 (m, 1H), 5.13-5.01 (m, 2H), 3.88 (s, 3H), 3.83-3.73 (m, 4H), 3.73-3.64 (m, 4H), 3.57-3.53 (m, 4H), 3.30 (s, 3H), 3.27-3.17 (m, 4H), 3.05-2.96 (m, 2H), 2.90-2.79 (m, 2H), 2.64-2.55 (m, 4H), 2.16-2.04 (m, 6H). LC-MS: m/z 450.8 [(M+2H)/2]²⁺. |
| A4 | 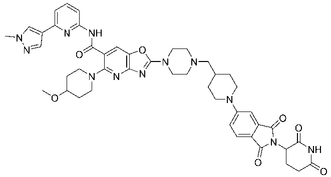 | ¹H NMR (300 MHz, DMSO-*d₆*): δ 13.43 (s, 1H), 11.09 (s, 1H), 8.38-8.18 (m, 2H), 8.14-7.97 (m, 2H), 7.80 (t, *J =* 8.4 Hz, 1H), 7.66 (d, *J =* 8.4 Hz, 1H), 7.40 (d, *J =* 7.5 Hz, 1H), 7.32 (s, 1H), 7.24 (d, *J =* 8.4 Hz, 1H), 5.12-5.00 (m, 1H), 4.17-3.97 (m, 2H), 3.88 (s, 3H), 3.78-3.56 (m, 4H), 3.52-3.36 (m, 6H), 3.28-3.13 (m, 4H), 3.08-2.90 (m, 4H), 2.88-2.67 (m, 2H), 2.65-2.55 (m, 2H), 2.32-2.15 (m, 4H), 2.13-1.97 (m, 3H), 1.88-1.77 (m, 2H), 1.25-1.13 (m, 2H). LC-MS: m/z 871.4 [M+H]⁺. |
| A5 | 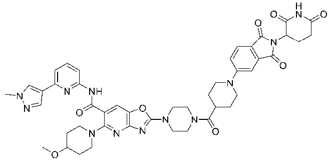 | ¹H NMR (300 MHz, DMSO-*d₆*): δ 13.43 (s, 1H), 11.10 (s, 1H), 8.31 (s, 1H), 8.28 (s, 1H), 8.11-8.02 (m, 2H), 7.80 (t, *J =* 7.8 Hz, 1H), 7.67 (d, *J =* 8.4 Hz, 1H), 7.40 (d, *J =* 7.8 Hz, 1H), 7.35 (s, 1H), 7.26 (d, *J* = 8.1 Hz, 1H), 5.07- 4.90 (m, 1H), 4.16-4.02 (m, 2H), 3.88 (s, 3H), 3.81-3.60 (m, 10H), 3.46 (s, 3H), 3.29-3.19 (m, 4H), 3.11-2.96 (m, 4H), 2.63-2.54 (m, 2H), 2.20-2.02 (m, 4H), 1.81-1.55 (m, 4H). LC-MS: m/z 885.4 [M+H]⁺. |
| A6 | 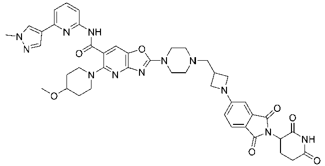 | ¹H NMR (300 MHz, DMSO-*d₆*): δ 13.43 (s, 1H), 11.09 (s, 1H), 8.40-8.21 (m, 2H), 8.15-7.95 (m, 2H), 7.80 (t, *J =* 7.2 Hz, 1H), 7.64 (d, *J=* 8.1 Hz, 1H), 7.40 (d, *J=* 7.5 Hz, 1H), 6.78 (s, 1H), 6.65 (d, *J* = 8.4 Hz, 1H), 5.11-5.00 (m, 1H), 4.24-4.05 (m, 2H), 3.88 (s, 3H), 3.78-3.65 (m, 4H), 3.52-3.36 (m, 6H), 3.28-3.17 (m, 2H), 3.10-2.92 (m, 2H), 2.90-2.80 (m, 1H), 2.78-2.64 (m, 2H), 2.63-2.50 (m, 8H), 2.21-1.98 (m, 4H). LC-MS: m/z 843.4 [M+H]⁺. |
| A7 | 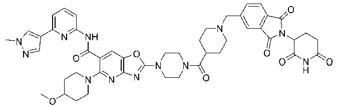 | ¹H NMR (300 MHz,DMSO-*d*₆): δ 13.42 (s, 1H), 11.14 (s, 1H), 8.30 (s, 1H), 8.28 (s, 1H), 8.09-8.01 (m, 2H), 7.92-7.87 (m, 1H), 7.85 (s, 1H), 7.83 (s, 1H), 7.80-7.75 (m, 1H), 7.40 (d, J = 7.6 Hz, 1H), 5.20-5.10 (m, 1H), 3.88 (s, 3H), 3.74-3.59 (m, 10H), 3.51-3.43 (m, 2H), 3.38 (s, 3H), 3.26-3.18 (m, 2H), 3.07-2.96 (m, 2H), 2.90-2.81 (m, 2H), 2.72-2.55 (m, 4H), 2.19-2.00 (m, 6H), 1.78-1.53 (m, 4H). LC-MS: m/z899.4 [M+H]⁺. |
| A8 | 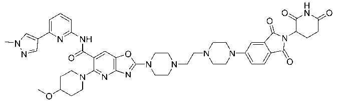 | ¹H NMR (300 MHz, DMSO-*d₆*): δ 13.43 (s, 1H), 11.08 (s, 1H), 8.35-8.25 (m, 1H), 8.12-8.05 (m, 2H), 8.05 (s, 1H), 7.83-7.75 (m, 1H), 7.68 (d, *J =* 8.4 Hz, 1H), 7.40 (d, *J =* 7.8 Hz, 1H), 7.34 (s, 1H), 7.26 (d, *J =* 8.7 Hz, 1H), 5.12-5.02 (m, 1H), 3.88 (s, 3H), 3.80-3.63 (m, 4H), 3.41-3.30 (m, 6H), 3.29-3.16 (m, 8H), 3.08-2.70 (m, 6H), 2.53-2.30 (m, 6H), 2.29-1.93 (m, 6H). LC-MS: m/z 886.4 [M+H]⁺. |
| A9 | 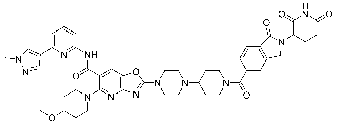 | ¹H NMR (300 MHz, DMSO-*d₆*): δ 13.42 (s, 1H), 11.01 (s, 1H), 8.32-8.18 (m, 2H), 8.10-7.97 (m, 2H), 7.84-7.72 (m, 2H), 7.64 (s, 1H), 7.51 (d, *J=* 6.9 Hz, 1H), 7.40 (d, *J= 7.8* Hz, 1H), 5.16-5.09 (m, 1H), 4.50-4.36 (m, 2H), 3.88 (s, 3H), 3.75-3.68 (m, 4H), 3.64-3.55 (m, 6H), 3.29-3.15 (m, 4H), 3.08-2.97 (m, 2H), 2.91-2.83 (m, 1H), 2.82-2.58 (m, 8H), 2.30-1.95 (m, 4H), 1.92-1.66 (m, 2H), 1.54-1.32 (m, 2H). LC-MS: m/z 871.4 [M+H]⁺. |
| A11 | 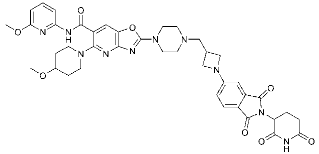 | ¹H NMR (300 MHz, DMSO-*d₆*): δ 13.20 (s, 1H), 11.07 (s, 1H), 8.26 (s, 1H), 7.85 (d, *J =* 7.2 Hz, 1H), 7.73 (t, *J =* 7.8 Hz, 1H), 7.64 (d, *J* = 7.8 Hz, 1H), 6.78 (s, 1H), 6.65 (d, *J* = 7.2 Hz, 1H), 6.56 (d, *J* = 7.5 Hz, 1H), 5.11-4.99 (m, 1H), 4.26-4.08 (m, 2H), 3.85 (s, 3H), 3.79-3.65 (m, 4H), 3.47-3.34 (m, 4H), 3.31-3.25 (m, 6H), 3.24-3.16 (m, 2H), 3.03-2.92 (m, 2H), 2.79-2.65 (m, 2H), 2.64-2.52 (m, 5H), 2.22-2.07 (m, 2H), 2.01-1.88 (m, 2H). LC-MS: m/z 792.9 [M+H]⁺. |
| A12 | 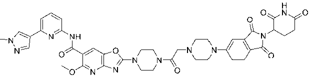 | ¹H NMR (400 MHz, DMSO-*d₆*): δ 11.08 (s, 1H), 10.39 (s, 1H), 8.28 (s, 1H), 8.26 (s, 1H), 8.07-7.97 (m, 2H), 7.80 (t, *J* = 8.0 Hz, 1H), 7.68 (d, *J* = 8.4 Hz, 1H), 7.41 (d, *J* = 8.0 Hz, 1H), 7.35 (s, 1H), 7.27 (d, *J* = 8.8 Hz, 1H), 5.11-5.02 (m, 1H), 4.16 (s, 3H), 3.90 (s, 3H), 3.84-3.74 (m, 4H), 3.73-3.63 (m, 4H), 3.50-3.45 (m, 4H), 3.32-3.29 (m, 4H), 2.93-2.82 (m, 1H), 2.63-2.57 (m, 4H), 2.07-1.97 (m, 1H). LC-MS: m/z 816.8 [M+H]⁺. |
| A13 | 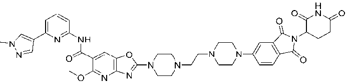 | ¹H NMR (300 MHz, DMSO-*d₆*): δ 11.10 (s, 1H), 10.39 (s, 1H), 8.31-8.20 (m, 2H), 8.07-7.98 (m, 2H), 7.84-7.76 (m, 1H), 7.71-7.64 (m, 1H), 7.44-7.38 (m, 1H), 7.34 (s, 1H), 7.28-7.22 (m, 1H), 5.10-5.01 (m, 1H), 4.15 (s, 3H), 3.90 (s, 3H), 3.73-3.65 (m, 4H), 3.29-3.25 (m, 6H), 2.96-2.73 (m, 2H), 2.61-2.53 (m, 12H).LC-MS: m/z 802.8 [M+H]⁺. |
| A35 | 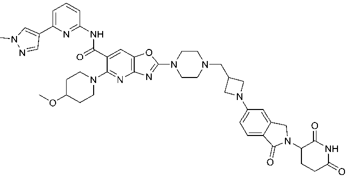 | ¹H NMR (300 MHz, DMSO-*d₆*) δ 13.43 (s, 1H), 10.94 (s, 1H), 8.30 - 8.24 (m, 2H), 8.08 - 8.03 (m, 2H), 7.80 (t, *J =* 8.1 Hz, 1H), 7.48 (d, *J =* 8.4 Hz, 1H), 7.40 (d, *J =* 7.5 Hz, 1H), 6.53 - 6.45 (m, 2H), 5.05 - 4.99 (m, 1H), 4.33 - 4.26 (m, 1H), 4.20 - 4.13 (m, 1H), 4.06 - 4.00 (m, 2H), 3.88 (s, 3H), 3.74 - 3.68 (m, 4H), 3.59 (s, 3H), 3.27 - 3.19 (m, 5H), 3.07 - 2.95 (m, 4H), 2.90 - 2.83 (m, 1H), 2.70 - 2.65 (m, 2H), 2.58 - 2.54 (m, 4H), 2.38 - 2.25 (m, 2H), 2.21 - 2.14 (m, 2H), 2.09 - 2.04 (m, 1H), 1.98 - 1.90 (m, 1H). LC-MS (m/z): 828.9 [ M + H ]⁺ |
| A37 | 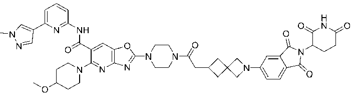 | ¹H NMR (300 MHz, DMSO-*d₆*): δ 13.41 (s, 1H), 11.07 (s, 1H), 8.30-8.26 (m, 2H), 8.07-8.03 (m, 2H), 7.79 (t, *J* = 8.1 Hz, 1H), 7.62 (d, *J=* 8.1 Hz, 1H), 7.39 (d, *J=* 7.5 Hz, 1H), 6.75 (s, 1H), 6.62 (d, *J* = 8.5 Hz, 1H), 5.09-5.01 (m, 1H), 4.11-4.04 (m, 2H), 3.98-3.92 (m, 2H), 3.88 (s, 3H), 3.76-3.70 (m, 2H), 3.69-3.58 (m, 6H), 3.49-3.41 (m, 2H), 3.27-3.19 (m, 4H), 3.07-2.95 (m, 3H), 2.89-2.81 (m, 1H), 2.62-2.57 (m, 1H), 2.55-2.53 (m, 2H), 2.41-2.35 (m, 2H), 2.20-2.14 (m, 2H), 2.09-2.03 (m, 2H), 2.01-1.88 (m, 4H). LC-MS: m/z 910.8 [M+H]⁺. |
| A38 | 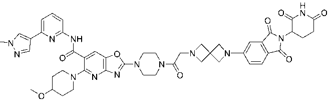 | ¹H NMR (300 MHz, DMSO-*d₆*): δ 13.43 (s, 1H), 11.09 (s, 1H), 8.39-8.25 (m, 2H), 8.15-8.00 (m, 2H), 7.80 (t, *J =* 7.2 Hz, 1H), 7.64 (d, *J=* 8.1 Hz, 1H), 7.41 (d, *J=* 7.5 Hz, 1H), 6.81 (s, 1H), 6.66 (d, *J* = 6.9 Hz, 1H), 5.09-5.01 (m, 1H), 4.15 (s, 3H), 3.96-3.82 (m, 4H), 3.81-3.56 (m, 10H), 3.53-3.42 (m, 6H), 3.31-3.18 (m, 4H), 3.10-2.97 (m, 2H), 2.93-2.77(m, 2H), 2.69-2.56 (m, 2H), 2.22-1.99 (m, 4H). LC-MS: m/z 911.8 [M+H]⁺. |
| A40 | 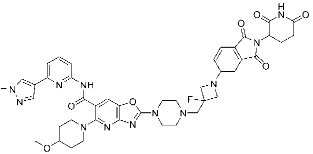 | ¹H NMR (400 MHz, DMSO-*d₆*): δ 13.42 (s, 1H), 11.08 (s, 1H), 8.28-8.26 (m, 2H), 8.07-8.04 (m, 2H), 7.79 (t, *J* = 8.0 Hz, 1H), 7.68 (d, *J =* 8.4 Hz, 1H), 7.39 (d, *J =* 7.6 Hz, 1H), 6.91 (d, *J =* 2.4 Hz, 1H), 6.76 (dd, *J =* 8.4, 2.0 Hz, 1H), 5.07 (dd, *J* = 13.2, 5.2 Hz, 1H), 4.28-4.13 (m, 4H), 3.88 (s, 3H), 3.74-3.69 (m, 4H), 3.48-3.44 (m, 1H), 3.39 (s, 3H), 3.33-3.32 (m, 2H), 3.26-3.20 (m, 2H), 3.03-2.99 (m, 2H), 2.96-2.93 (m, 1H), 2.89-2.82 (m, 1H), 2.74-2.68 (m, 4H), 2.62-2.56 (m, 2H), 2.21-2.15 (m, 2H), 2.08-2.01 (m, 2H). LC-MS: m/z 860.8 [M+H]⁺. |
| A41 | 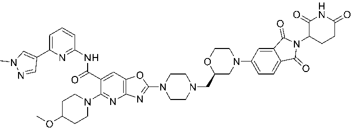 | ¹H NMR (400 MHz, DMSO-*d₆*): δ 13.43 (s, 1H), 11.08 (s, 1H), 8.29-8.24 (m, 2H), 8.08-8.03 (m, 2H), 7.80 (t, *J* = 8.4 Hz, 1H), 7.72 (d, *J* = 8.4 Hz, 1H), 7.42-7.36 (m, 2H), 7.29 (d, *J* = 6.4 Hz, 1H), 5.12-5.02 (m, 1H), 4.01-3.92 (m, 2H), 3.90-3.83 (m, 4H), 3.80-3.69 (m, 5H), 3.68-3.60 (m, 1H), 3.51-3.44 (m, 2H), 3.42-3.38 (m, 4H), 3.27-3.19 (m, 4H), 3.06-2.98 (m, 2H), 2.92-2.82 (m, 1H), 2.77-2.70 (m, 1H), 2.69-2.61 (m, 4H), 2.58-2.55 (m, 2H), 2.22-2.14 (m, 2H), 2.10-2.00 (m, 2H). LC-MS: m/z 872.8 [M+H]⁺. |
| A44 | 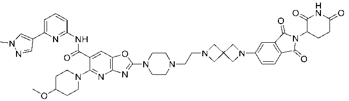 | ¹H NMR (300 MHz, DMSO-*d₆*): δ 13.42 (s, 1H), 11.08 (s, 1H), 8.31-8.24 (m, 2H), 8.09-8.02 (m, 2H), 7.79 (t, *J* = 8.1 Hz, 1H), 7.64 (d, *J* = 8.1 Hz, 1H), 7.39 (d, *J* = 7.8 Hz, 1H), 6.79 (s, 1H), 6.65 (d, *J* = 7.8 Hz, 1H), 5.10-5.00 (m, 1H), 4.12 (s, 3H), 3.88 (s, 3H), 3.77-3.66 (m, 4H), 3.51-3.46 (m, 3H), 3.28-3.16 (m, 8H), 3.07-2.94 (m, 4H), 2.91-2.80 (m, 2H), 2.60-2.55 (m, 4H), 2.40-2.34 (m, 2H), 2.19-2.13 (m, 2H), 2.10-1.95 (m, 4H). LC-MS: m/z 897.9 [M+H]⁺. |
| A45 | 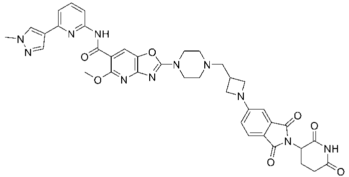 | ¹H NMR (400 MHz, DMSO-*d₆*): δ 11.07 (s, 1H), 10.39 (s, 1H), 8.28 (s, 1H), 8.24 (s, 1H), 8.06-7.99 (m, 2H), 7.80 (t, *J* = 8.0 Hz, 1H), 7.64 (d, *J =* 8.0 Hz, 1H), 7.40 (d, *J =* 7.2 Hz, 1H), 6.78 (s, 1H), 6.65 (d, *J* = 8.4 Hz, 1H), 5.09-5.00 (m, 1H), 4.16 (s, 3H), 3.90 (s, 3H), 3.74-3.69 (m, 4H), 3.33-3.29 (m, 6H), 3.09-3.00 (m, 1H), 2.93-2.82 (m, 1H), 2.72-2.66 (m, 2H), 2.60-2.55 (m, 4H), 2.05-1.95 (m, 1H). LC-MS: m/z 759.8 [M+H]⁺. |
| A46 | 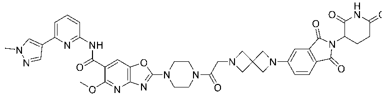 | ¹H NMR (300 MHz, DMSO-*d₆*) δ 11.08 (s, 1H), 10.40 (s, 1H), 8.35 - 8.24 (m, 2H), 8.10 - 7.99 (m, 2H), 7.80 (t, *J=* 7.5 Hz, 1H), 7.64 (d, *J= 8.1* Hz, 1H), 7.41 *(d, J=* 7.2 Hz, 1H), 6.80 (s, 1H), 6.66 (d, *J =* 7.8 Hz, 1H), 5.09 - 5.01 (m, 1H), 4.25 - 4.12 (m, 4H), 4.14 (s, 3H), 3.90 (s, 3H), 3.81 - 3.42 (m, 14H), 2.66 - 2.56 (m, 2H), 2.07 - 1.94 (m, 2H). LC-MS (m/z): 828.8 [M+H]⁺ |
| A47 | 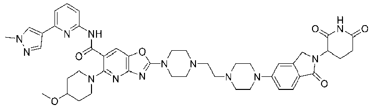 | ¹H NMR (300 MHz, DMSO-*d₆*) δ 13.43 (s, 1H), 10.96 (s, 1H), 8.31 - 8.22 (m, 2H), 8.09 - 8.01 (m, 2H), 7.80 (t, *J =* 8.1 Hz, 1H), 7.52 (d, *J =* 8.4 Hz, 1H), 7.40 (d, *J =* 7.5 Hz, 1H), 7.10 - 7.03 (m, 2H), 5.08 - 5.01 (m, 1H), 4.36 - 4.29 (m, 1H), 4.23 - 4.16 (m, 1H), 3.88 (s, 3H), 3.74 - 3.67 (m, 4H), 3.30 - 3.11 (m, 11H), 3.05 - 2.96 (m, 2H), 2.91 - 2.84 (m, 1H), 2.64 - 2.54 (m, 12H), 2.43 - 2.30 (m, 2H), 2.22 - 2.14 (m, 2H), 2.08 - 1.95 (m, 2H). LC-MS (m/z): 872.1 [ M + H ]⁺ |
| A49 | 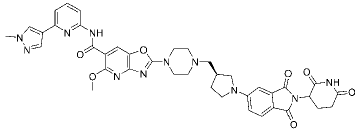 | ¹H NMR (300 MHz, DMSO-*d₆*): δ 11.08 (s, 1H), 10.39 (s, 1H), 8.29 (s, 1H), 8.24 (s, 1H), 8.12-7.95 (m, 2H), 7.80 (t, *J* = 7.5 Hz, 1H), 7.65 (d, *J =* 8.4 Hz, 1H), 7.41 (d, *J =* 7.5 Hz, 1H), 6.90 (s, 1H), 6.82 (d, *J =* 8.1 Hz, 1H), 5.09-5. 00 (m, 1H), 4.16 (s, 3H), 3.90 (s, 3H), 3.82-3.65 (m, 4H), 3.64-3.48 (m, 4H), 3.22-3.05 (m, 4H), 2.91-2.83 (m, 1H), 2.75-2.58 (m, 5H), 2.20-2.12 (m, 1H), 2.05-1.97 (m, 1H), 1.82-1.73 (m, 1H). LC-MS: m/z 773.8 [M+H]⁺. |
| A50 | 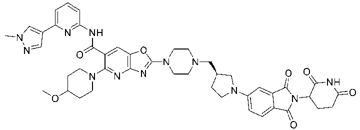 | ¹H NMR (300 MHz, DMSO-*d₆*): δ 13.44 (s, 1H), 11.08 (s, 1H), 8.35-8.20 (m, 2H), 8.12-7.99 (m, 2H), 7.80 (t, *J =* 7.8 Hz, 1H), 7.65 (d, *J =* 8.4 Hz, 1H), 7.39 (d, *J =* 7.2 Hz, 1H), 6.91 (s, 1H), 6.82 (d, *J* = 8.4 Hz, 1H), 5.09-5.01 (m, 1H), 3.88 (s, 3H), 3.81-3.67 (m, 4H), 3.65-3.44 (m, 10H), 3.32-3.17 (m, 4H), 3.08-2.92 (m, 2H), 2.74-2.54 (m, 6H), 2.30-1.90 (m, 6H), 1.84-1.71 (m, 1H). LC-MS: m/z 856.8 [M+H]⁺. |
| A51 | 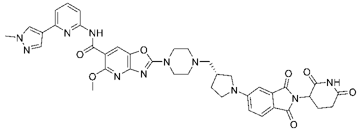 | ¹H NMR (300 MHz, DMSO-*d₆*): δ 11.09 (s, 1H), 10.39 (s, 1H), 8.29 (s, 1H), 8.23 (s, 1H), 8.12-7.95 (m, 2H), 7.80 (t, *J* = 7.5 Hz, 1H), 7.65 (d, *J =* 8.4 Hz, 1H), 7.41 (d, *J =* 7.5 Hz, 1H), 6.90 (s, 1H), 6.82 (d, *J =* 8.1 Hz, 1H), 5.10-4.99 (m, 1H), 4.16 (s, 3H), 3.90 (s, 3H), 3.82-3.64 (m, 4H), 3.63-3.46 (m, 4H), 3.30-3.04 (m, 4H), 2.94-2.82 (m, 1H), 2.72-2.53 (m, 5H), 2.20-2.09 (m, 1H), 2.06-1.96 (m, 1H), 1.83-1.73 (m, 1H). LC-MS: m/z 773.8 [M+H]⁺. |
| A52 | 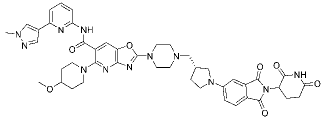 | ¹H NMR (300 MHz, DMSO-*d₆*): δ 13.43 (s, 1H), 11.09 (s, 1H), 8.36-8.19 (m, 2H), 8.15-7.98 (m, 2H), 7.79 (t, *J =* 7.8 Hz, 1H), 7.64 (d, *J =* 8.4 Hz, 1H), 7.39 (d, *J =* 7.2 Hz, 1H), 6.90 (s, 1H), 6.81 (d, *J* = 8.4 Hz, 1H), 5.11-5.01 (m, 1H), 3.88 (s, 3H), 3.81-3.66 (m, 4H), 3.64-3.39 (m, 10H), 3.30-3.09 (m, 3H), 3.08-2.92 (m, 2H), 2.91-2.80 (m, 1H), 2.72-2.53 (m, 6H), 2.48-2.37 (m, 2H), 2.22-1.98 (m, 4H), 1.85-1.68 (m, 1H). LC-MS: m/z 856.8 [M+H]⁺. |
| A53 | 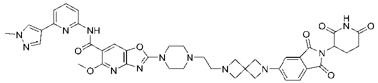 | ¹H NMR (300 MHz, DMSO-*d₆*) δ 11.08 (s, 1H), 10.39 (s, 1H), 8.30 - 8.21 (m, 2H), 8.06 - 7.99 (m, 2H), 7.80 (t, *J=* 8.1 Hz, 1H), 7.64 (d, *J* = 8.4 Hz, 1H), 7.40 (d, *J =* 7.5 Hz, 1H), 6.79 (s, 1H), 6.66 (d, *J =* 8.4 Hz, 1H), 5.08 - 5.01 (m, 1H), 4.17 - 4.11 (m, 7H), 3.90 (s, 3H), 3.77 - 3.71 (m, 4H), 3.52 - 3.48 (m, 4H), 2.93 - 2.78 (m, 2H), 2.74 - 2.67 (m, 2H), 2.65 - 2.59 (m, 4H), 2.47 - 2.37 (m, 3H), 2.04 - 1.96 (m, 1H). LC-MS (m/z): 814.8 [ M + H ]⁺ |
| A55 | 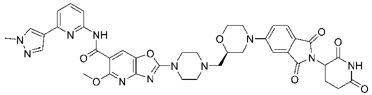 | ¹H NMR (300 MHz, DMSO-*d₆*) δ 11.10 (s, 1H), 10.39 (s, 1H), 8.29 (s, 1H), 8.23 (s, 1H), 8.10 - 7.97 (m, 2H), 7.80 (t, *J =* 7.8 Hz, 1H), 7.72 (d, *J =* 8.1 Hz, 1H), 7.45 - 7.33 (m, 2H), 7.29 (d, *J=* 8.4 Hz, 1H), 5.13 - 5.03 (m, 1H), 4.16 (s, 3H), 4.02 - 3.82 (m, 5H), 3.81 - 3.54 (m, 6H), 3.53 - 3.43 (m, 2H), 3.03 - 2.78 (m, 3H), 2.74 - 2.53 (m, 7H), 2.07 - 1.97 (m, 1H). LC-MS (m/z): 789.8 [M+H]⁺ |
| A56 | 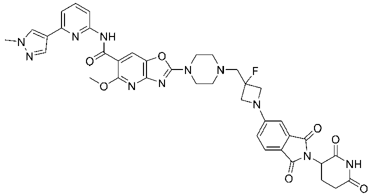 | ¹H NMR (300 MHz, DMSO-*d₆*) δ 11.09 (s, 1H), 10.39 (s, 1H), 8.40 - 8.19 (m, 2H), 8.16 - 7.94 (m, 2H), 7.80 (t, *J=* 7.8 Hz, 1H), 7.69 (d, *J= 8.1* Hz, 1H), 7.41 *(d, J=* 7.2 Hz, 1H), 6.91 (s, 1H), 6.77 (d, *J* = 8.1 Hz, 1H), 5.13 - 5.01 (m, 1H), 4.36 - 4.08 (m, 5H), 4.07 - 3.81 (m, 5H), 3.80 - 3.60 (m, 4H), 3.58 - 3.49 (m, 2H), 3.06 - 2.91 (m, 2H), 2.79 - 2.53 (m, 5H), 2.07 - 1.94 (m, 1H). LC-MS (m/z): 777.8 [M+H]⁺ |
| A57 | 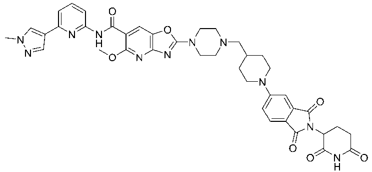 | ¹H NMR (300 MHz, DMSO-*d₆*) δ 11.09 (s, 1H), 10.39 (s, 1H), 8.30 - 8.20 (m, 2H), 8.06 - 8.00 (m, 2H), 7.80 (t, *J=* 8.1 Hz, 1H), 7.65 (d, *J=* 8.4 Hz, 1H), 7.40 (d, *J =* 7.5 Hz, 1H), 7.32 (s, 1H), 7.24 (d, *J* = 8.4 Hz, 1H), 5.10 - 5.03 (m, 1H), 4.16 (s, 3H), 4.09 - 4.02 (m, 2H), 3.90 (s, 3H), 3.74 - 3.68 (m, 4H), 3.32 - 3.15 (m, 4H), 3.06 - 2.78 (m, 4H), 2.63 - 2.56 (m, 2H), 2.25 - 2.18 (m, 2H), 2.04 - 1.97 (m, 1H), 1.85 - 1.78 (m, 2H), 1.23 - 1.13 (m, 2H). LC-MS (m/z): 787.8 [ M + H ] ⁺ |
| A59 | 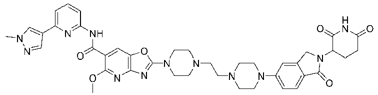 | ¹H NMR (300 MHz, DMSO-*d₆*) δ 10.96 (s, 1H), 10.39 (s, 1H), 8.31 - 8.21 (m, 2H), 8.07 - 7.99 (m, 2H), 7.80 (t, *J=* 7.8 Hz, 1H), 7.53 (d, *J=* 8.4 Hz, 1H), 7.40 (d, *J =* 7.5 Hz, 1H), 7.11 - 7.03 (m, 2H), 5.08 - 5.00 (m, 1H), 4.37 - 4.29 (m, 1H), 4.24 - 4.19 (m, 1H), 4.16 (s, 3H), 3.90 (s, 3H), 3.75 - 3.67 (m, 4H), 3.52 - 3.42 (m, 6H), 3.10 - 3.03 (m, 1H), 2.96 - 2.80 (m, 2H), 2.65 - 2.57 (m, 8H), 2.43 - 2.29 (m, 2H), 2.00 - 1.91 (m, 1H). LC-MS (m/z): 788.8 [ M + H ]⁺ |
| A60 | 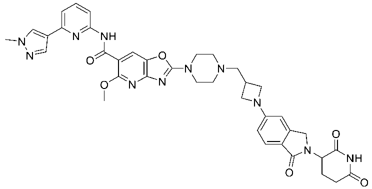 | ¹H NMR (300 MHz, DMSO-*d₆*) δ 10.93 (s, 1H), 10.38 (s, 1H), 8.31 - 8.20 (m, 2H), 8.08 - 7.99 (m, 2H), 7.85 - 7.76 (m, 1H), 7.51 - 7.46 (m, 1H), 7.44 - 7.37 (m, 1H), 6.53 - 6.46 (m, 2H), 5.09 - 5.01 (m, 1H), 4.35 - 4.25 (m, 2H), 4.16 (s, 3H), 4.07 - 4.00 (m, 2H), 3.91 (s, 3H), 3.75 - 3.68 (m, 4H), 3.64 - 3.54 (m, 4H), 3.03 - 2.97 (m, 1H), 2.92 - 2.86 (m, 1H), 2.70 - 2.65 (m, 2H), 2.58 - 2.56 (m, 2H), 2.37 - 2.32 (m, 1H), 2.01 - 1.91 (m, 2H). LC-MS (m/z): 745.9 [ M + H ]⁺ |
| A61 | 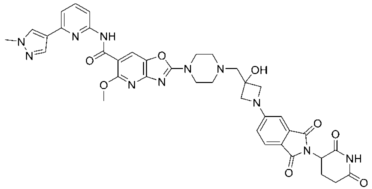 | ¹H NMR (300 MHz, DMSO-*d₆*) δ 11.09 (s, 1H), 10.38 (s, 1H), 8.29 (s, 1H), 8.22 (s, 1H), 8.11 - 7.96 (m, 2H), 7.80 (t, *J =* 7.5 Hz, 1H), 7.64 (d, *J =* 7.8 Hz, 1H), 7.40 (d, *J =* 7.2 Hz, 1H), 6.82 (s, 1H), 6.69 (d, *J* = 7.8 Hz, 1H), 5.90 (s, 1H), 5.11 - 5.01 (m, 1H), 4.16 (s, 3H), 4.11 - 4.00 (m, 2H), 3.90 (s, 3H), 3.86 - 3.78 (m, 2H), 3.77 - 3.62 (m, 4H), 2.96 - 2.66 (m, 7H), 2.63 - 2.53 (m, 2H), 2.07 - 1.95 (m, 1H). LC-MS (m/z): 775.8 [M+H]⁺ |
| A64 | 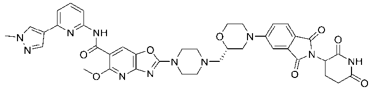 | ¹H NMR (300 MHz, DMSO-*d₆*) δ 11.10 (s, 1H), 10.39 (s, 1H), 8.29 (s, 1H), 8.23 (s, 1H), 8.13 - 7.95 (m, 2H), 7.80 (t, *J =* 7.8 Hz, 1H), 7.72 (d, *J =* 8.1 Hz, 1H), 7.48 - 7.33 (m, 2H), 7.29 (d, *J=* 7.5 Hz, 1H), 5.14 - 5.04 (m, 1H), 4.16 (s, 3H), 4.02 - 3.82 (m, 5H), 3.81 - 3.54 (m, 6H), 3.53 - 3.42 (m, 2H), 3.03 - 2.78 (m, 3H), 2.76 - 2.53 (m, 7H), 2.07 - 1.98 (m, 1H). LC-MS (m/z): 789.8 [M+H]⁺ |
| A65 | 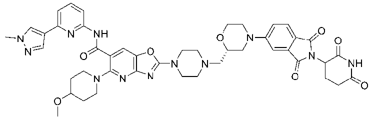 | ¹H NMR (300 MHz, DMSO-*d₆*) δ 13.42 (s, 1H), 11.10 (s, 1H), 8.34 - 8.19 (m, 2H), 8.12 - 7.99 (m, 2H), 7.79 (t, *J=* 7.8 Hz, 1H), 7.71 (d, *J= 8.1* Hz, 1H), 7.44 - 7.32 (m, 2H), 7.28 (d, J = 8.1 Hz, 1H), 5.13 - 5.04 (m, 1H), 4.03 - 3.92 (m, 2H), 3.91 - 3.81 (m, 4H), 3.80 - 3.66 (m, 5H), 3.65 - 3.53 (m, 2H), 3.52 - 3.38 (m, 6H), 3.26 - 3.17 (m, 2H), 3.06 - 2.82 (m, 4H), 2.74 - 2.54 (m, 7H), 2.22 - 2.11 (m, 2H), 2.08 - 1.97 (m, 2H). LC-MS (m/z): 872.8 [M+H]⁺ |
| A67 | 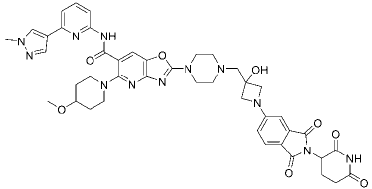 | ¹H NMR (300 MHz, DMSO-*d₆*) δ 13.44 (s, 1H), 11.09 (s, 1H), 8.39 - 8.21 (m, 2H), 8.14 - 7.98 (m, 2H), 7.80 (t, *J=* 7.8 Hz, 1H), 7.65 (d, *J= 8.1* Hz, 1H), 7.40 (d, *J =* 7.5 Hz, 1H), 6.82 (s, 1H), 6.69 (d, *J =* 6.9 Hz, 1H), 5.89 (s, 1H), 5.11 - 5.01 (m, 1H), 4.15 - 4.03 (m, 2H), 3.98 - 3.76 (m, 5H), 3.75 - 3.60 (m, 4H), 3.58 - 3.40 (m, 6H), 3.31 - 3.17 (m, 2H), 3.11 - 2.94 (m, 2H), 2.93 - 2.64 (m, 6H), 2.62 - 2.54 (m, 2H), 2.23 - 1.93 (m, 4H). LC-MS (m/z): 858.8 [M+H]⁺ |

### Experiment 1: IRAK4 Protein Degradation Activity Assay

### Experimental Procedures

Human acute monocytic leukemia cell line THP-1 (ATCC) was used and cultured in a sterile incubator at 37-38 °C, pH 7.2-7.4, under 5% CO₂. THP-1 cells (7×10⁵ per well) were seeded in 6-well plates and treated with compounds at various concentrations for 24 hours. Proteins were extracted using RIPA lysis buffer, mixed with protein loading buffer, and boiled at 98 °C for 10 minutes.

20 µL of each sample was separated by 7.5% SDS-PAGE and transferred to an NC membrane. The membrane was blocked with 5% non-fat milk (PBS + 0.05% Tween-20) at room temperature for 1 hour, then incubated with primary antibodies overnight at 4 °C (anti-IRAK4, 4363S, CST; anti-Actin, A2066, Sigma). After incubation with a secondary antibody (Goat anti-Rabbit, IRDye^{®} 680RD, LI-COR) at room temperature for 1 hour, detection was performed using an Odyssey CLx dual-color infrared laser imaging system.

Degrader-5 was used as the positive control (Cell Chemical Biology 2020, 27, 1500-1509).

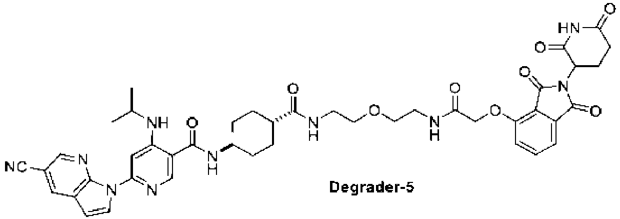

### 2. Experimental Results

**Table 2 IRAK4 degradation activity of compounds in THP-1 cells**

| NO. | Protein Degradation (%) 0.1 µM | Protein Degradation (%) 0.3 µM | Protein Degradation (%) 1.0 µM |
|---|---|---|---|
| **Degrader-5** | 9 | 34 | 48 |
| **A1** | - | 10 | 0 |
| **A2** | - | - | 40 |
| **A3** | 87 | 91 | 86 |
| **A4** | 82 | 90 | 90 |
| **A5** | 76 | 80 | 84 |
| **A6** | 80 | 82 | 81 |
| **A7** | 34 | 13 | 48 |
| **A8** | 71 | 58 | 48 |
| **A11** | 0 | 11 | 19 |
| **A12** | 33 | 37 | 46 |
| **A13** | 40 | 54 | 61 |
| **A35** | 92 | 83 | 94 |
| **A37** | 66 | 69 | 65 |
| **A38** | 72 | 56 | 63 |
| **A40** | 63 | 66 | 67 |
| **A41** | 68 | 66 | 65 |
| **A44** | 84 | 82 | 81 |
| **A45** | 64 | 65 | 67 |
| **A46** | 36 | 45 | 51 |
| **A47** | 74 | 63 | 58 |
| **A49** | 45 | 59 | 53 |
| **A50** | 19 | 46 | 68 |
| **A51** | 59 | 56 | 68 |
| **A52** | 66 | 65 | 67 |
| **A53** | 71 | 77 | 82 |
| **A55** | 59 | 70 | 66 |
| **A56** | 19 | 38 | 43 |
| **A57** | 31 | 31 | 33 |
| **A59** | 38 | 41 | 39 |
| **A60** | 57 | 55 | 53 |
| **A61** | 34 | 44 | 47 |
| **A65** | 52 | 43 | 36 |
| **A67** | 38 | 43 | 51 |

Currently, there is no unified standard in the field for defining the degree of IRAK4 degradation. Based on the preliminary research and analysis of the present disclosure, the protein degraders of the present disclosure are considered likely to exert biological effects when they degrade IRAK4 protein by more than 30%. Owing to the unique physiological functions and mechanisms of PROTACs, the degradation activity may not necessarily be linear with biological effects; that is, PROTAC molecules with relatively weak degradation capacity may still exhibit favorable biological effects.

The degradation activity assay results show that the compounds prepared in the present disclosure can effectively degrade IRAK4 protein in cells. Among them, Compounds A3, A4, A5, A6, A8, A13, A35, A37, A38, A40, A41, A44-A47, A49-A53, A55, A60, and A67 display favorable degradation activity, all superior to the positive control Degrader-5 under the same test conditions.

### Experiment 2: FLT3 Protein Degradation Activity Assay

### 1. Experimental Procedures

Human acute myeloid leukemia cell line MOLM-13 (ATCC) was used and cultured in a sterile incubator at 37-38 °C, pH 7.2-7.4, under 5% CO₂. MOLM-13 cells (7×10⁵ per well) were seeded in 6-well plates and treated with compounds at various concentrations for 24 hours. Proteins were extracted using RIPA lysis buffer, mixed with protein loading buffer, and boiled at 98 °C for 10 minutes.

20 µL of each sample was separated by 7.5% SDS-PAGE and transferred to an NC membrane. The membrane was blocked with 5% non-fat milk (PBS + 0.05% Tween-20) at room temperature for 1 hour, then incubated with primary antibodies overnight at 4 °C (anti-FLT3, ab245116, Abcam; anti-Actin, A2066, Sigma). After incubation with a secondary antibody (Goat anti-Rabbit, IRDye^{®} 680RD, LI-COR) at room temperature for 1 hour, detection was performed using an Odyssey CLx dual-color infrared laser imaging system.

### 2. Experimental Results

**Table 3 FLT3 degradation activity of compounds in MOLM-13 cells**

| NO. | Protein Degradation (%) 0.3 µM | Protein Degradation (%) 1.0 µM |
|---|---|---|
| **A3** | 45 | 52 |
| **A5** | 64 | 66 |
| **A6** | 41 | 49 |
| **A8** | 30 | 50 |
| **A9** | 70 | 82 |
| **A13** | 38 | 50 |
| **A35** | 69 | 68 |
| **A37** | 79 | 74 |
| **A38** | 73 | 80 |
| **A40** | 42 | 33 |
| **A44** | 85 | 82 |
| **A45** | 26 | 43 |
| **A46** | 32 | 31 |
| **A47** | 57 | 67 |
| **A50** | 48 | 51 |
| **A52** | 44 | 52 |
| **A53** | 53 | 38 |
| **A55** | 53 | 32 |
| **A56** | 36 | 37 |
| **A57** | 39 | 61 |
| **A59** | 46 | 32 |
| **A61** | - | 32 |
| **A64** | 42 | 43 |
| **A65** | 42 | 61 |
| **A67** | 66 | 76 |

Currently, there is no unified standard in the field for defining the degree of FLT3 degradation. Based on the preliminary research and analysis of the present disclosure, the protein degraders of the present disclosure are considered likely to exert biological effects when they degrade FLT3 protein by more than 30%. Due to the unique physiological functions and mechanisms of PROTACs, the degradation capacity and biological effects may not necessarily be linear.

The degradation activity assay results show that the compounds prepared in the present disclosure can effectively degrade FLT3 protein in cells. Among them, Compounds A3, A5, A6, A8, A9, A13, A35, A37, A38, A40, A44, A46, A47, A50, A52, A53, A55-A57, A59, A64, A65, and A67 exhibit favorable degradation activity.

Dual-target IRAK4/FLT3 protein degraders have been rarely studied, and it remains unclear whether their biological effects and synergistic mechanisms require substantial degradation of both target proteins simultaneously.

### Experiment 3: Compound Inhibition of Tumor Cell Proliferation Assay

### 1. Experimental Procedures

Human acute myeloid leukemia cell line MOLM-13 (ATCC) was used and cultured in a sterile incubator at 37-38 °C, pH 7.2-7.4, under 5% CO₂. MOLM-13 cells (1×10⁴ per well) were seeded in 96-well plates, treated with compounds at various concentrations, and incubated further.

After 48 hours of cell culture, an equal volume of CellCounting-Lite reagent (DD1101-01, Vazyme) was added, mixed by shaking for 5 minutes, and left at room temperature for 10 minutes. Cell proliferation was measured using a VICTOR Nivo multimode plate reader.

### 2. Experimental Results

**Table 4 Inhibitory activity of compounds against tumor cell proliferation**

| NO. | MOLM-13 IC₅₀ (nM) |
|---|---|
| **A3** | 87 |
| **A4** | 516 |
| **A5** | 41 |
| **A6** | 157 |
| **A8** | 129 |
| **A9** | 87 |
| **A37** | 88 |
| **A40** | 95 |
| **A44** | 54 |

According to the present disclosure, compounds with an IC₅₀ ≤ 500 nM are considered to exhibit favorable inhibitory activity against tumor cell proliferation.

The tumor cell proliferation assay results show that Compounds A3, A5, A6, A8, A9, A37, A40, and A44 possess inhibitory activity against the proliferation of MOLM-13 tumor cells.

## Claims

1. A compound having the structure of formula (I), or a pharmaceutically acceptable salt or solvate thereof: 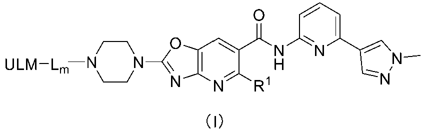 wherein,
R¹ is selected from the group consisting of the following moieties: 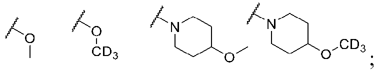
ULM is selected from the group consisting of the following moieties: 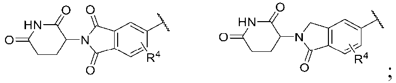
Lₘ is a linking moiety composed of m fully identical, partially identical, or mutually different L groups, and covalently links ULM to the piperazine ring;
m is selected from the group consisting of 1, 2, 3, 4, and 5;
each L is independently selected from the group consisting of the following groups:
1) C₃₋₁₂ cycloalkylene substituted with 1 to 3 R^{b} groups independent of each other;
2) C₆₋₁₀ arylene substituted with 1 to 3 R^{b} groups independent of each other;
3) 4- to 12-membered heterocycloalkylene substituted with 1 to 3 R^{b} groups independent of each other;
4) 5- to 12-membered heteroarylene substituted with 1 to 3 R^{b} groups independent of each other;
5) C₁₋₁₂ alkylene substituted with 1 to 3 R^{d} groups independent of each other;
6) C₂₋₁₂ alkenylene substituted with 1 to 3 R^{d} groups independent of each other;
7) C₂₋₁₂ alkynylene substituted with 1 to 3 R^{d} groups independent of each other;
8) a moiety having 1 to 6 units of ethylene glycol or propylene glycol; and
9) -C(O)-, -C(O)O-, -O-, -N(R^{c})-, -S-, -S(O)-, -C(S)-, -C(S)O-, -S(O)₂-, -S(O)N(R^{c})-, -S(O)₂N(R^{c})-, -C(O)-N(R^{c})-, -N(R^{c})C(O)-N(R^{c})-, and -OC(O)-N(R^{c})-;
if present, R⁴ is selected from the group consisting of hydrogen, deuterium, halogen, cyano, C₁₋₆ alkyl, C₃₋₆ cycloalkyl, C₆₋₁₀ aryl, C₅₋₁₂ heteroaryl, -OR^{c}, -C(O)R^{c}, -C(O)OR^{c}, -C(O)-N(R^{c})₂, -N(R^{c})₂, -N(R^{c})C(O)-R^{c}, -N(R^{c})C(O)O-R^{c}, -N(R^{c})C(O)N(R^{c})₂, -OC(O)R^{c}, -OC(O)-N(R^{c})₂, -SR^{c}, -S(O)R^{c}, -S(O)₂R^{c}, and -S(O)₂-N(R^{c})₂;
if present, each R^{b} is independently selected from the group consisting of hydrogen, deuterium, halogen, cyano, C₁₋₆ alkyl, C₃₋₆ cycloalkyl, C₆₋₁₀ aryl, C₅₋₁₂ heteroaryl, -OR^{c}, -C(O)R^{c}, -C(O)OR^{c}, -C(O)-N(R^{c})₂, -N(R^{c})₂, -N(R^{c})C(O)-R^{c}, -N(R^{c})C(O)O-R^{c}, -N(R^{c})C(O)N(R^{c})₂, -OC(O)R^{c}, -OC(O)-N(R^{c})₂, -SR^{c}, -S(O)R^{c}, -S(O)₂R^{c}, and -S(O)₂-N(R^{c})₂; if present, said C₁₋₆ alkyl, C₃₋₆ cycloalkyl, C₆₋₁₀ aryl, or C₅₋₁₂ heteroaryl is optionally substituted with 1 to 3 R^{d} groups; if present, any two non-adjacent R^{b} groups optionally form a bridged ring via carbon atoms; or any two R^{b} groups, together with the carbon atom to which they are attached, form a spiro ring;
if present, each R^{c} is independently selected from the group consisting of hydrogen, C₁₋₆ alkyl, and C₃₋₆ cycloalkyl;
if present, each R^{d} is independently selected from the group consisting of hydrogen, deuterium, halogen, hydroxy, amino, methylamino, dimethylamino, cyano, methyl, deuterated methyl, methoxy, and deuterated methoxy.

2. The compound according to claim 1, or the pharmaceutically acceptable salt or solvate thereof, wherein
Lₘ is a linking moiety composed of m fully identical, partially identical, or mutually different L groups, and covalently links ULM to the piperazine ring;
m is selected from the group consisting of 1, 2, 3, 4, and 5;
each L is independently selected from the group consisting of the following groups:
1) C₃₋₁₂ cycloalkylene substituted with 1 to 3 R^{b} groups independent of each other;
2) C₆₋₁₀ arylene substituted with 1 to 3 R^{b} groups independent of each other;
3) 4- to 12-membered heterocycloalkylene substituted with 1 to 3 R^{b} groups independent of each other;
4) 5- to 12-membered heteroarylene substituted with 1 to 3 R^{b} groups independent of each other;
5) a moiety having 1 to 6 units of ethylene glycol or propylene glycol; and
6) -C(O)-, -C(O)O-, -O-, -N(R^{c})-, -C(O)-N(R^{c})-, -N(R^{c})C(O)-N(R^{c})-, and -OC(O)-N(R^{c})-;
R^{b} and R^{c} are as defined in claim 1.

3. The compound according to claim 1, or the pharmaceutically acceptable salt or solvate thereof, wherein
Lₘ is a linking moiety composed of m fully identical, partially identical, or mutually different L groups, and covalently links ULM to the piperazine ring;
m is selected from the group consisting of 1, 2, 3, 4, and 5;
each L is independently selected from the group consisting of the following groups:
(1) -C(O)-, -CH₂-, -C(D)(H) -, -C(D)₂ -, -CR^{c}F-, -CF₂-, -O-, -N(R^{c})-, -C(O)-N(R^{c})-, -N(R^{c})C(O)-N(R^{c})-, and -OC(O)-N(R^{c})-; and
(2) one of the following moieties: 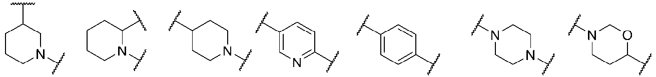 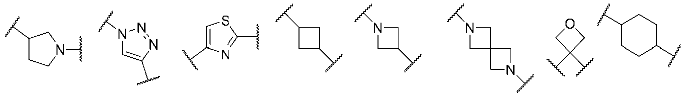 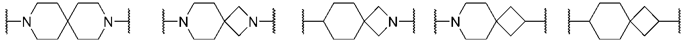 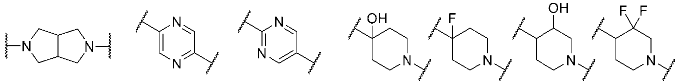 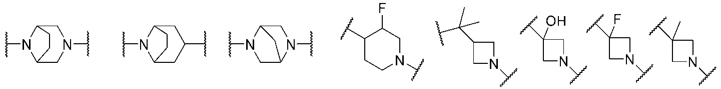 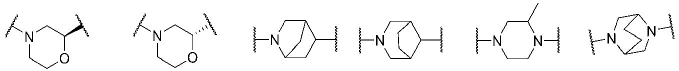 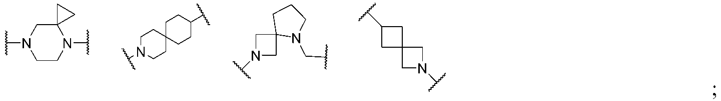
R^{c} is as defined in claim 1.

4. The compound according to claim 3, or the pharmaceutically acceptable salt or solvate thereof, wherein
at least one L is selected from the group consisting of the following moieties:

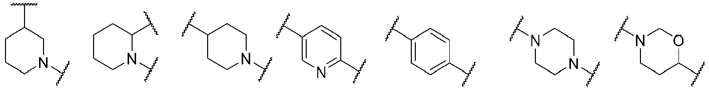

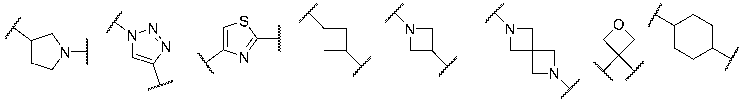

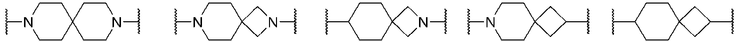

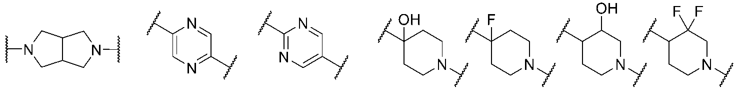

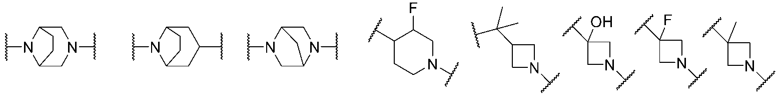

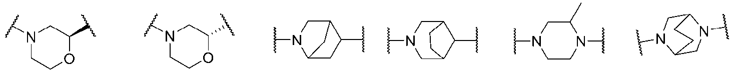

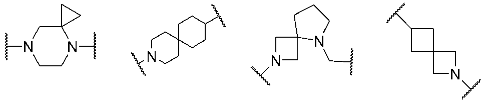

5. The compound according to any one of claims 1, 3 and 4, or the pharmaceutically acceptable salt or solvate thereof, wherein
Lₘ is selected from the group consisting of the following moieties:

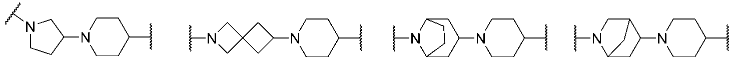

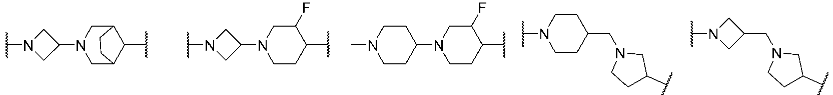

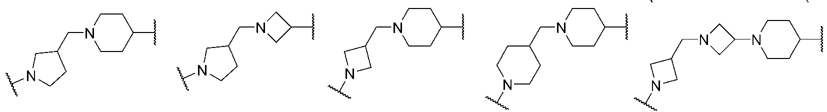

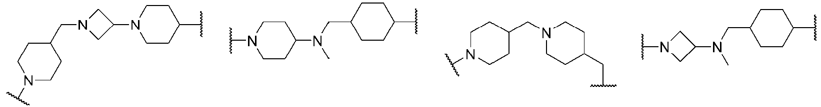

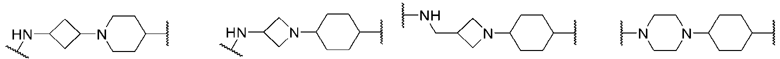

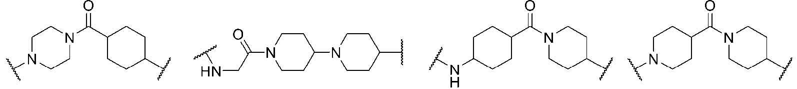

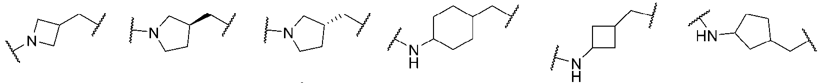

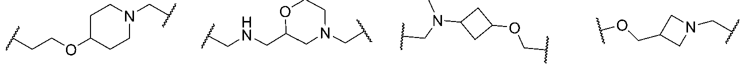

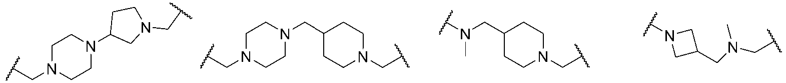

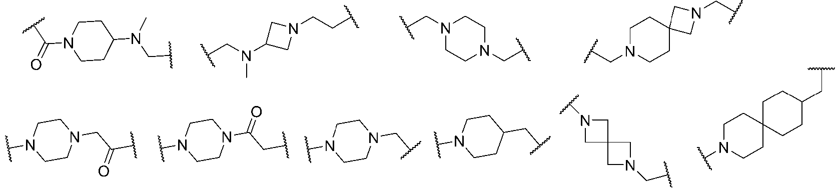

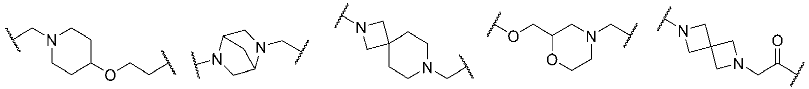

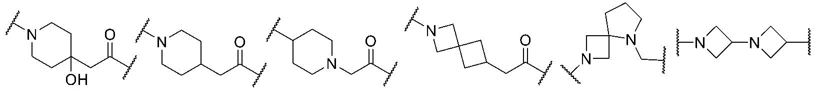

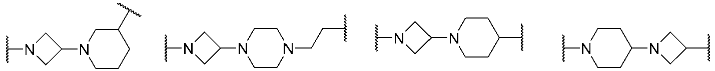

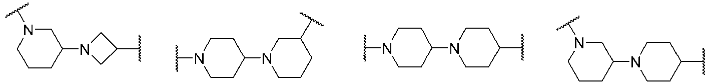

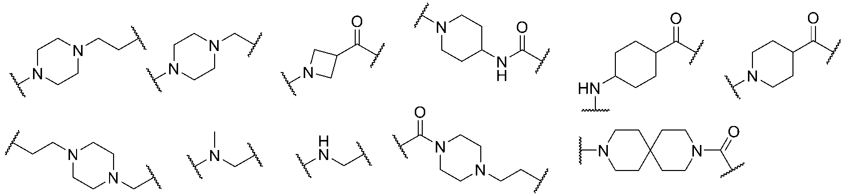

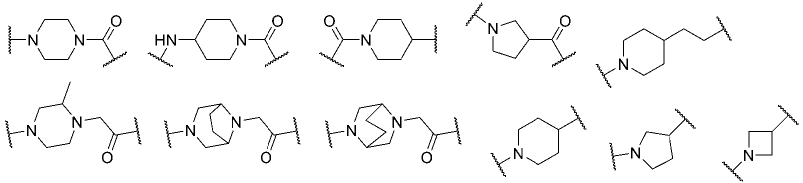

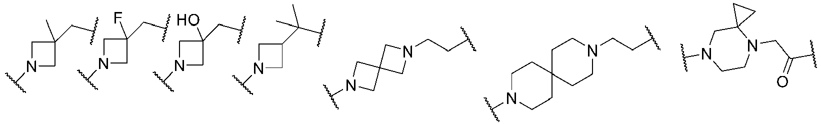

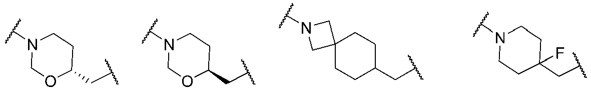

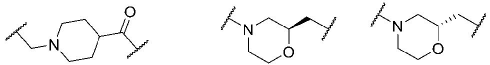

6. The compound according to any one of claims 1, 3, 4 and 5, or the pharmaceutically acceptable salt or solvate thereof, wherein
Lₘ is selected from the group consisting of the following moieties:

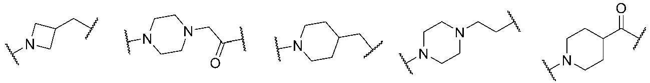

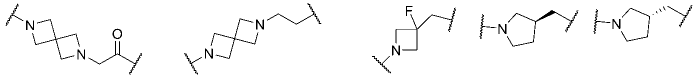

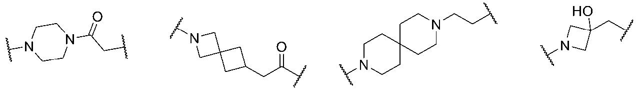

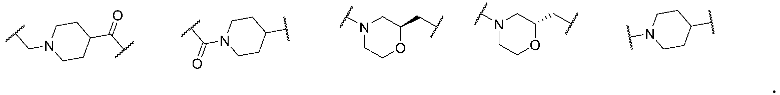

7. A compound selected from the group consisting of the following compounds, or a pharmaceutically acceptable salt or solvate thereof:

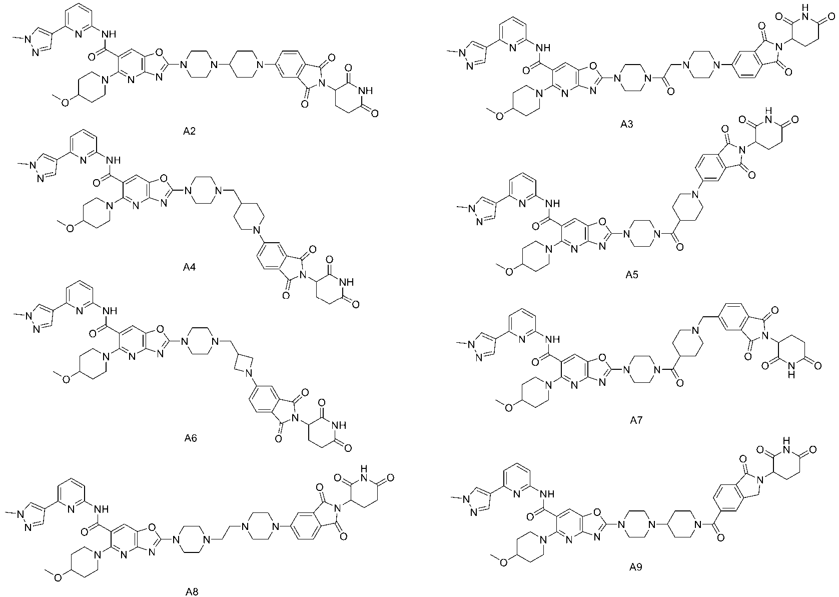

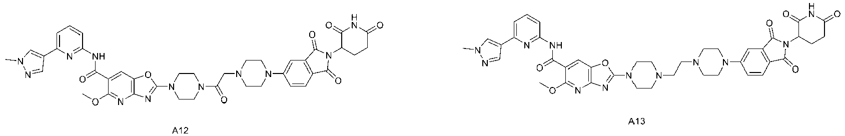

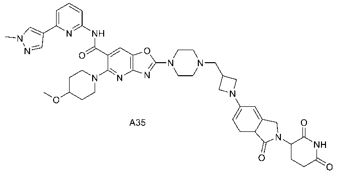

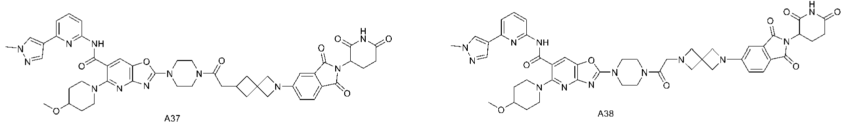

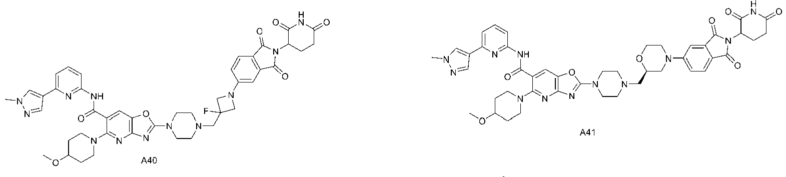

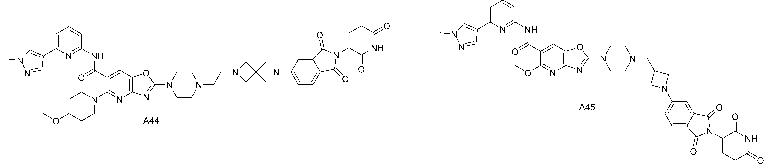

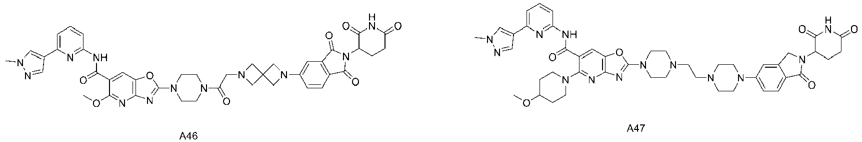

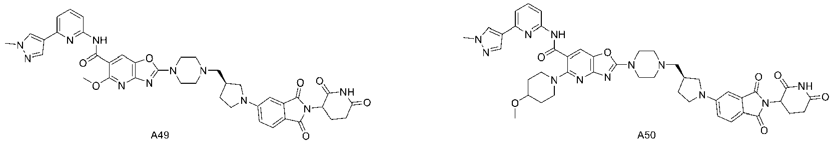

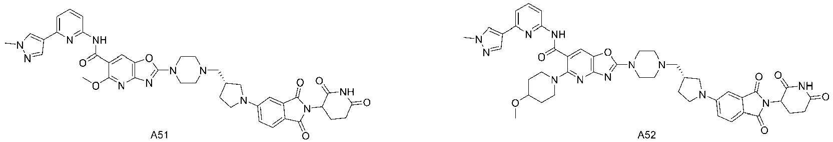

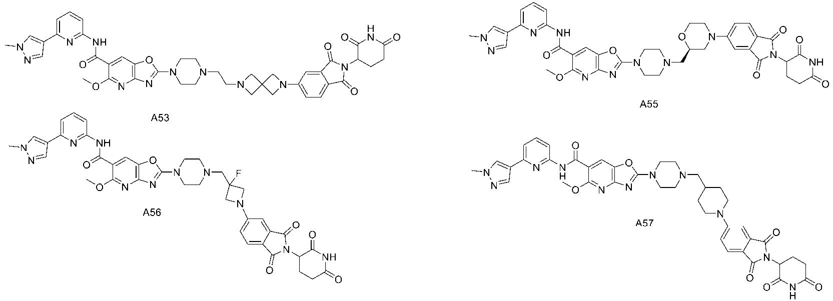

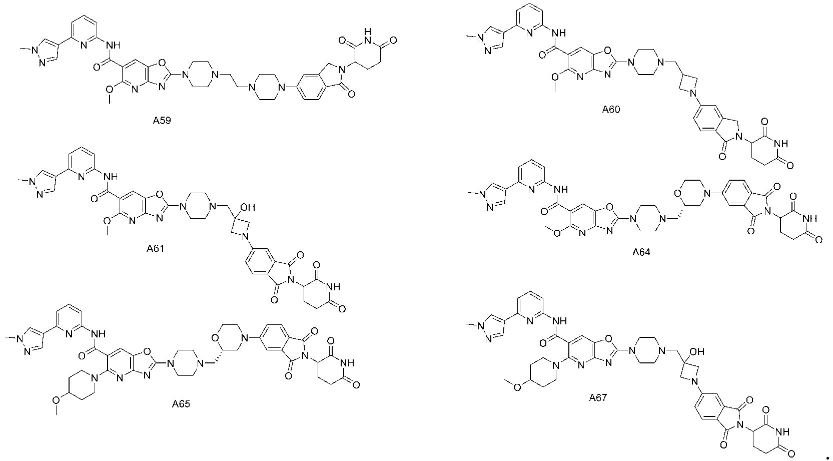

8. A pharmaceutical composition comprising the compound according to any one of claims 1-7, or the pharmaceutically acceptable salt or solvate thereof;
preferably, the pharmaceutical composition further comprises at least one pharmaceutically acceptable carrier.

9. A pharmaceutical combination comprising the compound according to any one of claims 1-7, or the pharmaceutically acceptable salt or solvate thereof, or the pharmaceutical composition according to claim 8;
preferably, the pharmaceutical combination further comprises at least one additional drug for the prevention and/or treatment of a disease and/or disorder responsive to IRAK4 and/or FLT3.

10. Use of the compound or the pharmaceutically acceptable salt or solvate thereof according to any one of claims 1-7, the pharmaceutical composition according to claim 8, or the pharmaceutical combination according to claim 9 in the manufacture of a medicament for the prevention and/or treatment of the disease and/or disorder at least partially responsive to IRAK4 and/or FLT3.

11. The use according to claim 10, wherein the disease and/or disorder includes:
lymphoma, leukemia, myelodysplastic syndrome, endometriosis, atopic dermatitis, hidradenitis suppurativa, psoriasis, systemic lupus erythematosus, multiple sclerosis, or rheumatoid arthritis.
